# EUROPEAN PATENT APPLICATION

(11) **EP 2 301 550 A1**
(43) Date of publication of application: **30.03.2011**
(21) Application number: 10185596.3
(22) Date of filing: 15.07.2002
(51) Int. Cl.: A61K 31/65

(54) **Tetracycline compounds having target therapeutic activities**

(30) Priority: 13.07.2001 US 305546 P; 12.07.2002 US 395741 P
(62) Divisional of application: 02748169.6
(71) Applicant: Paratek Pharmaceuticals, Inc., Boston, MA 02111 (US)
(72) Inventor: Levy, Stuart, B., Boston, MA 02116 (US); Draper, Michael, Windham, New Hampshire 03087 (US); Nelson, Mark, L., 47 Barnstable Road (US); Jones, Graham, Ithaca, NY 14850 (US)
(74) Representative: Johnston, Magnus George

(57) **Abstract**

Methods and compounds for treating osteomyelitis, bronchiectasis, cystic fibrosis, malaria or periodontitis, with a tetracycline compound of formula I: 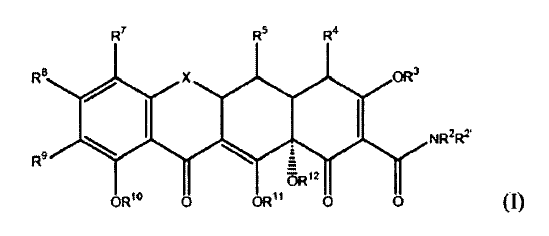 wherein:
the substituents R²-R¹² have the values defined in the claims.

## Description

### Related Applications

This application claims priority to U.S. Provisional Patent Application Serial No. 60/XXX,XXX, entitled "Methods of Using Substituted Tetracyclines to Treat Inflammatory Process Associated States," filed July 12, 2002; and U.S. Provisional Patent Application Serial No. 60/305,546, entitled "Methods of Using Substituted Tetracyclines to Treat Inflammatory Process Associated States," filed July 13, 2001. The entire contents of each aforementioned applications are hereby incorporated herein by reference in their entirety.

### Background of the Invention

Inflammation is the body's reaction to injury and infection. Major events involved in inflammatory processes include increased blood supply to the injured or infected area; increased capillary permeability enabled by retraction of endothelial cells; and migration of leukocytes out of the capillaries and into the surrounding tissue (Roitt et al., Immunology, Grower Medical Publishing, New York, 1989).

Increased capillary permeability allows larger molecules and cells to cross the endothelium that are not ordinarily capable of doing so, thereby allowing soluble mediators of immunity and leukocytes to reach the injured or infected site. Leukocytes, primarily neutrophil polymorphs (also known as polymorphonuclear leukocytes, neutrophils or PMNS) and macrophages, migrate to the injured site by a process known as chemotaxis. At the site of inflammation, tissue damage and complement activation cause the release of chemotactic peptides such as C5a. Complement activation products are also responsible for causing degranulation of phagocytic cells, mast cells and basophils, smooth muscle contraction and increases in vascular permeability (Mulligan et al. 1991 J. Immunol. 148:1479-1485).

The traversing of leukocytes from the bloodstream to extravascular sites of inflammation or immune reaction involves a complex but coordinated series of events. At the extravascular site of infection or tissue injury, signals are generated such as bacterial endotoxins, activated complement fragments or proinflammatory cytokines such as interleukin 1 (IL-1), interleukin 6 (IL-6), and tumor necrosis factor (TNF) which activate leukocytes and/or endothelial cells and cause one or both of these cell types to become adhesive. Initially, cells become transiently adhesive (manifested by rolling) and later, such cells become firmly adhesive (manifested by sticking). Adherent leukocytes travel across the endothelial cell surface, diapedese between endothelial cells and migrate through the subendothelial matrix to the site of inflammation or immune reaction (Harlan et al., Adhesion-Its role in Inflammatory Disease, W. H. Freeman & Co., New York, 1992).

Although leukocyte traversal of vessel walls to extravascular tissue is necessary for host defense against foreign antigens and organisms, leukocyte-endothelial interactions often have deleterious consequences for the host. For example, during the process of adherence and transendothelial migration, leukocytes release oxidants, proteases and cytokines that directly damage endothelium or cause endothelial dysfunction. Once at the extravascular site, emigrated leukocytes further contribute to tissue damage by releasing a variety of inflammatory mediators. Moreover, single leukocytes sticking within the capillary lumen or aggregation of leukocytes within larger vessels are responsible for microvascular occlusion and ischemia. Leukocyte-mediated vascular and tissue injury has been implicated in pathogenesis of a wide variety of clinical disorders such as acute and chronic allograft rejection, vasculitis, rheumatoid and other forms of inflammatory based arthritis, inflammatory skin diseases, adult respiratory distress syndrome, ischemia-reperfusion syndromes such as myocardial infarction, shock, stroke, organ transplantation, crush injury and limb replantation.

Many other serious clinical conditions involve underlying inflammatory processes in humans. For example, multiple sclerosis (MS) is an inflammatory disease of the central nervous system. In MS, circulating leukocytes infiltrate inflamed brain endothelium and damage myelin, with resultant impaired nerve conduction and paralysis (Yednock et al., 1992 Nature 366:63-66).

Infiltration of airways by inflammatory cells, particularly eosinophils, neutrophils and T lymphocytes are characteristic features of atopic or allergic asthma (Cotran et al., Pathological Basis of Disease, W. B. Saunders, Philadelphia, 1994). Cellular infiltration of the pancreas with resultant destruction of islet beta-cells is the underlying pathogenesis associated with insulin-dependent diabetes mellitus (Burkly et al. 1994 Diabetes 43: 529-534). Activation of inflammatory cells whose products cause tissue injury underlies the pathology of inflammatory bowel diseases such as Crohn's disease and ulcerative colitis. Neutrophils, eosinophils, mast cells, lymphocytes and macrophages contribute to the inflammatory response.

Various anti-inflammatory drugs are currently available for use in treating conditions involving underlying inflammatory processes. Their effectiveness however, is widely variable and there remains a significant clinical unmet need. This is especially true in the aforementioned diseases where available therapy is either of limited effectiveness or is accompanied by unwanted side effect profiles.

### Summary

In one embodiment, the invention pertains, at least in part, to a method for treating a disease with a tetracycline compound having a target therapeutic activity. The method includes administering to a subject an effective amount of a tetracycline compound having a target therapeutic activity, such that the disease is treated.

In a further embodiment, the tetracycline compound is of formula I: 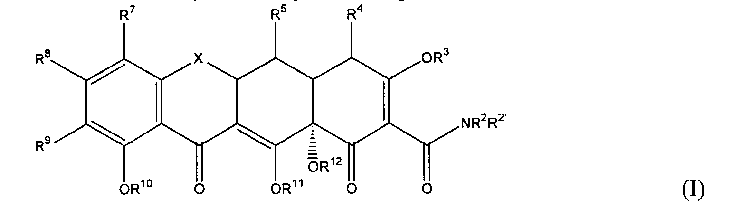 wherein
R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
R^{2'}, R³, R¹⁰, R¹¹ and R¹² are each hydrogen or a pro-drug moiety;
R⁴ is NR^{4'}R^{4"}, alkyl, alkenyl, alkynyl, hydroxyl, halogen, or hydrogen;
R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl, alkylsulfonyl, alkylamino, arylalkyl, alkyl carbonyloxy, or aryl carbonyloxy;
R⁶ and R^{6'} are each independently hydrogen, methylene, absent, hydroxyl, halogen, thiol, alkyl, alkenyl, alkynyl, aryl, alkoxy, alkylthio, alkylsulfinyl, alkylsulfonyl, alkylamino, or an arylalkyl;
R⁷ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH2)₀₋₃NR^{7c}C(=W')WR^{7a};
R⁸ is hydrogen, hydroxyl, halogen, thiol, nitro, alkyl, alkenyl, alkynyl, aryl, alkoxy, alkylthio, alkylsulfinyl, alkylsulfonyl, alkylamino, amino, arylalkenyl, arylalkynyl, acyl, aminoalkyl, heterocyclic, thionitroso, or -(CH₂)₀₋₃NR^{8c}C(=E')ER^{8a};
R⁹ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃NR^{9c}C(=Z')ZR^{9a};
R^{7a}, R^{7b}, R^{7c}, R^{7d}, R^{7e}, R^{7f}, R^{8a}, R^{8b}, R^{8c}, R^{8d}, R^{8e}, R^{9f}, R^{9a}, R^{9b}, R^{9c}, R^{9d}, R^{9e}, and R^{8f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
R¹³ is hydrogen, hydroxy, alkyl, alkenyl, alkynyl, alkoxy, alkylthio, aryl, alkylsulfinyl, alkylsulfonyl, alkylamino, or an arylalkyl;
E is CR^{8d}R^{8e}, S, NR^{8b} or O;
E' is O, NR^{8f}, or S;
W is CR^{7d}R^{7e}, S, NR^{7b} or O;
W' is O, NR^{7f}, or S;
X is CHC(R¹³Y'Y), C=CR¹³Y, CR^{6'}R⁶, S, NR⁶, or O;
Y' and Y are each independently hydrogen, halogen, hydroxyl, cyano, sulfhydryl, amino, alkyl, alkenyl, alkynyl, alkoxy, alkylthio, alkylsulfinyl, alkylsulfonyl, alkylamino, or an arylalkyl;
Z is CR^{9d}R^{9e}, S, NR^{9b} or O;
Z' is O, S, or NR^{9f}, and pharmaceutically acceptable salts, esters and enantiomers thereof.

In a further embodiment, the invention pertains, at least in part, to a method for treating an inflammatory process associated state in a subject, by administering to the subject an effective amount of a tetracycline compound.

In certain embodiments, the tetracycline is substituted at the 1, 2, 3, 4, 5, 6, 7, 8, 9, 10, 11, 11a, 12, 12a and/or 13 position. In a further embodiment, the substituted tetracycline compound is 3, 7, 9 and/or 10 substituted.

In a further embodiment, the invention pertains, at least in part, to methods for treating inflammation process associated states (IPAS) in subjects, by administering to the subject an effective amount of a tetracycline compound, such that the IPAS in the subject is treated. Examples of IPAS include, but are not limited to, diabetic complications, arteriosclerosis, atherosclerosis, etc.

In another embodiment, the invention pertains, at least in part, to a method for treating tissue wounds of a subject. The method includes contacting the subject's wound with an effective amount of a tetracycline compound.

In another embodiment, the invention also pertains, at least in part, to a method for treating ischemia or stroke in a subject. The method includes administering to a subject an effective amount of a tetracycline compound.

In yet another embodiment, the invention also pertains, at least in part, to a method for treating dry eye in a subject. The method includes administering to a subject an effective amount of a tetracycline compound.

In another embodiment, the invention also includes a method for treating acute lung injury in a subject, comprising administering to said subject an effective amount of a tetracycline compound.

In a further embodiment, the invention pertains to a method for treating a neurological disorder in a subject by administering to the subject an effective amount of a tetracycline compound, such that the neurological disorder in the subject is treated. Examples of neurological disorders include, but are not limited to, multiple sclerosis, Parkinson's disease, Huntington's disease, Alzheimer's disease, traumatic brain injury, amylotropic lateral sclerosis, spinal cord trauma, nerve damage, motor neuron disease, etc.

In another further embodiment, the invention pertains to a method for treating cancer in a subject, by administering to the subject an effective amount of a tetracycline compound, such that the cancer is treated.

In a further embodiment, the invention pertains to pharmaceutical compositions which contain a substituted tetracycline compound in combination with a second agent, *e.g.*, a chemotherapeutic agent, neuroprotective agent, and/or an anti-infective agent.

The invention also pertains, at least in parts, to a packaged composition for the treatment of disease. The packaged composition includes a tetracycline compound having target therapeutic activity and directions for using it for treatment of the disease.

The invention also pertains to pharmaceutical compositions comprising tetracycline compounds disclosed herein, as well as the tetracycline compounds *per se.*

### Detailed Description of the Invention

### 1. Methods for Treating Diseases with a Tetracycline Compound Having Target Therapeutic Activity

In one embodiment, the invention pertains, at least in part, to a method for treating a disease with a tetracycline compound having a target therapeutic activity. The method includes administering to a subject an effective amount of a tetracycline compound having a target therapeutic activity, such that the disease is treated.

The language "target therapeutic activity" ("TTA") includes activities of tetracycline compounds in a subject that differ from antibacterial and/or antiinfective activity or are in addition to antibacterial and/or antiinfective activity, but result in treatment of a disease as described herein. It should be understood that the tetracycline compound can have antibacterial and/or antiinfective activity, but the treatment of the disease occurs through a different and/or additional target therapeutic activity. Examples of target therapeutic activities include activities that allow for treatment of inflammatory process associated states (IPAS), neurological disorders (*e.g.*, neurodegenerative disorders, neuropsychiatric disorders, etc.), cancer, and other disorders which can be treated with the tetracycline compounds of the invention. Examples of specific TTAs are described in further detail below and in the Examples. Tetracycline compounds of the invention may have one or more TTAs.

The term "tetracycline compound" does not include minocycline, doxycycline, or tetracycline. The term includes substituted tetracycline compounds or compounds with a similar ring structure to tetracycline. Examples of tetracycline compounds include: chlortetracycline, oxytetracycline, demeclocycline, methacycline, sancycline, chelocardin, rolitetracycline, lymecycline, apicycline; clomocycline, guamecycline, meglucycline, mepylcycline, penimepicycline, pipacycline, etamocycline, penimocycline, etc. Other derivatives and analogues comprising a similar four ring structure are also included (See Rogalski, "Chemical Modifications of Tetracyclines," the entire contents of which are hereby incorporated herein by reference). Table 1 depicts tetracycline and several known other tetracycline derivatives.

**Table 1**

| | | |
|---|---|---|
| 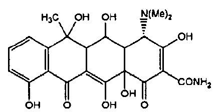 | 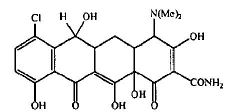 | 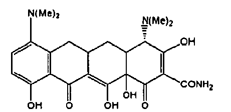 |
| Oxytetracycline | Demeclocycline | Minocycline |
| 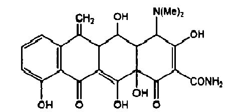 | 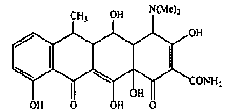 | 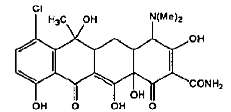 |
| Methacycline | Doxycycline | Chlortetracycline |
| 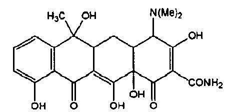 | 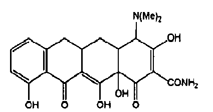 | 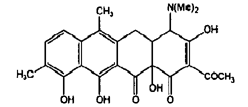 |
| Tetracycline | Sancycline | Chelocardin |

Other tetracycline compounds which may be modified using the methods of the invention include, but are not limited to, 6-demethyl-6-deoxy-4-dedimethylaminotetracycline; tetracyclino-pyrazole; 7-chloro-4-dedimethylaminotetracycline; 4-hydroxy-4-dedimethylaminotetracycline; 12a-deoxy-4-dedimethylaminotetracycline; 5-hydroxy-6a-deoxy-4-dedimethylaminotetracycline; 4-dedimethylamino-12a-deoxyanhydrotetracycline; 7-dimethylamino-6-demethyl-6-deoxy-4-dedimethylaminotetracycline; tetracyclinonitrile; 4-oxo-4-dedimethylaminotetracycline 4,6-hemiketal; 4-oxy-11a Cl-4-dedimethylaminotetracycline-4,6-hemiketal; 5a,6-anhydro-4-hydrazon-4-dedimethylamino tetracycline; 4-hydroxyimino-4-dedimethylamino tetracyclines; 4-hydroxyimino-4-dedimethylamino 5a,6-anhydrotetracyclines; 4-amino-4-dedimethylamino-5a, 6 anhydrotetracycline; 4-methylamino-4-dedimethylamino tetracycline; 4-hydrazono-11a-chloro-6-deoxy-6-demethyl-6-methylene-4-dedimethylamino tetracycline; tetracycline quaternary ammonium compounds; anhydrotetracycline betaines; 4-hydroxy-6-methyl pretetramides; 4-keto tetracyclines; 5-keto tetracyclines; 5a, 11a dehydro tetracyclines; 11a Cl-6, 12 hemiketal tetracyclines; 11a Cl-6-methylene tetracyclines; 6, 13 diol tetracyclines; 6-benzylthiomethylene tetracyclines; 7, 11a -dichloro-6-fluoro-methyl-6-deoxy tetracyclines; 6-fluoro (α)-6-demethyl-6-deoxy tetracyclines; 6-fluoro (β)-6-demethyl-6-deoxy tetracyclines;6-α acetoxy-6-demethyl tetracyclines; 6-β acetoxy-6-demethyl tetracyclines; 7, 13-epithiotetracyclines; oxytetracyclines; pyrazolotetracyclines; 11a halogens of tetracyclines; 12a formyl and other esters of tetracyclines; 5, 12a esters of tetracyclines; 10, 12a- diesters of tetracyclines; isotetracycline; 12-a-deoxyanhydro tetracyclines; 6-demethyl-12a-deoxy-7-chloroanhydrotetracyclines; B-nortetracyclines; 7-methoxy-6-demethyl-6-deoxytetracyclines; 6-demethyl-6-deoxy-Sa-epitetracyclines; 8-hydroxy-6-demethyl-6-deoxy tetracyclines; monardene; chromocycline; 5a methyl-6-demethyl-6-deoxy tetracyclines; 6-oxa tetracyclines, and 6 thia tetracyclines.

The term "tetracycline compounds" includes substituted tetracycline compounds as defined below, and as described in the specification, in Formula I, Table 2 and/or in Table 3. The tetracycline compounds may or may not have antibacterial or antiinfective activity. In certain embodiments of the invention, the tetracycline compound has antiinfective and/or antibacterial activity. In other embodiments of the invention, the tetracycline compound does not have significant antiinfective or antibacterial therapeutic activity.

The term "subject" includes animals (*e.g.*, mammals, *e.g.*, cats, dogs, horses, pigs, cows, sheep, rodents, rabbits, squirrels, bears, primates (*e.g.*, chimpanzees, gorillas, and humans)) which are capable of (or currently) suffering from a target disease, such as, but not limited to IPAS, neurological disorders, and cancer.

The language "effective amount" of the tetracycline compound is that amount necessary or sufficient to treat or prevent a target disease of the invention such as, for example, an IPAS, a neurological disorder, or cancer in a subject, *e.g.* prevent the various morphological and somatic symptoms of the particular disease. The effective amount can vary depending on such factors as the size and weight of the subject, the type of illness, or the particular tetracycline compound. For example, the choice of the tetracycline compound can affect what constitutes an "effective amount". One of ordinary skill in the art would be able to study the aforementioned factors and make the determination regarding the effective amount of the tetracycline compound without undue experimentation.

The regimen of administration can affect what constitutes an effective amount. The tetracycline compound can be administered to the subject either prior to or after the onset of a disease which is treatable. Further, several divided dosages, as well as staggered dosages, can be administered daily or sequentially, or the dose can be continuously infused, orally administered, administered by inhalation, or can be a bolus injection. Further, the dosages of the tetracycline compound(s) can be proportionally increased or decreased as indicated by the exigencies of the therapeutic or prophylactic situation.

The term "target disease" includes diseases or disorders which may be treated and/or prevented by the administration of a tetracycline compound having target therapeutic activity. Examples of target diseases include, but are not limited to, IPAS, neurological disorders, and cancer.

The term "treated," "treating" or "treatment" includes therapeutic and/or prophylactic treatment. The treatment includes the diminishment or alleviation of at least one symptom associated or caused by the state, disorder or disease being treated. For example, treatment can be diminishment of one or several symptoms of a disorder or complete eradication of a disorder.

In one embodiment, the invention pertains to a method for treating a disease (*e.g.,* an IPAS, a neurological disorder, cancer, *etc.*) in a subject, by administering to said subject an effective amount of a tetracycline compound such that said disease is treated.

In one embodiment, the tetracycline compound used in any of the methods is an anti-infective and/or anti-microbial. In another, the tetracycline compound used in any one of the above described methods is not anti-infective and/or anti-microbial.

The term "antiinfective" includes antibacterial, antimicrobial, antifungal, antiparasitic, antibiotic, and antiviral activities of the tetracycline compounds. For example, an antiinfective tetracycline compound includes compounds that reduce the ability of a microbe to produce infection in a host or that reduces the ability of a microbe to multiply or remain infective in an environment. Antiinfective tetracycline compounds include those compounds that are static or cidal for microbes, e.g., an antimicrobial compound that inhibits proliferation and/or viability of a microbe. The antiinfective tetracycline compounds include compounds that increase susceptibility of microbes to the tetracycline compound or another agent, e.g., antibiotic, or decrease the infectivity or virulence of a microbe. The antiinfective properties of tetracycline compounds of the invention can be determined by using assays known in the art as well as the assays described herein.

In another embodiment, the invention pertains to methods for treating diseases with tetracycline compounds having target therapeutic activity, by administering an effective amount of a tetracycline compound having target therapeutic activity in combination with a second agent.

The language "in combination with" a second agent or treatment includes coadministration of the tetracycline compound, and with the second agent or treatment, administration of the tetracycline compound first, followed by the second agent or treatment and administration of the second agent or treatment first, followed by the tetracycline compound. The second agent may be any agent which is known in the art to treat, prevent, or reduce the symptoms of a target disease, such as, for example, IPAS, neurological disorder, cancer, etc. Furthermore, the second agent may be any agent of benefit to the patient when administered in combination with the administration of an tetracycline compound. Examples of second agents include chemotherapeutic agents, neuroprotective agents, and antiinfective agents, as described below.

### A. Inflammatory Process Associated States

In one embodiment, the invention pertains to a method for treating an inflammatory process associated state (IPAS) in a subject. The method includes administering to a subject an effective amount of a tetracycline compound of formula (I), such that the inflammatory process associated state is treated.

The term "inflammatory process associated state" or "IPAS" includes states in which inflammation or inflammatory factors (e.g., matrix metalloproteinases (MMPs), nitric oxide (NO), TNF, interleukins, plasma proteins, cellular defense systems, cytokines, lipid metabolites, proteases, toxic radicals, mitochondria, apoptosis, adhesion molecules, *etc*.) are involved or are present in an area in aberrant amounts, e.g., in amounts which may be advantageous to alter, e.g., to benefit the subject. The inflammatory process is the response of living tissue to damage. The cause of inflammation may be due to physical damage, chemical substances, micro-organisms, tissue necrosis, cancer or other agents. Acute inflammation is short-lasting, lasting only a few days. If it is longer lasting however, then it may be referred to as chronic inflammation.

Not to be limited by theory, it is believed that tetracycline compounds may treat inflammatory disorders in subjects by direct inhibition or inhibition of production of secretions of MMPs, nitric oxide (NO), tumor necrosis factor (TNF), and/or other factors associated with inflammatory processes. Inflammatory disorders include both acute inflammatory disorders, chronic inflammatory disorders, and recurrent inflammatory disorders. Acute inflammatory disorders are generally of relatively short duration, and last for from about a few minutes to about one to two days, although they may last several weeks. The main characteristics of acute inflammatory disorders include increased blood flow, exudation of fluid and plasma proteins (edema) and emigration of leukocytes, such as neutrophils. Chronic inflammatory disorders, generally, are of longer duration, *e.g.*, weeks to months to years or even longer, and are associated histologically with the presence of lymphocytes and macrophages and with proliferation of blood vessels and connective tissue. Recurrent inflammatory disorders include disorders which recur after a period of time or which have periodic episodes. Examples of recurrent inflammatory disorders include asthma and multiple sclerosis. Some disorders may fall within one or more categories.

Inflammatory disorders are generally characterized by heat, redness, swelling, pain and loss of function. Examples of causes of inflammatory disorders include, but are not limited to, microbial infections (*e.g.*, bacterial, viral and fungal infections), physical agents (*e.g.*, burns, radiation, and trauma), chemical agents (*e.g.*, toxins and caustic substances), tissue necrosis and various types of immunologic reactions. NO is believed to be one of a number of reactive products produced in the immune and inflammatory responses to such insults. In particular, elevated levels of NO production common to chronic inflammation are a likely contributor to the non-specific tissue destruction often seen in such conditions.

Examples of inflammatory disorders include, but are not limited to, osteoarthritis, rheumatoid arthritis, acute and chronic infections (bacterial, viral and fungal); acute and chronic bronchitis, sinusitis, and other respiratory infections, including the common cold; acute and chronic gastroenteritis and colitis; acute and chronic cystitis and urethritis; acute respiratory distress syndrome; cystic fibrosis; acute and chronic dermatitis; acute and chronic conjunctivitis; acute and chronic serositis (pericarditis, peritonitis, synovitis, pleuritis and tendinitis); uremic pericarditis; acute and chronic cholecystis; acute and chronic vaginitis; acute and chronic uveitis; drug reactions; insect bites; burns (thermal, chemical, and electrical); and sunburn.

The term "NO associated state" includes states which involve or are associated with nitric oxide (NO) or inducible nitric oxide synthase (iNOS). NO associated state includes states which are characterized by aberrant amounts of NO and/or iNOS. Preferably, the NO associated state can be treated by administering tetracycline compounds of the invention, e.g., compounds of formula I. In certain embodiments, the invention includes 1, 2, 3, 4, 5, 6, 7, 8, 9, 10, 11, 11a, 12, 12a and/or 13 substituted tetracycline compounds. In other embodiments, the compounds described in U.S. Patents Nos. 6,231,894; 6,015,804; and 5,789,395 are not included. The entire contents of each of these patents are hereby incorporated herein by reference. In other embodiments, minocycline is not included.

Other examples of NO associated states include, but are not limited to, malaria, senescence, diabetes, vascular stroke, neurodegenerative disorders (*e.g.*, Alzheimer's disease, Huntington's disease, amylotropic lateral sclerosis, etc.), cardiac disease (*e.g.*, re-perfusion-associated injury following infarction), juvenile diabetes, inflammatory disorders, osteoarthritis, rheumatoid arthritis, acute and chronic infections (*e.g.*, bacterial, viral and fungal); restenosis; acute and chronic bronchitis, sinusitis, and other respiratory infections, including the common cold; acute and chronic gastroenteritis and colitis; acute and chronic cystitis and urethritis; hepatitis; acute and chronic dermatitis; acute and chronic conjunctivitis; acute and chronic serositis (pericarditis, peritonitis, synovitis, pleuritis and tendinitis); uremic pericarditis; acute and chronic cholecystis; acute and chronic vaginitis; acute and chronic uveitis; drug reactions; insect bites; burns (thermal, chemical, and electrical); and sunburn.

The term "inflammatory process associated state" also includes, in one embodiment, matrix metalloproteinase associated states (MMPAS). MMPAS include states characterized by aberrant amounts of MMPs or MMP activity.

Matrix metalloproteinases (MMP's) are believed to damage a subject's connective tissue and basement membranes as a complication of the inflammatory and/or immune response and other disease processes, such as cancer cell invasion and metastasis. MMP's are generally zinc and calcium-dependent for hydrolytic cleavage of substrate proteins and are secreted or released by a variety of host cells, such as, polymorphonuclear neutrophils (PMN's), macrophages, bone cells, epithelium and fibroblasts.

MMP's are also expressed during physiological processes such as wound repair, reproduction, tissue growth and remodeling. Examples of matrix metalloproteinase associated states ("MMPAS's'') include, but are not limited to, arteriosclerosis, corneal ulceration, emphysema, osteoarthritis, multiple sclerosis(Liedtke et al., Ann. Neurol. 1998, 44:35-46; Chandler et al., J. Neuroimmunol.1997, 72:155-71), osteosarcoma, osteomyelitis, bronchiectasis, chronic pulmonary obstructive disease, skin and eye diseases, periodontitis, osteoporosis, rheumatoid arthritis, ulcerative colitis, cystic fibrosis, inflammatory disorders, tumor growth and invasion (Stetler-Stevenson et al., Annu. Rev. Cell Biol. 1993, 9:541-73; Tryggvason et al., Biochim. Biophys. Acta 1987, 907:191-217; Li et al., Mol. Carcinog. 1998, 22:84-89),metastasis, acute lung injury, stroke, ischemia, diabetes, aortic or vascular aneurysms, skin tissue wounds, dry eye, bone and cartilage degradation (Greenwald et al., Bone 1998, 22:33-38; Ryan et al., Curr. Op. Rheumatol. 1996, 8;238-247).

In one embodiment, the tetracycline compounds of the invention do not include those described in U.S. Pat. Nos. 5,459,135; 5,321,017; 5,308,839; 5,258,371; 4,935,412; 4,704,383, 4,666,897, and RE 34,656, incorporated herein by reference in their entirety.

In another embodiment, the IPAS is diabetes or diabetic complications, *e.g.*, juvenile diabetes, diabetes mellitus, diabetes type I, diabetes type II, or complications associated with anyone of the aforementioned states such as diabetic ulcers. In a further embodiment, protein glycosylation is not affected by the administration of the tetracycline compounds. In another embodiment, the tetracycline compound of the invention is administered in combination with standard diabetic therapies, such as, be not limited to insulin therapy. In a further embodiment, the tetracycline compounds used to treat diabetes do not include those compounds described in U.S. Patents Nos 5,929,055; and 5,532,227, incorporated herein by reference in their entirety.

In another embodiment, the IPAS disorder is a bone mass disorder. Bone ma disorders include disorders where a subjects bones are disorders and states where the formation, repair or remodeling of bone is advantageous. For example, bone mass disorders include osteoporosis (*e.g.*, a decrease in bone strength and density), bone fractures, bone formation associated with surgical procedures (*e.g.*, facial reconstruction), osteogenesis imperfecta (brittle bone disease), hypophosphatasia, Paget's disease, fibrous dysplasia, osteopetrosis, myeloma bone disease, and the depletion of calcium in bone, such as that which is related to primary hyperparathyroidism. Bone mass disorders include all states in which the formation, repair or remodeling of bone is advantageous to the subject as well as all other disord associated with the bones or skeletal system of a subject which can be treated with the tetracycline compounds of the invention.

In a further embodiment, the tetracycline compounds of the invention used tc treat bone mass disorders do not include U.S. Patents Nos. 5,459,135; 5,231,017; 5,998,390; 5,770,588; RE 34,656; 5,308,839; 4,925,833; 3,304,227; and 4,666,897,0 of which is hereby incorporated herein by reference in its entirety.

In another embodiment, the IPAS disorder is acute lung injury. Acute lung injuries include acute respiratory distress syndrome (ARDS), adult respiratory distre syndrome, post-pump syndrome (PPS), and trauma. Trauma includes any injury to living tissue caused by an extrinsic agent or event. Examples of trauma include, but not limited to, crush injuries, contact with a hard surface, or cutting or other damage the lungs.

The invention also pertains to a method for treating acute lung injury by administering a tetracycline compound (*e.g.*, a 1, 2, 3, 4, 5, 6, 7, 8, 9, 10, 11, 11a, 12 12a and/or 13 substituted tetracycline compound).

The invention also includes methods for treating chronic lung disorders by administering a tetracycline compound, such as those described herein. The method includes administering to a subject an effective amount of a tetracycline compound s that the chronic lung disorder is treated. Examples of chronic lung disorders include but are not limited, to asthma, cystic fibrosis, and emphysema.

In a further embodiment, the tetracycline compounds of the invention used to treat acute and/or chronic lung disorders do not include those described in U.S. Patents No. 5,977,091; 6,043,231; 5,523,297; and 5,773,430, each of which is hereby incorporated herein by reference in its entirety.

In yet another embodiment, the IPAS disorder is ischemia, stroke, or ischemic stroke. The invention also pertains to a method for treating ischemia, stroke, or ischemic stroke by administering an effective amount of a tetracycline compound of the invention (*e.g.*, a 1, 2, 3, 4, 5, 6, 7, 8, 9, 10, 11, 11a, 12, 12a and/or 13 substituted tetracycline compound). In a further embodiment, the tetracycline compounds used to treat ischemia, stroke, or ischemic stroke do not include minocycline, or the compounds described in U.S. Patents No. 6,231,894; 5,773,430; 5,919,775 or 5,789,395, incorporated herein by reference.

In another embodiment, the IPAS is a skin wound. The method pertains, at least in part, to a method for improving the healing response of the epithelialized tissue (*e.g.*, skin, mucusae) to acute traumatic injury (*e.g.*, cut, bum, scrape, *etc.*). The method may include using a tetracycline compound of the invention (which may or may not have antibacterial activity) to improve the capacity of the epithelialized tissue to heal acute wounds. The method may increase the rate of collagen accumulation of the healing tissue. The method may also decrease the proteolytic activity in the epthithelialized tissue by decreasing the collagenolytic and/or gelatinolytic activity of MMPs. In a further embodiment, the tetracycline compound of the invention is administered to the surface of the skin (*e.g.*, topically).

In a further embodiment, the tetracycline compound of the invention used to treat a skin wound does not include those described in U.S. Patent Nos. 5,827,840; 4,704,383; 4,935,412; 5,258,371; 5,308,8391 5,459,135; 5,532,227; or 6,015,804; each of which is incorporated herein by reference in its entirety. In a further embodiment, the tetracycline compound is substituted at the 1, 2, 3, 4, 5, 6, 7, 8, 9, 10, 11, 11a, 12, 12a and/or 13 position.

In yet another embodiment, the IPAS is an aortic or vascular aneurysm in vascular tissue of a subject (*e.g.*, a subject having or at risk of having an aortic or vascular aneurysm, *etc*.). The tetracycline compound may by effective to reduce the size of the vascular aneurysm or it may be administered to the subject prior to the onset of the vascular aneurysm such that the aneurysm is prevented. In one embodiment, the vascular tissue is an artery, *e.g.*, the aorta, *e.g.*, the abdominal aorta. In a further embodiment, the tetracycline compound of the invention used to treat the aortic of vascular aneurysm is not described in U.S. Patent Nos. 6,043,225 or 5,834,449, incorporated herein by reference in their entirety.

In yet another embodiment, the invention pertains to a method for treating dry eye or other eye disorders in a subject, by administering an effective amount of a tetracycline compound, *e.g.*, a compound of formula I, *e.g.*, a 1, 2, 3, 4, 5, 6, 7, 8, 9, 10, 11, 11a, 12, 12a and/or 13 substituted tetracycline compound tetracycline compound. In a further embodiment, the tetracycline compound of the invention used to treat dry eye is not described in U.S. Patent No. 5,308,624 nor 5,698,533, incorporated herein by reference in their entirety.

The ability of a tetracycline compound to treat an IPAS associated disorder can be determined through the use of assays and screening methods known in the art. For example, one art recognized *in vitro* method for determining the anti-inflammatory effects by the inhibition of nitric oxide and IL-12 synthesis is described in D'Agostino, P. et al. Int Immunopharmacol. 2001 Sep;1(9-10):1765-76. The LSMA assay, described in Example 4, may also be used. In one embodiment of the invention, the substituted tetracycline compounds of the invention inhibit nitric oxide synthesis better than doxycycline, as determined by the assay. In a further embodiment, the substituted tetracycline compounds of the invention inhibit nitric oxide synthesis 10% or greater, 25% or greater, 30% or greater, 35% or greater, 40% or greater, 45% or greater, 50% or greater, 55% or greater, 60% or greater, 65% or greater, 70% or greater, 75% or greater, 80% or greater, 85% or greater, 90% or greater, 95% or greater, or 100% or greater better than doxycycline.

In a further embodiment, the IPAS is a state which is associated with an infection such as hepatitis (*e.g.*, viral hepatitis) or sinusitis (*e.g.*, chronic sinusitis). The methods of the invention may comprise administering the tetracycline compound of the invention in combination with an antiinfective agent. The antiinfective agent may be an antiinfective tetracycline or another antiinfective agent known in the art to treat viral, fungal, parasitic or bacterial infections.

The compounds of the invention may also be tested *in vivo* for treatment of IPAS disorders. The substituted tetracycline compounds of the invention may be tested for use in the treatment of IPAS disorders using many known assays and models.

For example, the tetracycline compounds of the invention may be tested *in vivo* for effectiveness in treating aortic aneurysisms (*e.g.*, using the model described in Curci, et al. J. Vasc. Surg. 2000; 31: 326-342 or the model described in Example 17); diabetic complications (*e.g.*, using the model described in Ryan et al. Curr. Med. Chem. 2001;8(3):305-316 or in Example 18); arteriosclerosis, such as atherosclerosis (*e.g.*, using the model described in Bendeck, et al. Amer. J. Path. 2001:160(3): 1089-1095 or the model described in Example 19); acute respiratory distress syndrome (ARDS, *e.g.*, using the model described in Carney et al. Circulation. 1999 Jul 27;100(4):400-6, or in the assay described in Example 20); septic shock (*e.g.*, using the model described in Antimicrob Agents Chemother. 1997 Jan;41(1):117-21, Shapira et al. Infect Immun. 1996 Mar;64(3):825-8, or the model described in Example 21); wound healing (*e.g.*, using the model described in Pirila, et al. Curr. Med. Chem. 2001;8:281-294 or the model described in Example 22), arthritis, osteoporosis (*e.g.*, using the model described in Ramamurthy, et al. Curr. Met Chem. 2001;8:295-303 or the model described in Example 24), or other IPAS disorders using art recognized techniques. The efficacy of the compounds of the invention for the treatment of dry eye syndrome can be tested using the procedure outline in Solomon et al. Invest. Opthamol. & Visual Science. 2000:41(9); 2544-2557; Sobrin et al. Invest. Opthamol. & Visual Science. 2000: 41(7): 1703-1709).

In a further embodiment, the tetracycline compounds of the invention are found to be effective for the treatment for at least one of the above mentioned disorders using one of the listed models or assays or by using other techniques known in the art to determine efficacy.

### B. Neurological Disorders and Neuroprotection

In one embodiment, the invention pertains to methods for treating neurological disorders using tetracycline compounds having target activity. The method includes administering to a subject an effective amount of a tetracycline compound, such that the neurological disorder is treated.

Examples of neurological disorders include both neuropsychiatric and neurodegenerative disorders, but are not limited to, such as Alzheimer's disease, dementias related to Alzheimer's disease (such as Pick's disease), Parkinson's and other Lewy diffuse body diseases, senile dementia, Huntington's disease, Gilles de la Tourette's syndrome, multiple sclerosis, amylotropic lateral sclerosis (ALS), progressive supranuclear palsy, epilepsy, and Creutzfeldt-Jakob disease; autonomic function disorders such as hypertension and sleep disorders, and neuropsychiatric disorders, such as depression, schizophrenia, schizoaffective disorder, Korsakoff's psychosis, mania, anxiety disorders, or phobic disorders; learning or memory disorders, *e.g.*, amnesia or age-related memory loss, attention deficit disorder, dysthymic disorder, major depressive disorder, mania, obsessive-compulsive disorder, psychoactive substance use disorders, anxiety, phobias, panic disorder, as well as bipolar affective disorder, *e.g.*, severe bipolar affective (mood) disorder (BP-1), bipolar affective neurological disorders, *e.g.*, migraine and obesity, and traumatic brain injury. Further neurological disorders include, for example, those listed in the American Psychiatric Association's Diagnostic and Statistical Manual of Mental Disorders (DSM), the most current version of which is incorporated herein by reference in its entirety.

In one embodiment, the tetracycline compounds of the invention used to treat neurological disorders include substituted tetracycline compounds which may be further substituted at the 1, 2, 3, 4, 5, 6, 7, 8, 9, 10, 11, 11a, 12, 12a and/or 13 position.

Examples of neuroprotective agents that can be administered in combination with the tetracycline compounds of the invention to treat neurological disorders include, but are not limited to, compounds that remove protein build up (*e.g.*, geldanamycin), anti-inflammatory agents (*e.g.*, glucocorticoids, non-steroidal anti-inflammatory drugs (*e.g.*, ibuprofin, aspirin, *etc*.), omega-3 fatty acids (*e.g.,* EPA, DHA, *etc*.), minocycline, dexanabionol, *etc*.), compounds that increase energy available to cells (*e.g.*, creatine, creatine phosphate, dichloroacetate, nicotinamide, riboflavin, carnitine, *etc*.), antioxidants (*e.g.*, plant extracts (*e.g.*, gingko biloba), co-enzyme Q-10, vitamin E (alpha-tocopherol), vitamin C (ascorbic acid), vitamin A (beta-carotene), selenium, lipoic acid, selegine, *etc*.), anti-glutamate therapies (*e.g.*, remacemide, riluzole, lamotrigine, gabapentin, *etc*.), GABA-ergic therapies (*e.g.*, baclofen, muscimol, *etc*.), gene transcription regulators (*e.g.*, glucocorticoids, retinoic acid, *etc*.), erythropoietin, TNF-α antagonists, cholinesterase inhibitors, N-methyl-D-aspartate (NMDA) antagonists, opiod antagonists, neuronal membrane stabilizers (*e.g.*, CDP-choline, *etc*.), calcium and sodium channel blockers, prednisone, *etc.*

Examples of *in vitro* models which can be used to identify tetracyclines which have neuroprotection activity include the NSNA described in Example 6. Other assays which can be used include those described in Shukla C et al., Neuropathol Appl Neurobiol. 2002 Mar;28(2):169 and Zhu S, et al. Nature 2002 May 2;417(6884):74-8. In a further embodiment, the tetracycline compounds of the invention are found to have neuroprotective activity as measured by the NSNCL assay.

Another example of an *in vitro* model is the NE Assay (NMDA exposure assay) which measures the protection of cultured cortical neurons from excitotoxic injury induced by NMDA exposure by tetracycline compounds. This assay is described in Example 5 and a similar model is described in Tikka, TM et al. J Immunol. 2001 Jun 15;166(12):7527-33. In a further embodiment, the tetracycline compounds of the invention are found to protect cultured cortical neurons as determined by the NE assay.

The ability of tetracycline compounds to protect dopaminergic cells can be determined by using the assay described in Example 7 (*In vitro* Parkinson's Disease Assay), or in Le, W et al. J Neurosci. 2001 Nov 1;21(21):8447-55. This assay can be used to determine the ability of the tetracycline compounds to treat Parkinson's disease. Microglial activation and oxidative stress are components of the pathology of Parkinson's disease (PD). The neuroprotective qualities of tetracycline compounds can be assessed using an *in vitro* model of nigral injury. In this model, lipopolysaccharide-induced microglial activation leads to injury of a dopaminergic cell line (MES 23.5 cells) and dopaminergic neurons in primary mesencephalic cell cultures. In an embodiment, the tetracycline compounds of the invention are able to protect dopaminergic cells as tested in the *in vitro* Parkinson's Disease Assay.

The tetracycline compounds of the invention may also be tested in *in vitro* models for inhibition of cytochrome C release (CCR Assay). Examples of such assays are described in Example 8 and in the literature (*e.g.*, Zhu S. et al. Nature. 2002 May 2;417(6884):74-8). In a further embodiment, the tetracycline compounds of the invention are determined to inhibit the cytochrome C release as measured by the CCR Assay. Other *in vitro* assays that can be used to test the efficacy of the tetracycline compounds of the invention to treat particular states include the Motor Neuron Disease Assay described in Example 25 or the assay described in Tikka et al. Brain. 2002:125(4):722-731.

The tetracycline compounds can also be tested for neuroprotective and ability to treat neurological diseases *in vivo.* For example, the ability of the tetracycline compounds to treat neurological disorders can be determined using *in vivo* models for amylotropic lateral sclerosis (*e.g.*, Example 9 or as described in Zhu S et al. Nature. 2002 May 2; 417 (6884):74-8), Huntington's disease (*e.g.*, Example 10, or as described in Chen, M. et al. Nat Med. 2000 Jul;6(7):797-801); Parkinson's disease (*e.g.*, Example 11, or as described in Wu, D.C. et al. J Neurosci. 2002 Mar 1;22(5):1763-71; or Du, Y. et al. PNAS 2001 Dec 4;98(25):14669-74); Multiple Sclerosis (*e.g.*, Example 12, or as described in Brundula V. et al. Brain. 2002 Jun;125(Pt 6):1297-308 or Popovic N. et al. Ann Neurol. 2002 Feb;51(2):215-23); stroke (*e.g.*, Example 13, or as described in Yrjanheikki, J. et al. PNAS 1998 Dec 22;95(26):15769-74 or Yrjanheikki, J. et al. PNAS 1999 Nov 9;96(23):13496-500); or traumatic brain injury (*e.g.*, Example 23, or as described in Meijia, et al. Neurosurgery. 2001:48(6):1393-1399). In a further embodiment, the invention pertains to tetracycline compounds of the invention which are found to be effective for treatment in at least one of the above referenced models.

In one embodiment, the tetracycline compound for the treatment of the neurological disorder is not one described in U.S. 6,277,393; WO 02/20022; WO 99/30720; or U.S. 6,319,910. In another embodiment, the tetracycline compound is not a compound described in US 20010014670, when the neurological disorder is Alzheimer's disease. In a further embodiment, the tetracycline compound is not a compound described in US 20020022608A1, when the neurological disorder is multiple sclerosis. The contents of each of these references are hereby incorporated herein by reference. In another embodiment, the tetracycline compound is not minocycline.

In a further embodiment, the tetracycline compounds of the invention are found to be effective for the treatment for at least one of the above mentioned disorders using one of the listed models or assays or by using other techniques known in the art to determine efficacy.

### C. Cancer and Related Disorders

In another embodiment, the target disease is cancer. In an embodiment, the invention pertains, at least in part, to methods for treating cancer in a subject by administering to the subject an effective amount of a tetracycline compound, such that the cancer in said subject is treated.

Examples of cancers which the tetracycline compounds of the invention may be useful to treat include all solid tumors, *i.e.*, carcinomas *e.g.*, adenocarcinomas, and sarcomas. Adenocarcinomas are carcinomas derived from glandular tissue or in which the tumor cells form recognizable glandular structures. Sarcomas broadly include tumors whose cells are embedded in a fibrillar or homogeneous substance like embryonic connective tissue. Examples of carcinomas which may be treated using the methods of the invention include, but are not limited to, carcinomas of the prostate, breast, ovary, testis, lung, colon, and breast. The methods of the invention are not limited to the treatment of these tumor types, but extend to any solid tumor derived from any organ system. Examples of treatable cancers include, but are not limited to, colon cancer, bladder cancer, breast cancer, melanoma, ovarian carcinoma, prostatic carcinoma, lung cancer, and a variety of other cancers as well. The methods of the invention also cause the inhibition of cancer growth in adenocarcinomas, such as, for example, those of the prostate, breast, kidney, ovary, testes, and colon.

In an embodiment, the invention pertains to a method for treating a subject suffering or at risk of suffering from cancer, by administering an effective amount of a tetracycline compound, such that inhibition cancer cell growth occurs, i.e., cellular proliferation, invasiveness, metastasis, or tumor incidence is decreased, slowed, or stopped. The inhibition may result from inhibition of an inflammatory process, down-regulation of an inflammatory process, some other mechanism, or a combination of mechanisms. Alternatively, the tetracycline compounds may be useful for preventing cancer recurrence, for example, to treat residual cancer following surgical resection or radiation therapy. In a further embodiment, the compounds of the invention may be administered in combination with standard cancer therapy, such as, but not limited to, chemotherapeutic agents and radiation therapy.

The language "chemotherapeutic agent" is intended to include chemical reagents which inhibit the growth of proliferating cells or tissues wherein the growth of such cells or tissues is undesirable or otherwise treat at least one resulting symptom of such a growth. Chemotherapeutic agents are well known in the art (see *e.g.*, Gilman A.G., et al., The Pharmacological Basis of Therapeutics, 8th Ed., Sec 12:1202-1263 (1990)), and are typically used to treat neoplastic diseases. Examples of chemotherapeutic agents include: bleomycin, docetaxel (Taxotere), doxorubicin, edatrexate, etoposide, finasteride (Proscar), flutamide (Eulexin), gemcitabine (Gemzar), goserelin acetate (Zoladex), granisetron (Kytril), irinotecan (Campto/Camptosar), ondansetron (Zofran), paclitaxel (Taxol), pegaspargase (Oncaspar), pilocarpine hydrochloride (Salagen), porfimer sodium (Photofrin), interleukin-2 (Proleukin), rituximab (Rituxan), topotecan (Hycamtin), trastuzumab (Herceptin), tretinoin (Retin-A), Triapine, vincristine, and vinorelbine tartrate (Navelbine).

Other examples of chemotherapeutic agents include alkylating drugs such as Nitrogen Mustards (*e.g.*, Mechlorethamine (HN₂), Cyclophosphamide, Ifosfamide, Melphalan (L-sarcolysin), Chlorambucil, *etc*.); ethylenimines, methylmelamines (*e.g.,* Hexamethylmelamine, Thiotepa, *etc*.); Alkyl Sulfonates (*e.g.*, Busulfan, *etc*.), Nitrosoureas (*e.g.*, Carmustine (BCNU), Lomustine (CCNU), Semustine (methyl-CCNU), Streptozocin (streptozotocin), *etc*.), triazenes (*e.g.*, Decarbazine (DTIC; dimethyltriazenoimi-dazolecarboxamide)), Alkylators (*e.g.*, cisdiamminedichloroplatinum II (CDDP)), *etc.*

Other examples of chemotherapeutic agents include antimetabolites such as folic acid analogs (*e.g.*, Methotrexate (amethopterin)); pyrimidine analogs (*e.g.*, fluorouracil ('5-fluorouracil; 5-FU); floxuridine (fluorode-oxyuridine); FUdr; Cytarabine (cyosine arabinoside), *etc*.); purine analogs (*e.g.*, Mercaptopurine (6-mercaptopurine; 6-MP); Thioguanine (6-thioguanine; TG); and Pentostatin (2'-deoxycoformycin)), *etc.*

Other examples of chemotherapeutic agents also include vinca alkaloids (*e.g.*, Vinblastin (VLB) and Vincristine); topoisomerase inhibitors (*e.g.*, Etoposide, Teniposide, Camptothecin, Topotecan, 9-amino-campotothecin CPT-11, *etc*.); antibiotics (*e.g.*, Dactinomycin (actinomycin D), adriamycin, daunorubicin, doxorubicin, bleomycin, plicamycin (mithramycin), mitomycin (mitomycin C), Taxol, Taxotere, *etc*.); enzymes (*e.g.*, L-Asparaginase); and biological response modifiers (*e.g.,* interferon-; interleukin 2, *etc*.). Other chemotherapeutic agents include cisdiaminedichloroplatinum II (CDDP); Carboplatin; Anthracendione (*e.g.*, Mitoxantrone); Hydroxyurea; Procarbazine (N-methylhydrazine); and adrenocortical suppressants (*e.g.*, Mitotane, aminoglutethimide, *etc*.).

Other chemotherapeutic agents include adrenocorticosteroids (*e.g.*, Prednisone); progestins (*e.g.*, Hydroxyprogesterone caproate,; Medroxyprogesterone acetate, Megestrol acetate, *etc*.); estrogens (*e.g.*, diethylstilbestrol; ethenyl estradiol, *etc*.); antiestrogens (*e.g.* Tamoxifen, *etc*.); androgens (*e.g.*, testosterone propionate, Fluoxymesterone, *etc*.); antiandrogens (*e.g.*, Flutamide); and gonadotropin-releasing hormone analogs (*e.g.*, Leuprolide).

The language "radiation therapy" includes the application of a genetically and somatically safe level of x-rays, both localized and non-localized, to a subject to inhibit, reduce, or prevent symptoms or conditions associated with cancer or other undesirable cell growth. The term "x-rays" includes clinically acceptable radioactive elements and isotopes thereof, as well as the radioactive emissions therefrom. Examples of the types of emissions include alpha rays, beta rays including hard betas, high energy electrons, and gamma rays. Radiation therapy is well known in the art (see *e.g.*, Fishbach, F., Laboratory Diagnostic Tests, 3rd Ed., Ch. 10: 581-644 (1988)), and is typically used to treat neoplastic diseases.

In one embodiment, the tetracycline compounds for treating cancer do not include, for example the tetracycline compounds described in U.S. Patent Nos. 6,100,248; 5,843,925; 5,837,696; 5,668,122; WO 98/31224; US 20020045603; WO 99/49871; WO 01/87823; WO 00/28983; U.S. 5,574,026; , incorporated herein by reference in their entirety.

In a further embodiment, the tetracycline compound of the invention is administered in a dosage effective to inhibit the enzymatic activity of at least one matrix metalloproteinase, such as collagenase or gelatinase (*e.g.*, gelatinase A or gelatinase B) associated with cancerous tumors (*e.g.*, neoplasms) in the subject, *e.g.*, a mammal.

In a further embodiment, the tetracycline compounds of the invention are found to modulate angiogenesis as determined by the Rabbit Cornea Angiogenesis Model described in Example 14. Other *in vitro* assays which can be used to determine the ability of the test tetracycline compounds of the invention's ability to inhibit angiogenesis include those described in Tamargo R.J. et al. Cancer Res. 1991 Jan 15;51(2):672-5 and Masumori N et al. Adv Dent Res. 1998 Nov;12(2):111-3. Another *in vitro* assay which can be used to determine the ability of a test compound to modulate undesired cell growth, include, for example, the *In vitro* Cancer Assay, described in Example 15. In another embodiment, the tetracycline compounds of the invention are found to inhibit or decrease tube formation as determined by the *In vitro* Cancer Assay.

In another embodiment, the tetracycline compounds of the invention are found to impair or prevent de novo tumor growth. The ability of the tetracycline compounds of the invention to impair or prevent de novo tumor growth can be determined, for example, by the assay described in Example 16, or by using assays described in the literature, such as, for example, Parangi S. et al. PNAS 1996 Mar 5;93(5):2002-7 or Seftor RE et al. Clin Exp Metastasis. 1998 Apr;16(3):217-25.

### 2. Substituted Tetracycline Compounds and Methods for their Synthesis

The term "substituted tetracycline compound" includes tetracycline compounds with one or more additional substituents, *e.g.*, at the 1, 2, 3, 4, 5, 6, 7, 8, 9, 10, 11, 11a, 12, 12a or 13 position or at any other position which allows the substituted tetracycline compound of the invention to perform its intended function, *e.g.*, treat target diseases such as IPAS, neurological disorders, and cancer.

Examples of substituted tetracycline compounds include compounds described in U.S. Patents Nos. 6,165,999; 5,834,450; 5,886,175; 5,567,697; 5,567,692; 5,530,557; 5,512,553; 5,430,162each of which is incorporated herein by reference in its entirety. Other examples of substituted tetracycline compounds include those described in, for example, WO 99/37307, WO 02/12170, WO 02/04407, WO 02/04406, WO 02/04404, WO 01/98260, WO 01/98259, WO 01/98236, WO 01/87824, WO 01/74761, WO 01/52858, WO 01/19784, WO 84/01895, U.S.S.N. 60/367,050, U.S.S.N. 09/895,797, U.S.S.N. 60/305,546, U.S.S.N. 60/346,930, U.S.S.N. 60/346,929, U.S.S.N. 60/347,065, U.S.S.N. 60/346,956, U.S.S.N. 60/367,049, U.S.S.N. 10/097,095, U.S.S.N. 10/097,135, U.S.S.N. 60/362,654, U.S.S.N. 60/367,045, U.S.S.N. 60/366,915, and U.S.S.N. 60/367,048. Other examples of substituted tetracycline compounds are described in EP 0582810 B1;EP 0536 515B1; EP 0582 789B1; EP 0582 829B1; EP 0582788B1; US 5,530,117; US 5,495,030; US 5,495,018; US 5,494,903; US 5,466,684; EP 0535 346B1; US 5,457,096; US 5,442,059; US 5,430,162; US 5,420,272; US 5,401,863; US 5,401,729; US 5,386,041; US 5,380,888; US 5,371,076; EP 618 190; US 5,326,759; EP 582 829; EP 528 810; EP 582 790; EP 582 789; EP 582 788; US 5,281,628; EP 536 515; EP 535 346; WO 96/34852; WO 95/22529A1; US 4,066,694; US 3,862,225; US 3,622,627; WO 01/87823A1; and WO 00/28983A1. Each of these aforementioned applications and patents are hereby incorporated herein by reference in its entirety. In addition, the invention pertains to each of the compounds shown in Tables 2 and 3, methods of using each of the compounds, and pharmaceutical compositions comprising each of the compounds.

Other substituted tetracyclines which can be used in the methods of the invention include compounds of the formula I: 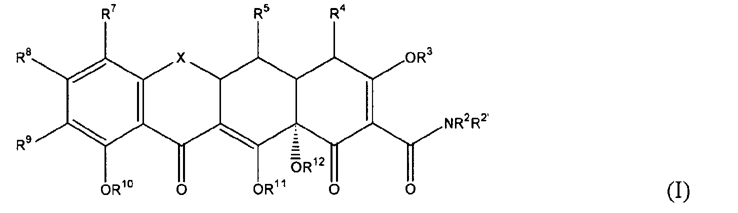 wherein:
R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
R^{2'}, R³, R¹⁰, R¹¹ and R¹² are each hydrogen or a pro-drug moiety;
R⁴ is NR^{4'}R^{4"}, alkyl, alkenyl, alkynyl, hydroxyl, halogen, or hydrogen;
R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl, alkylsulfonyl, alkylamino, arylalkyl, alkyl carbonyloxy, or aryl carbonyloxy;
R⁶ and R^{6'} are each independently hydrogen, methylene, absent, hydroxyl, halogen, thiol, alkyl, alkenyl, alkynyl, aryl, alkoxy, alkylthio, alkylsulfinyl, alkylsulfonyl, alkylamino, or an arylalkyl;
R⁷ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀-₃NR^{7c}C(=W')WR^{7a};
R⁸ is hydrogen, hydroxyl, halogen, thiol, nitro, alkyl, alkenyl, alkynyl, aryl, alkoxy, alkylthio, alkylsulfinyl, alkylsulfonyl, alkylamino, amino, arylalkenyl, arylalkynyl, acyl, aminoalkyl, heterocyclic, thionitroso, or -(CH₂)₀₋₃NR^{8c}C(=E')ER^{8a};
R⁹ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃NR^{9c}C(=Z')ZR^{9a}_{;}
R^{7a}, R^{7b}, R^{7c}, R^{7d}, R^{7e}, R^{7f}, R^{8a}, R^{8b}, R^{8c}, R^{8d}, R^{8e}, R^{8f}, R^{9a}, R^{9b}, R^{9c}, R^{9d}, R^{9e}, and R^{8f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
R¹³ is hydrogen, hydroxy, alkyl, alkenyl, alkynyl, alkoxy, alkylthio, aryl, alkylsulfinyl, alkylsulfonyl, alkylamino, or an arylalkyl;
E is CR^{8d}R^{8e}, S, NR^{8b} or O;
E' is O, NR^{8f}, or S;
W is CR^{7d}R^{7e}, S, NR^{7b} or O;
W' is O, NR^{7f}, or S;
X is CHC(R¹³Y'Y), C=CR¹³Y, CR^{6'}R⁶, S, NR⁶, or O;
Y' and Y are each independently hydrogen, halogen, hydroxyl, cyano, sulfhydryl, amino, alkyl, alkenyl, alkynyl, alkoxy, alkylthio, alkylsulfinyl, alkylsulfonyl, alkylamino, or an arylalkyl;
Z is CR^{9d}R^{9c}, S, NR^{9b} or O;
Z' is O, S, or NR^{9f}, and pharmaceutically acceptable salts, esters and enantiomers thereof.

In a further embodiment, R², R^{2'}, R⁸, R¹⁰, R¹¹, and R¹² are each hydrogen, X is CR⁶R^{6'}, and R⁴ is NR^{4'}R^{4"}, wherein R^{4'} and R^{4"} are each methyl. In another embodiment, R⁴ is hydrogen. R⁹ may also be hydrogen.

In an embodiment, the substituted tetracycline compounds used in the methods and compositions of the invention are substituted sancycline compounds, *e.g.*, with substitution at the, for example, 2, 5, 6, 7,8, 9, 10, 11, 11a, 12, 12a position and/or, in the case of methacycline, 13. In substituted sancycline compounds of the invention, R^{2'}, R³, R¹⁰, R¹¹, and R¹² are each hydrogen or a prodrug moiety; R^{4'} and R^{4"} are each alkyl (*e.g.*, lower alkyl, *e.g.*, methyl); X is CR⁶R^{6'}; and R², R⁵, R⁶, R^{6'}, and R⁸ are each, generally, hydrogen. In an embodiment, the substituted tetracycline compound is a substituted tetracycline (*e.g.*, generally, wherein R⁴ is NR^{4'}R^{4"}, R^{4'} and R^{4"} are methyl, R⁵ is hydrogen and X is CR⁶R^{6'}, wherein R⁶ is methyl and R^{6'} is hydroxy); substituted doxycycline (*e.g.*, wherein R⁴ is NR^{4'}R^{4"}, R^{4'} and R^{4"} are methyl, R⁵ is hydroxyl and X is CR⁶R^{6'}, wherein R⁶ is methyl and R^{6'} is hydrogen); substituted minocycline (*e*.*g*., wherein R⁴ is NR^{4'}R^{4"}, R^{4'} and R^{4"} are methyl; R⁵ is hydrogen and X is CR⁶R^{6'} wherein R⁶ and R^{6'} are hydrogen atoms and R⁷ is dimethylamino) or substituted sancycline (wherein R⁴ is NR^{4'}R^{4"}, R^{4'} and R^{4"} are methyl; R⁵ is hydrogen and X is CR⁶R^{6'} wherein R⁶ and R^{6'} are hydrogen atoms).

In certain embodiments, R⁷ is substituted or unsubstituted aryl. The aryl group may be substituted with one or more substituents, such as, for example, alkyl, alkenyl, alkynyl, halogen, hydroxyl, alkylcarbonyloxy, ary!carbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. In one embodiment, the aryl R⁷ is substituted with at least one amino group or other functional group.

R⁷ also may be a substituted or unsubstituted heterocycle. Examples of heterocycles include pyrrole, furan, thiophene, thiazole, isothiazole, imidazole, triazole, tetrazole, pyrazole, oxazole, isooxazole, pyridine, pyrazine, pyridazine, pyrimidine, benzoxazole, benzodioxazole, benzothiazole, benzoimidazole, benzothiophene, methylenedioxophenyl, quinoline, isoquinoline, naphthridine, indole, benzofuran, purine, benzofuran, deazapurine, indolizine, morpholine, piperazine, piperidine, *etc.* Examples of substituents for the heterocyclic R⁷ group include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. In a further embodiment, the heterocyclic R⁷ group is substituted with an amino group or another functional group.

In another embodiment, R⁷ is substituted or unsubstituted alkenyl or, alternatively, substituted or unsubstituted alkynyl. Examples of possible substituents for the R⁷ alkynyl group include, but are not limited to, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

In yet another embodiment, R⁷ is substituted or unsubstituted alkyl. Examples of substituents for the alkyl R⁷ group include, but are not limited to, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. In certain embodiments, R⁷ is substituted with aryl groups, carbonyl groups, and amino groups (-NH₂ groups, alkylamino groups, dialkylamino groups, alkenylamino groups, dialkenyl amino groups, arylamino groups, *etc*.).

In another further embodiment, R⁷ is -CH₂NR^{7c}C(=W')WR^{7a}. In certain embodiments, R^{7c} is hydrogen, and W and W' are each oxygen. In other embodiments, R⁷ is -NR^{7c}C(=W')WR^{7a}. In certain embodiments, R^{7c} is hydrogen, and W and W' are each oxygen.

In another embodiment, R⁷ is acyl, amino, oximyl, or a dimeric moiety. Each of these substituents may further be substituted with substituents such as, but not limited to, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

In yet another embodiment, R⁷ is hydrogen or dimethylamino. In a further embodiment, R⁹ is amino (*e.g.*, -NH₂, alkylamino, dialkylmino, alkenylamino, *etc*.). In another embodiment, R⁹ is substituted or unsubstituted alkyl. Examples of substituents for the alkyl group include, but are not limited to, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. In certain embodiments, the alkyl R⁹ group is substituted with an amino or amido group. The amino group may, for example, be further substituted with an alkylamino group or other group described above.

In another embodiment, R⁹ is substituted or unsubstituted aryl. The aryl group may be heterocyclic (pyrrole, furan, thiophene, thiazole, isothiazole, imidazole, triazole, tetrazole, pyrazole, oxazole, isooxazole, pyridine, pyrazine, pyridazine, pyrimidine, benzoxazole, benzodioxazole, benzothiazole, benzoimidazole, benzothiophene, methylenedioxophenyl, quinoline, isoquinoline, naphthridine, indole, benzofuran, purine, benzofuran, or deazapurine) or carbocyclic (*e.g.*, phenyl, *etc*.). Examples of substituents for aryl R⁹ groups include, but are not limited to, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. In a further embodiment, the aryl group is phenyl and substituted with amino group.

In another embodiment, R⁹ is substituted or unsubstituted alkynyl. In a further embodiment, R⁹ is -CH₂NR^{9c}C(=Z')ZR^{9a}. Examples of R^{9c} include hydrogen. Examples of Z' and Z include oxygen and nitrogen. In another embodiment, R^{9c} is hydrogen, Z' and Z are each oxygen.

In yet another embodiment, R⁹ is -NR^{9c}C(=Z')ZR^{9a}_{.} In an embodiment, R^{9c} is hydrogen, Z' is oxygen and Z is nitrogen.

In a further embodiment, R⁹ is substituted or unsubstituted alkyl or alkylamino. Examples of substituents for R⁹ include but are not limited to alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. In a further embodiment, R⁹ may be substituted with alkyl, *e.g.*, R⁹ may be alkylaminoalkyl. In addition, R⁷ may be substituted or unsubstituted alkyl, alkynyl, or a heterocycle. R⁷ also may be substituted with amino.

In a further embodiment, R⁹ is -NR^{9c}C(=Z')ZR^{9a}, R^{9c} is hydrogen, Z' is oxygen and Z is oxygen.

In yet another further embodiment, X is C=CR¹³Y, R¹³ is substituted or unsubstituted aryl and Y is hydrogen. Examples of substituents for R¹³ include, but are not limited to, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

In other embodiments, compounds of the invention include tetracycline compounds wherein R² is alkyl (*e.g.*, 2-alkyl doxycycline compounds). Other compounds also include compounds wherein R⁵ is an ester or prodrug moiety. Other compounds of the invention include compounds wherein R¹⁰ is alkyl.

Examples of substituted tetracycline compounds of the invention include compounds of Tables 2 and 3, the compounds shown below, and pharmaceutically acceptable esters, prodrugs and salts thereof.

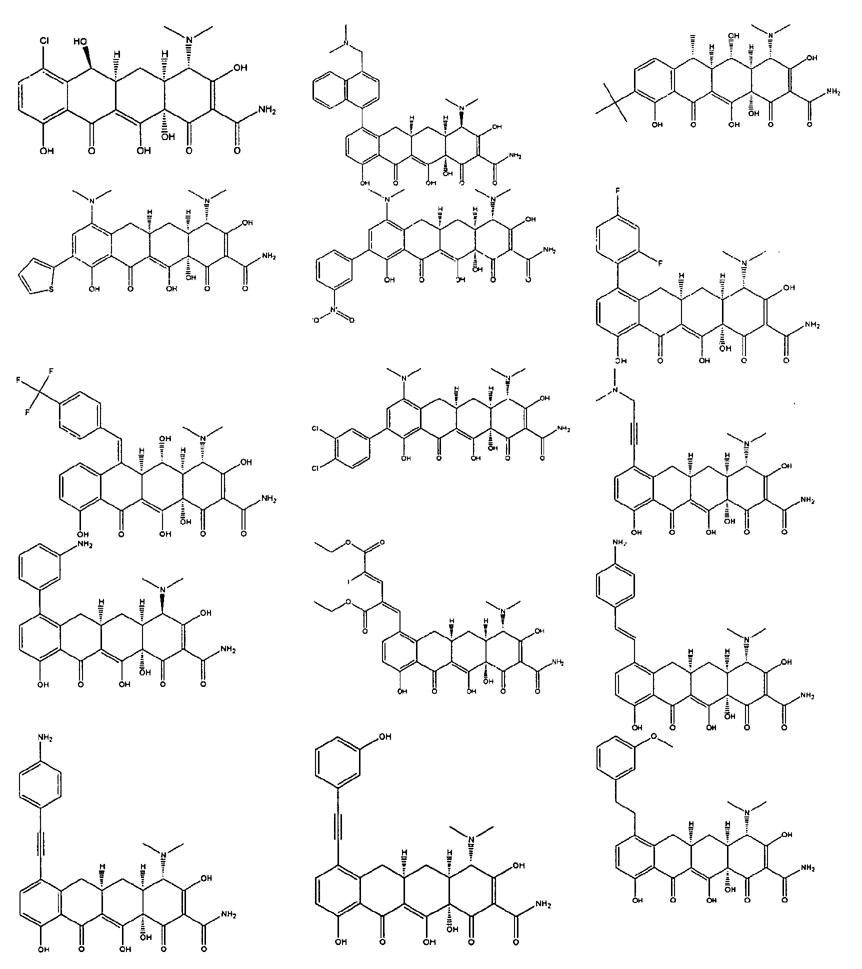

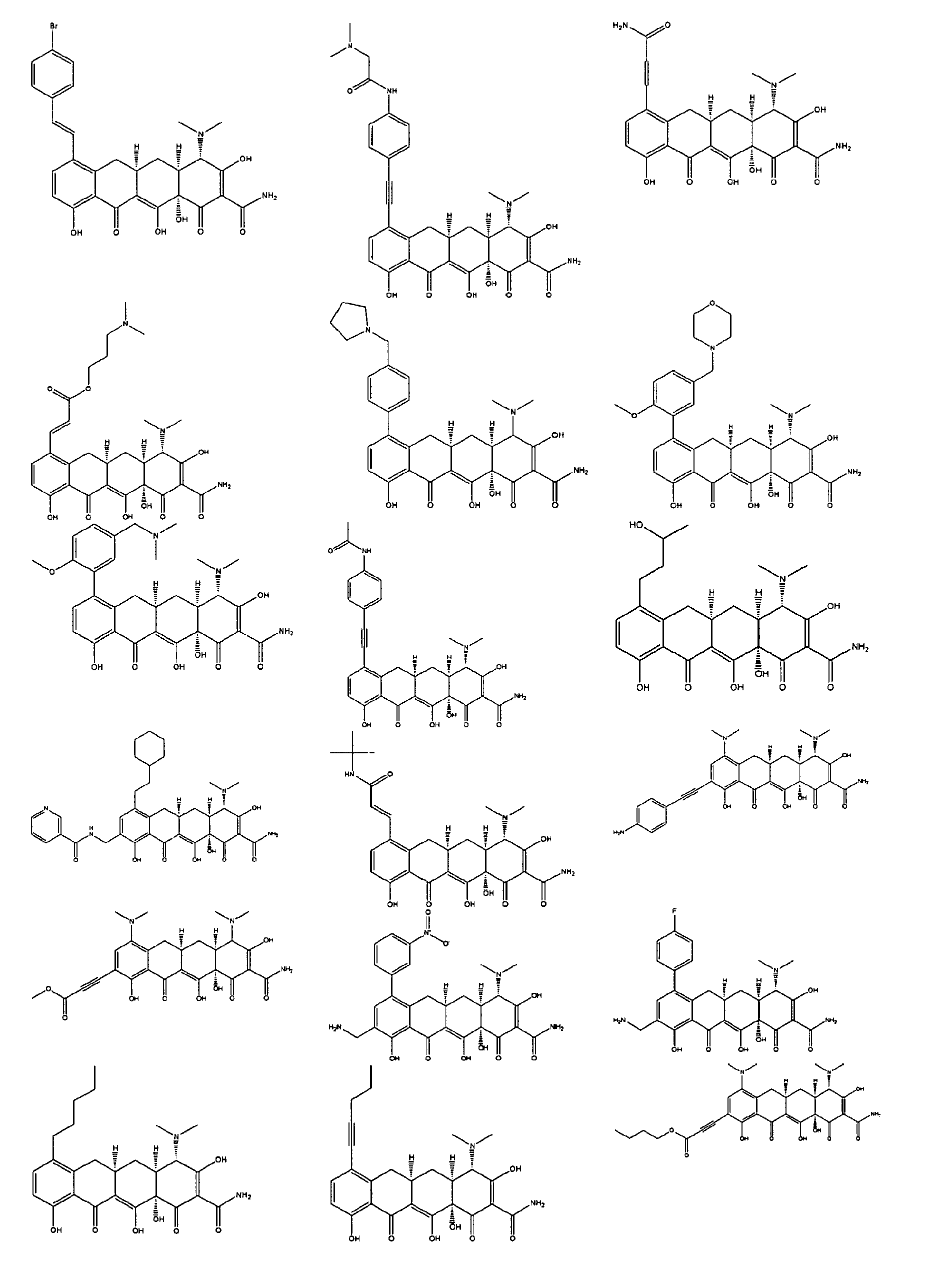

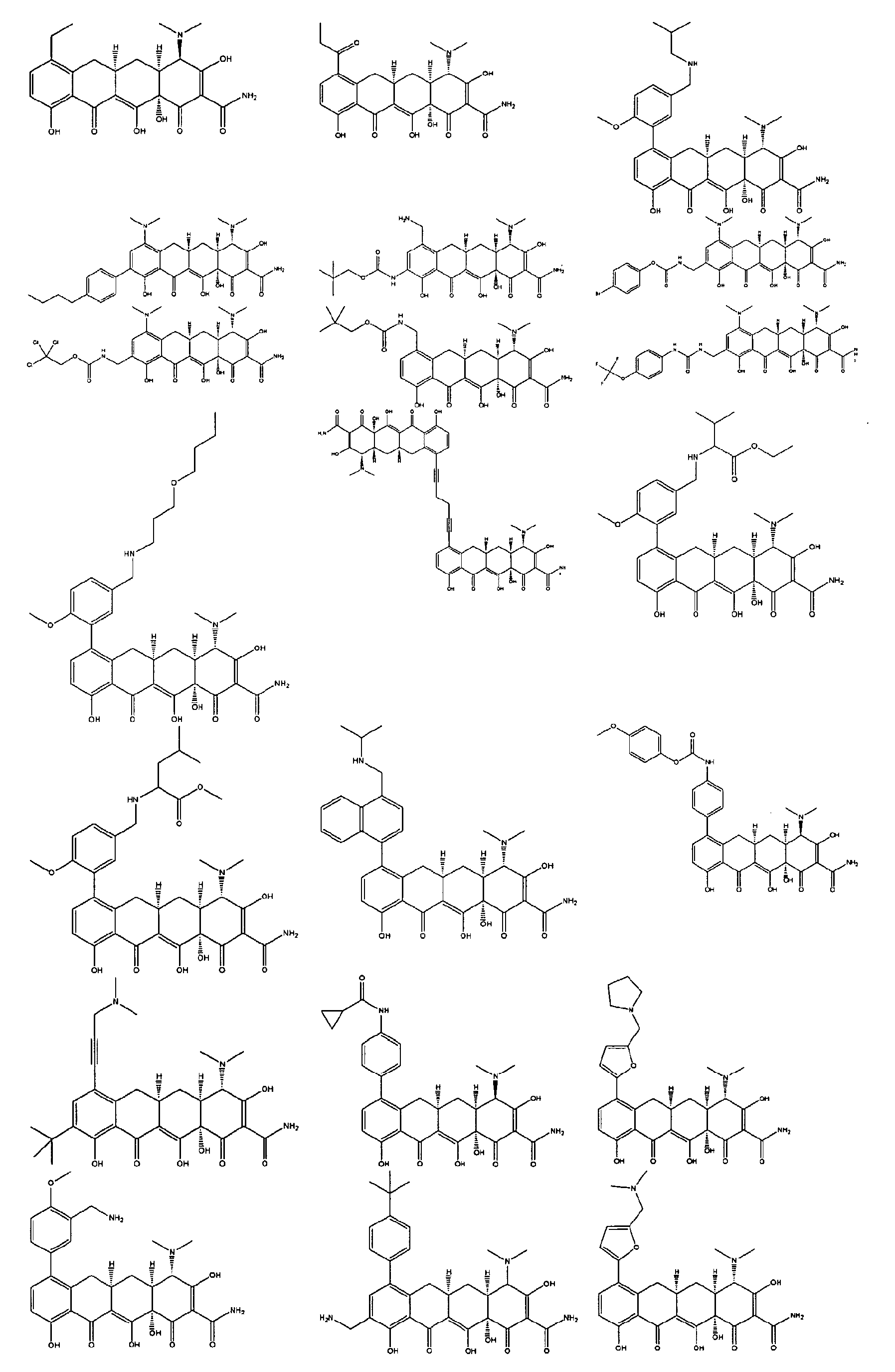

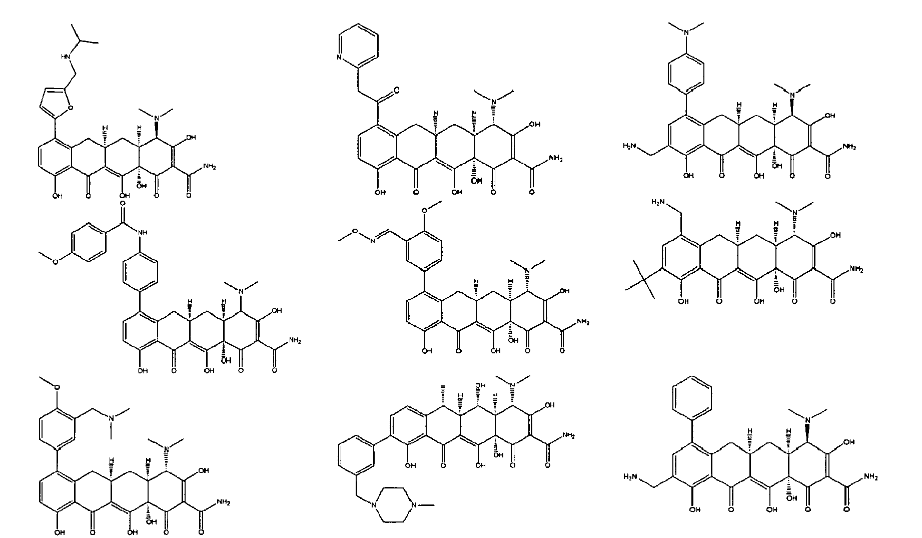

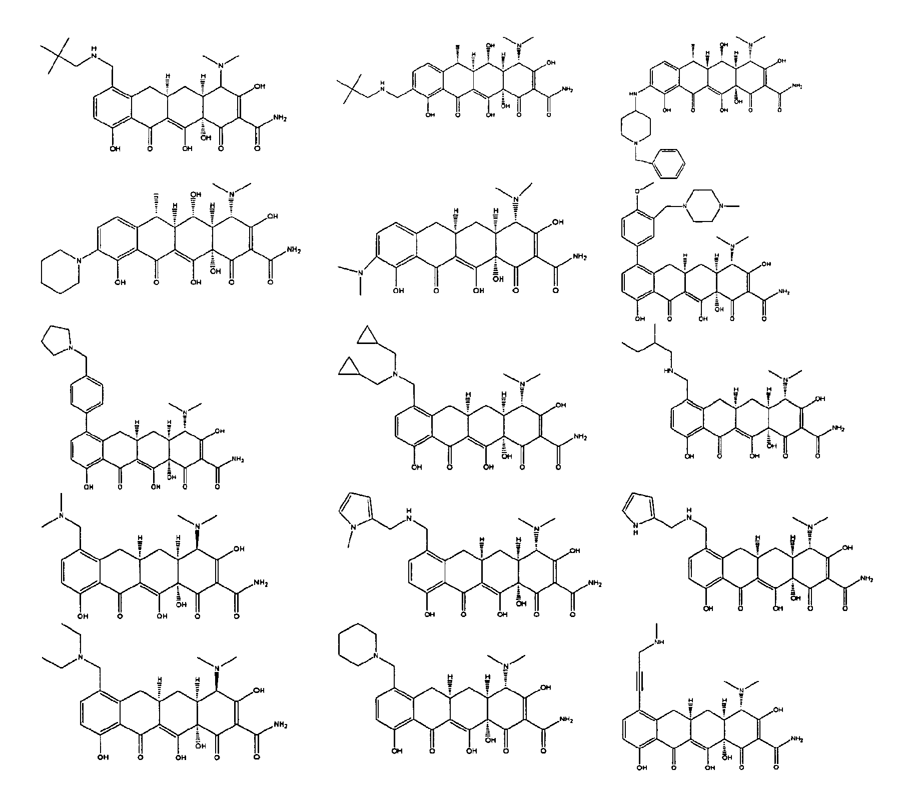

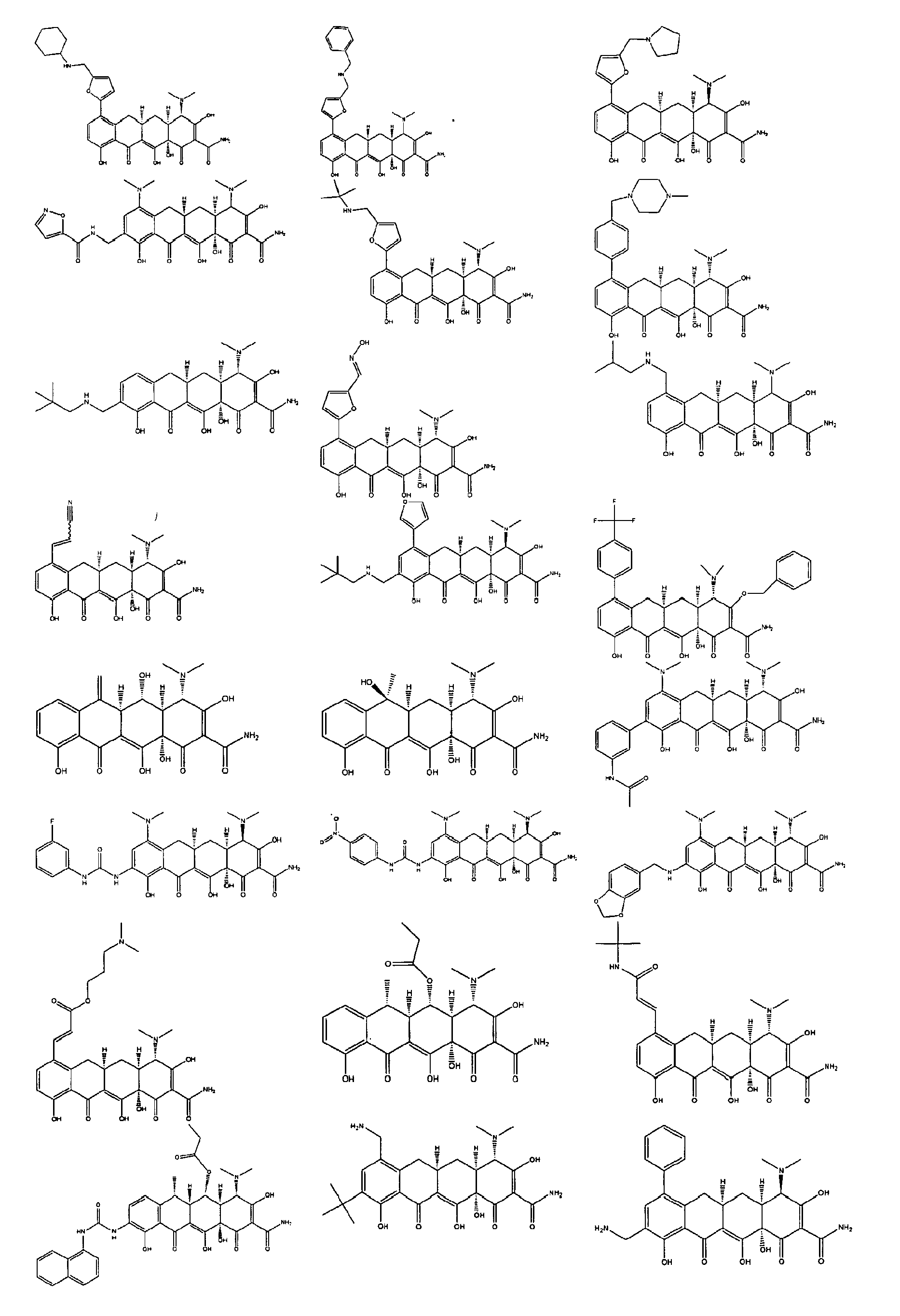

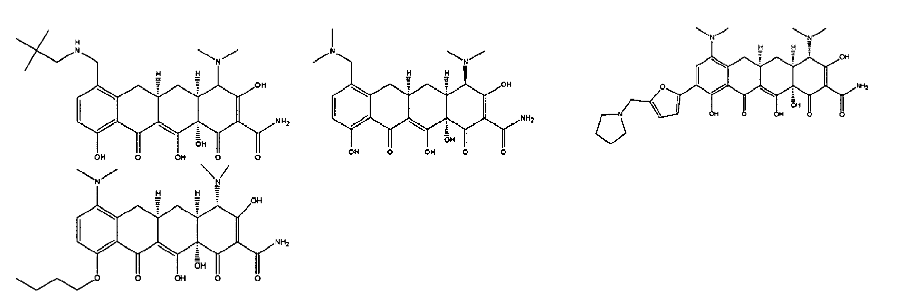

Other tetracycline compounds of the invention are shown in Tables 2 and 3.

In certain embodiments, the substituted tetracycline compounds of the invention have antibacterial activity against gram + and/or gram - bacteria. In certain embodiments, the tetracycline compounds of the invention do not have antibacterial activity against gram + and/or gram - bacteria. The results of an antibacterial MIC assay (as described in Example 3) is shown in Table 3 for both gram + and gram - bacteria. For illustrative purposes not to be construed as limiting, in Table 3 compounds with MIC less than or equal to 4 µg/ml are indicated with ** and compounds with an MIC of greater than 4 µg/ml are indicated with *.

In other embodiments, compounds with MIC of greater than about 2 µg/ml, greater than about 3 µg/ml, greater than about 4 µg/ml, greater than about 5 µg/ml, greater than about 6 µg/ml, greater than about 8 µg/ml, greater than about 9 µg/ml, greater than about 10 µglml, greater than about 11 µg/ml, greater than about 12 µg/ml, greater than about 13 µg/ml, greater than about 14 µg/ml, greater than about 15 µg/ml, greater than about 16 µg/ml, greater than about 17 µg/ml, greater than about 18 µg/ml, greater than about 19 µg/ml, greater than about 20 µg/ml, greater than about 25 µg/ml, greater than about 30 µg/ml, greater than about 40 µg/ml, or greater than about 50 µg/ml for gram + and/or gram - bacteria are considered not to have anti-bacterial activity.

In other embodiments, compounds with MIC of less than about 50 µg/ml, less than about 40 µg/ml, less than about 30 µg/ml, less than about 25 µg/ml, less than about 20 µg/ml, less than about 15 µg/ml, less than about 14 µg/ml, less than about 13 µg/ml, less than about 12 µg/ml, less than about 11 µg/ml, less than about 10 µg/ml, less than about 9 µg/ml, less than about 8 µg/ml, less than about 6 µg/ml, less than about 5 µg/ml, less than about 4 µg/ml, less than about 3 µg/ml, less than about 2 µg/ml, less than about 1 µg/ml, or less than about 0.5 µg/ml for gram + and/or gram - bacteria are considered to have anti-bacterial activity.

In one embodiment, the tetracycline compound of the invention may retain antibiotic, antibacterial, or antimicrobial activity, it may have decreased antibiotic, antibacterial, or antimicrobial activity, or, it may have little to no antibiotic, antibacterial or antimicrobial activity. In an embodiment, the substituted tetracycline compound is substituted at the 1, 2, 3, 4, 5, 6, 7, 8, 9, 10, 11, 11a, 12, 12a and/or 13 position. In certain embodiments, the tetracycline compounds of the invention are 7 and/or 9 substituted, *e.g.*, 7 and/or 9-substituted tetracycline compounds (*e.g.*, compounds wherein R⁷ and/or R⁹ are not both hydrogen). In yet a further embodiment, the tetracycline compounds of the invention are 7 and/or 9 substituted sancycline compounds. Other examples of tetracycline compounds which may be used in the methods of the invention include those shown in Tables 2 and 3 below or otherwise described herein or incorporated by reference.

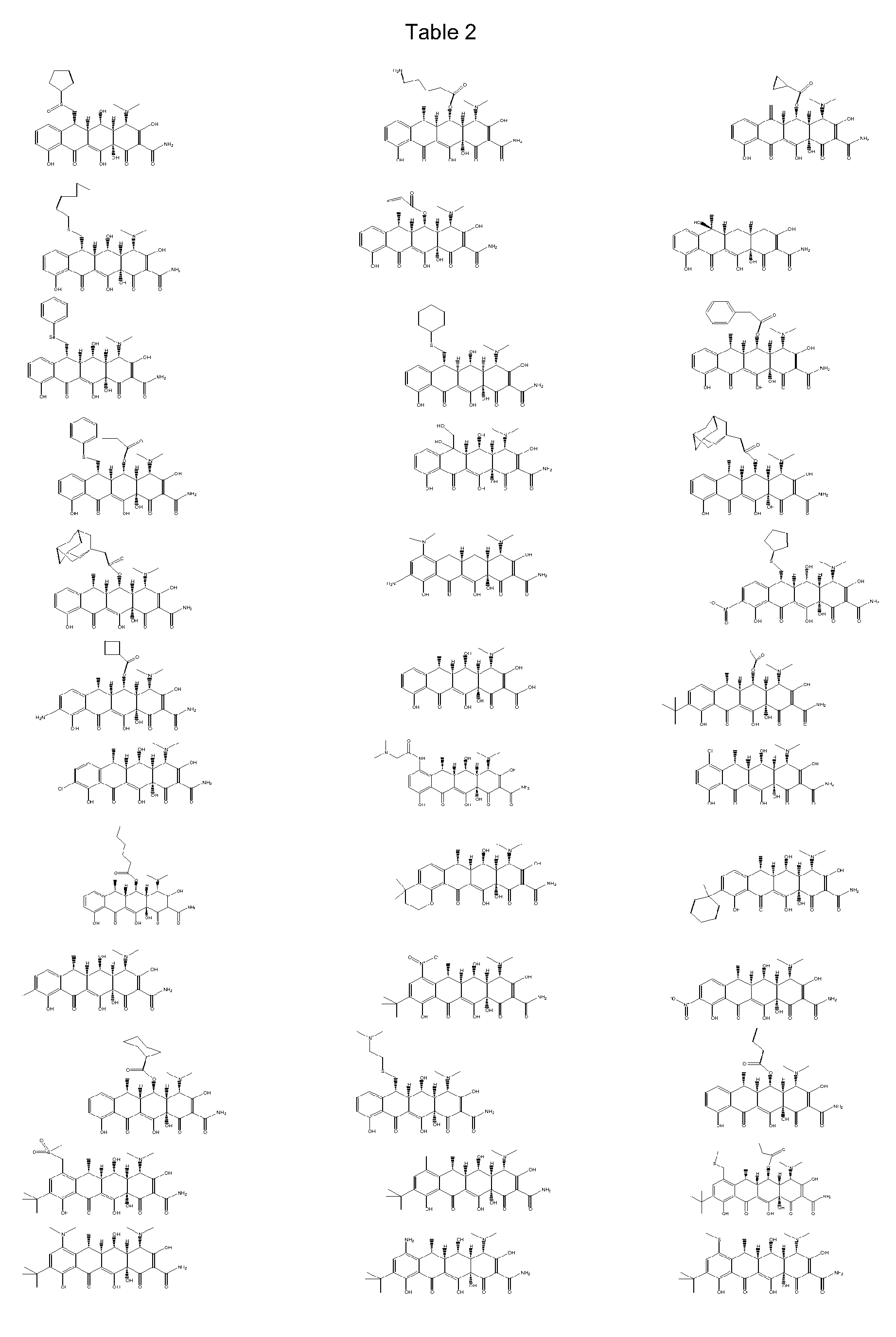

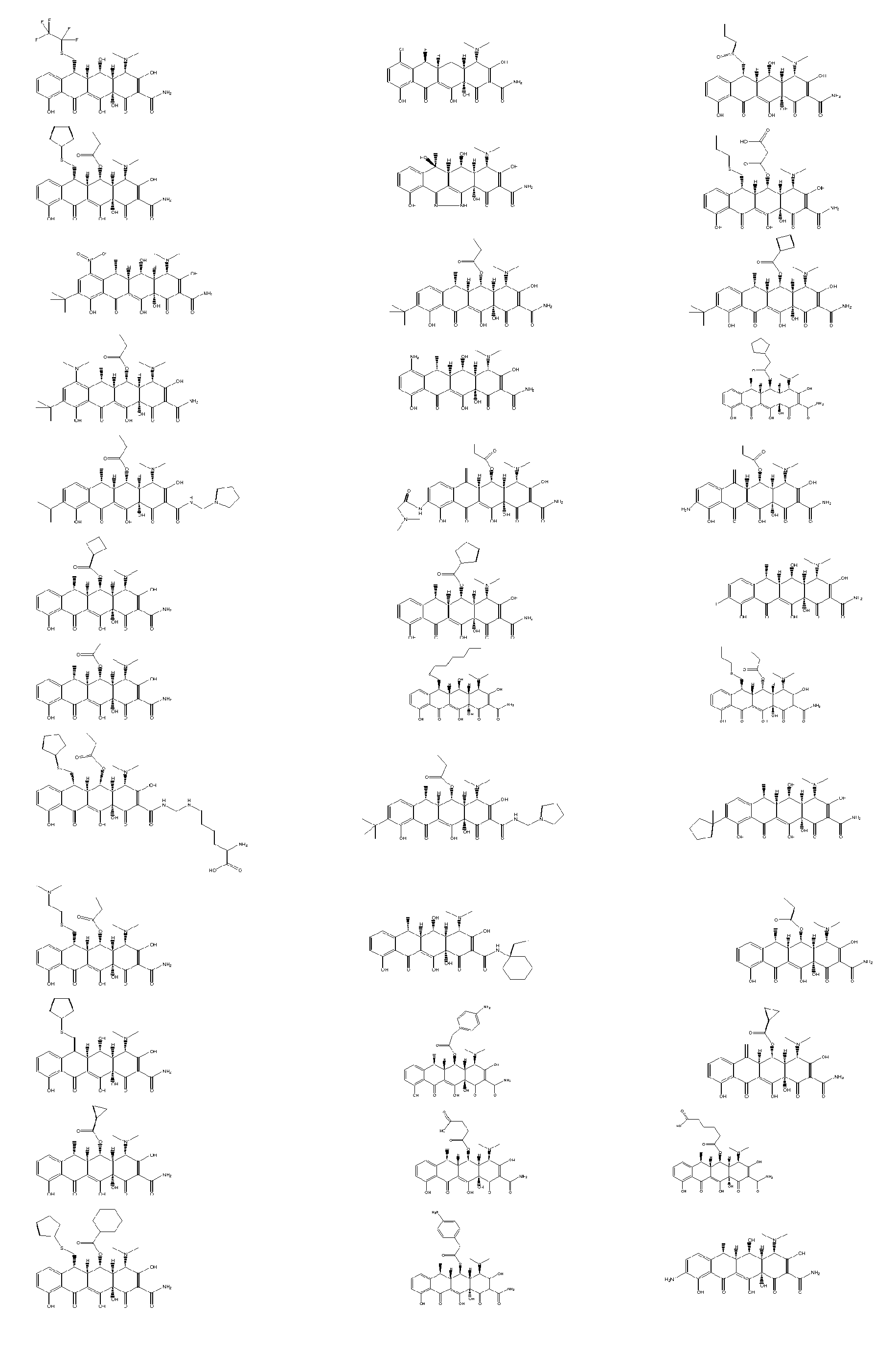

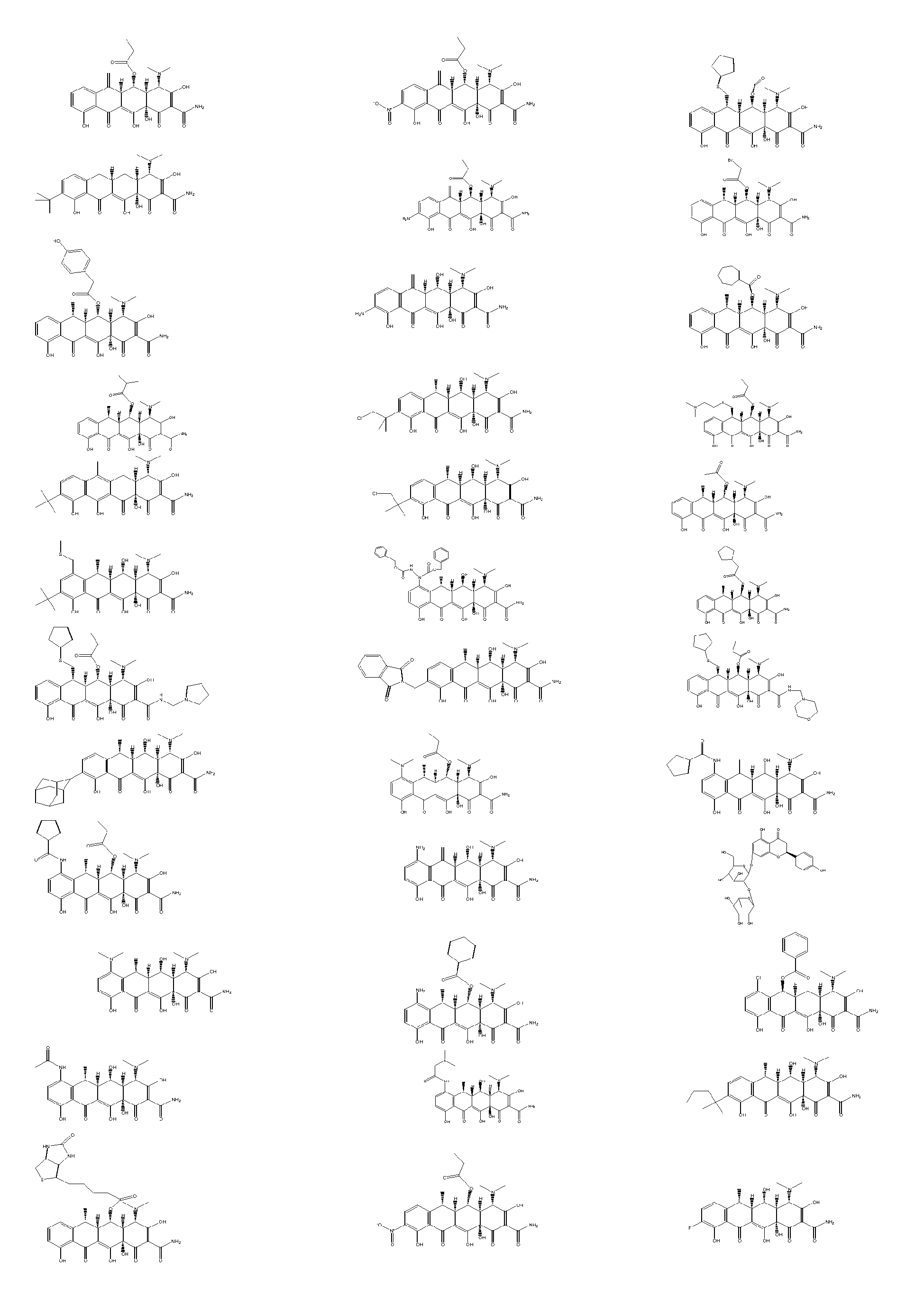

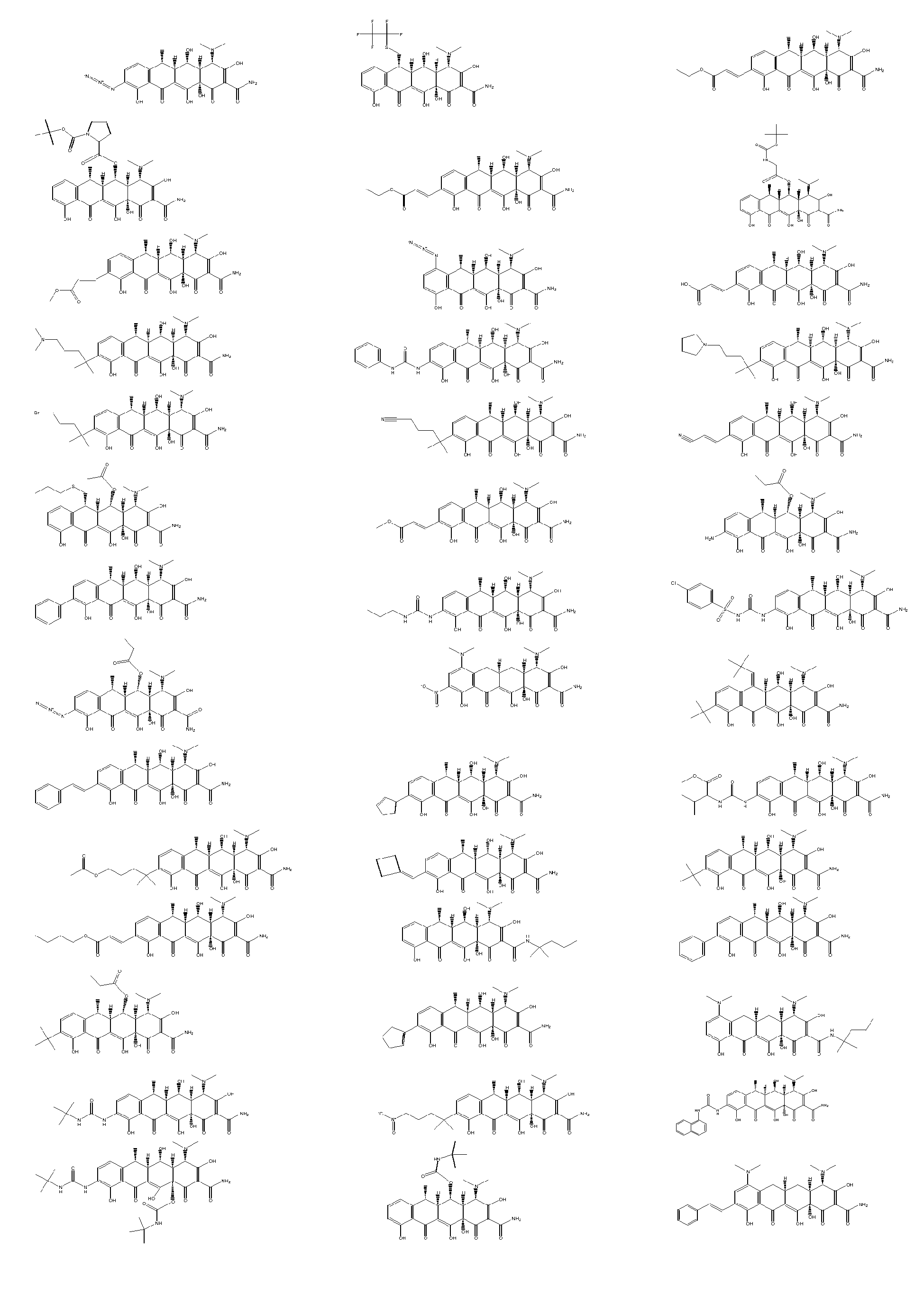

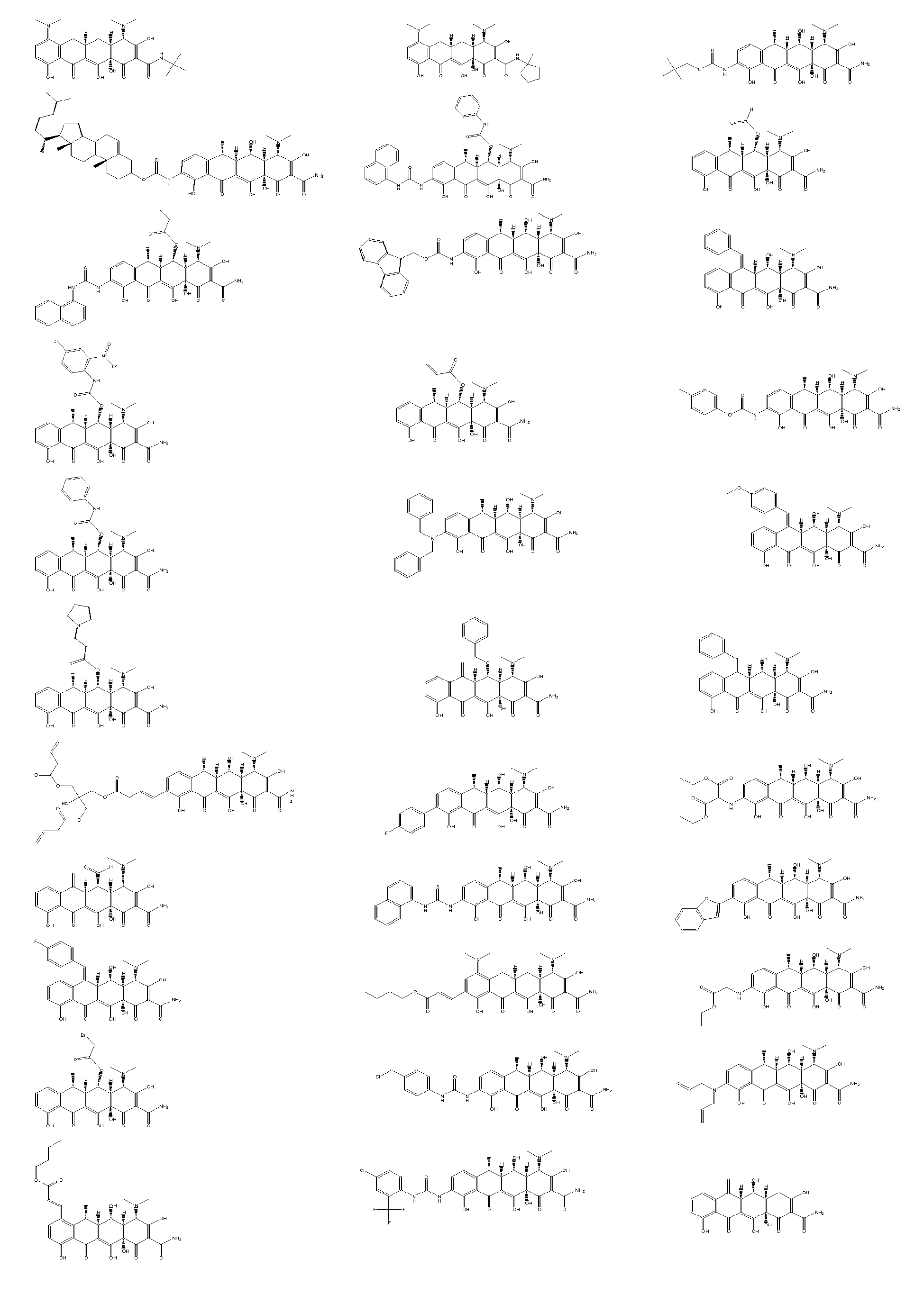

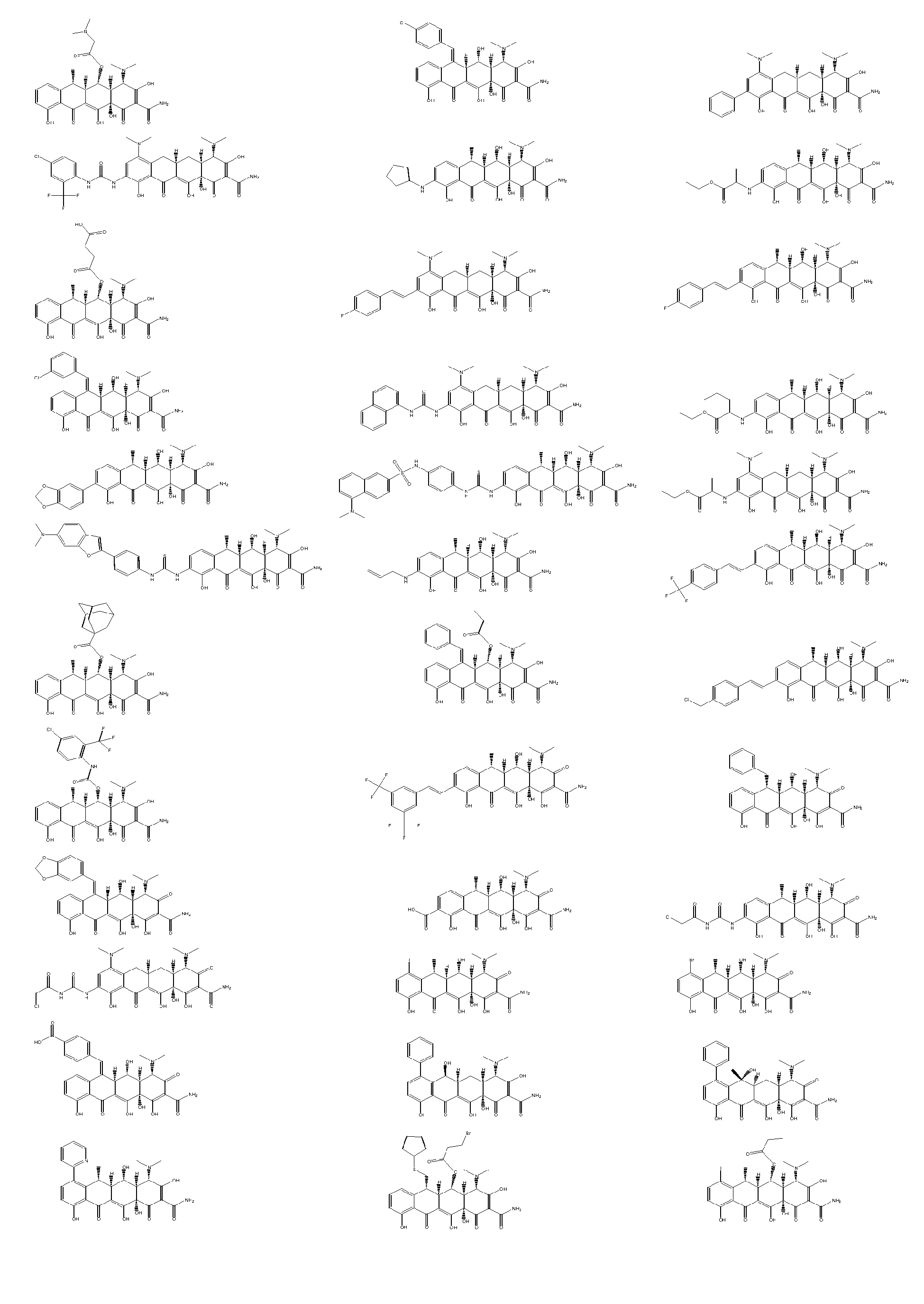

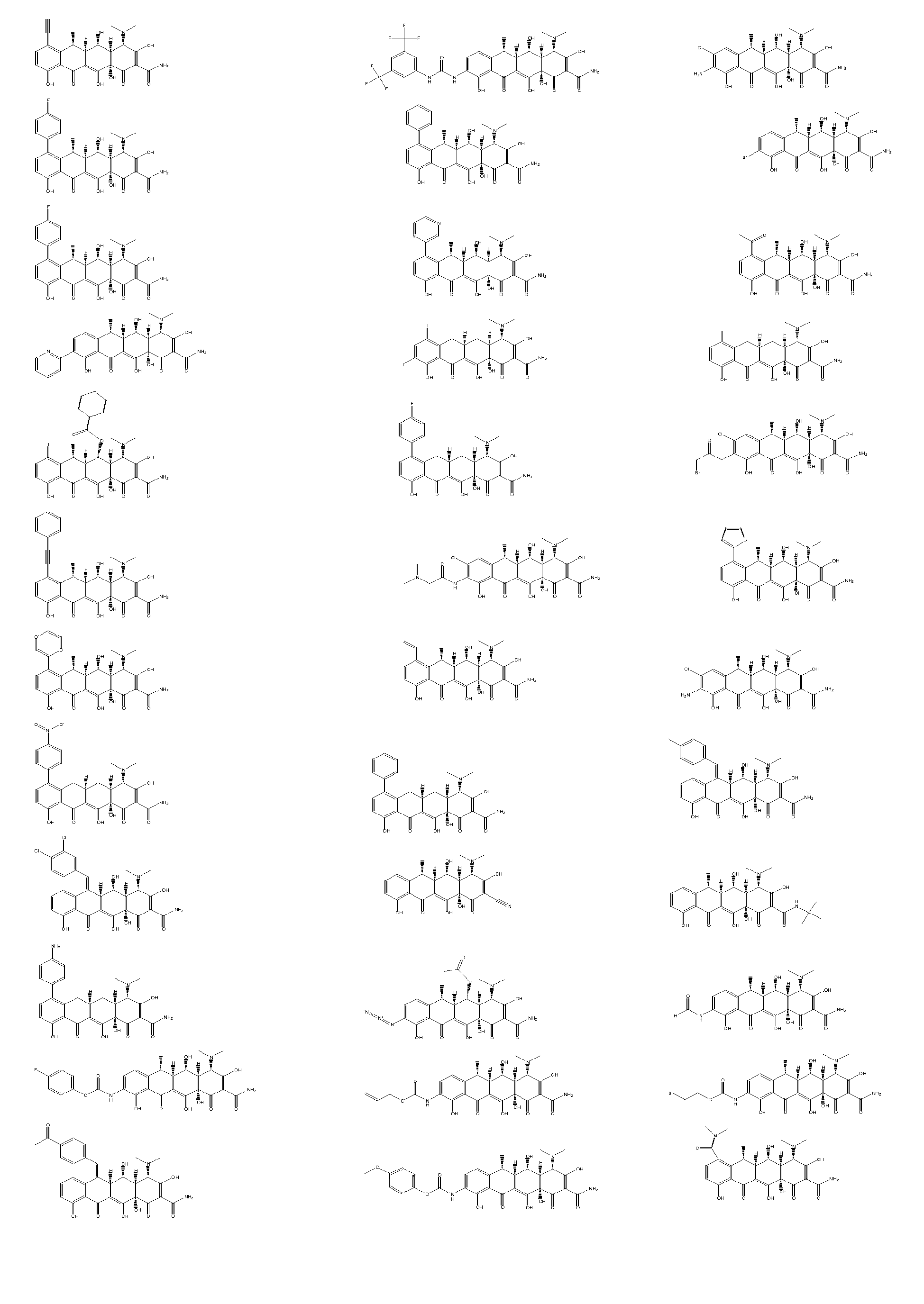

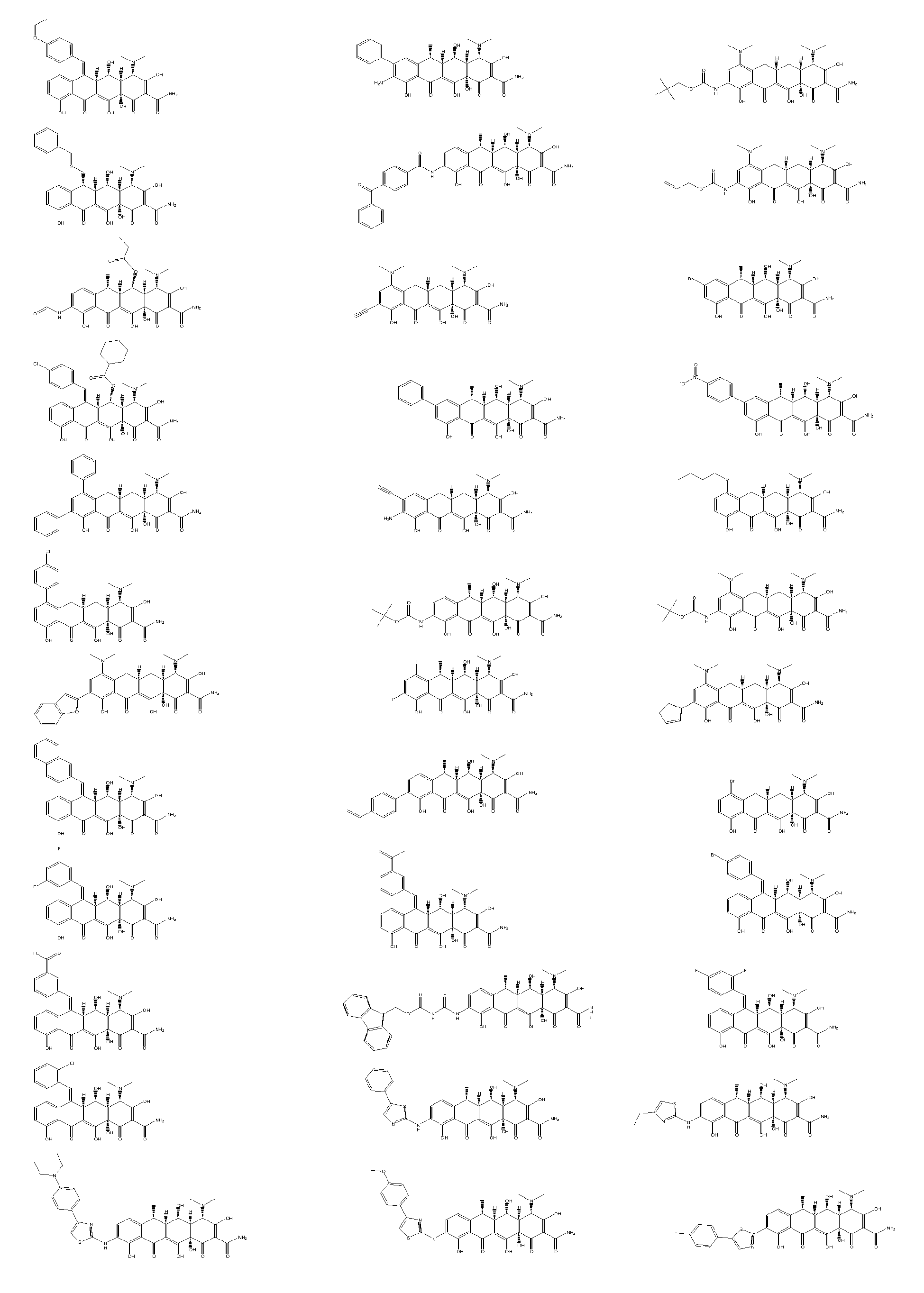

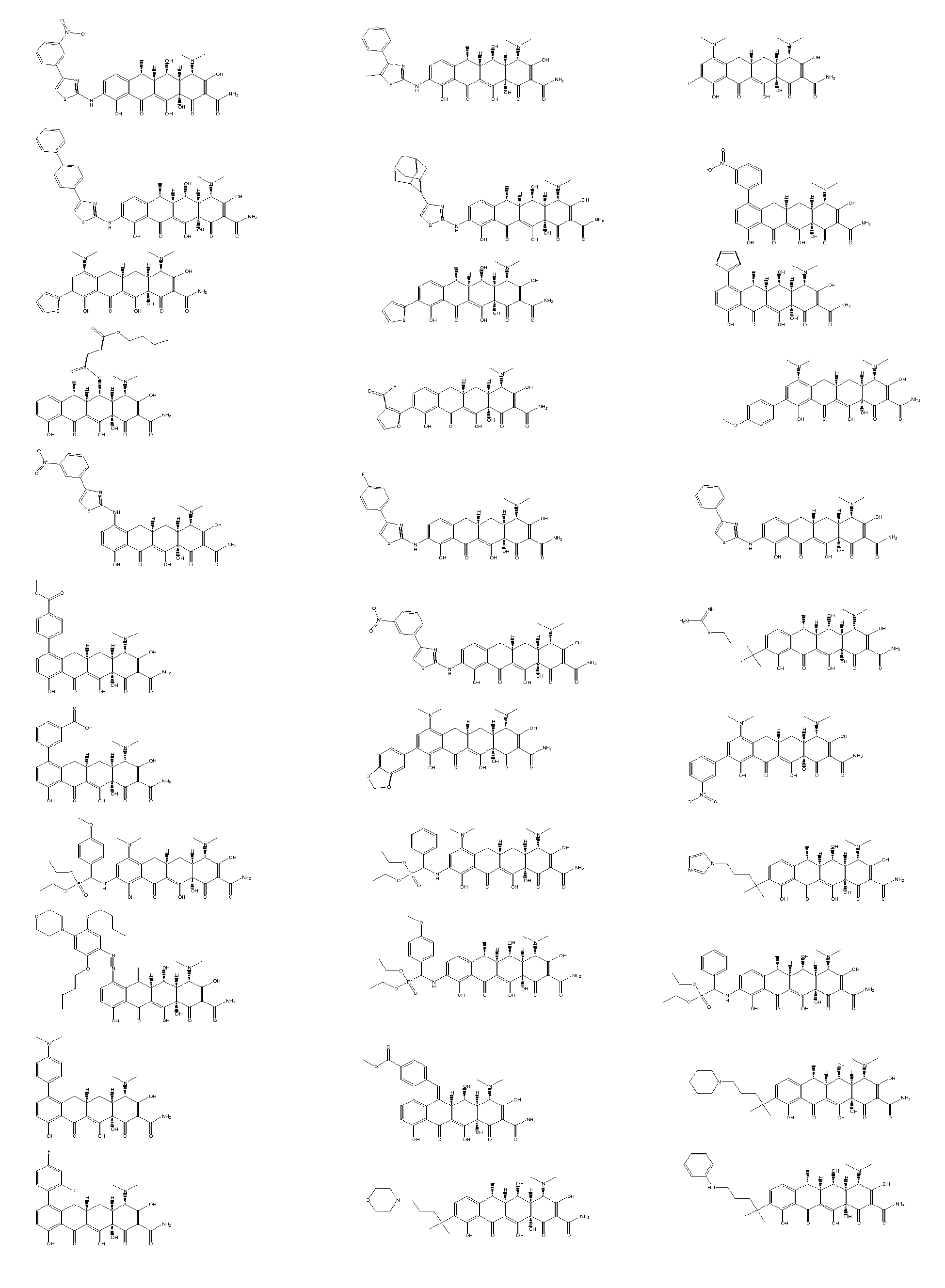

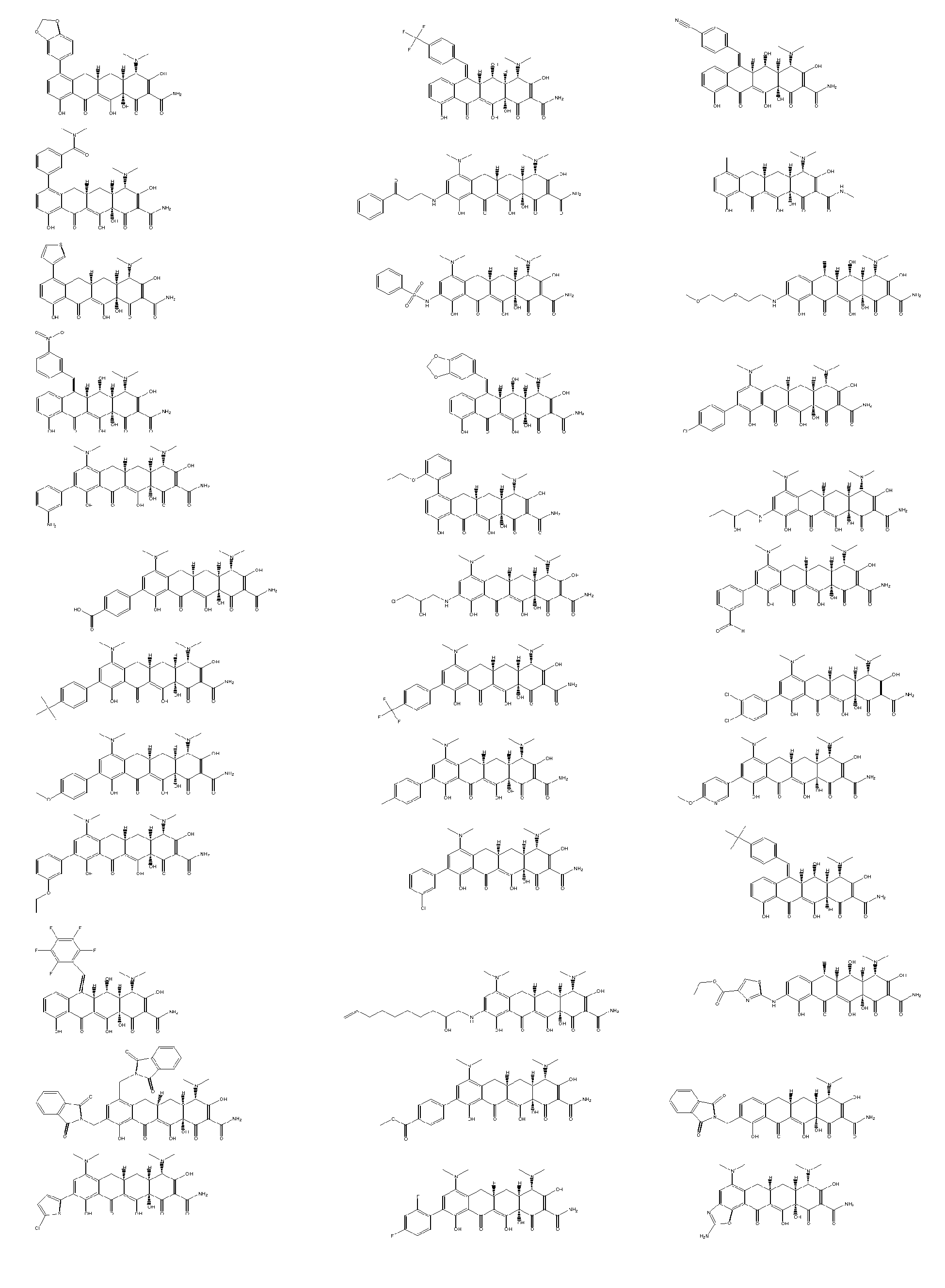

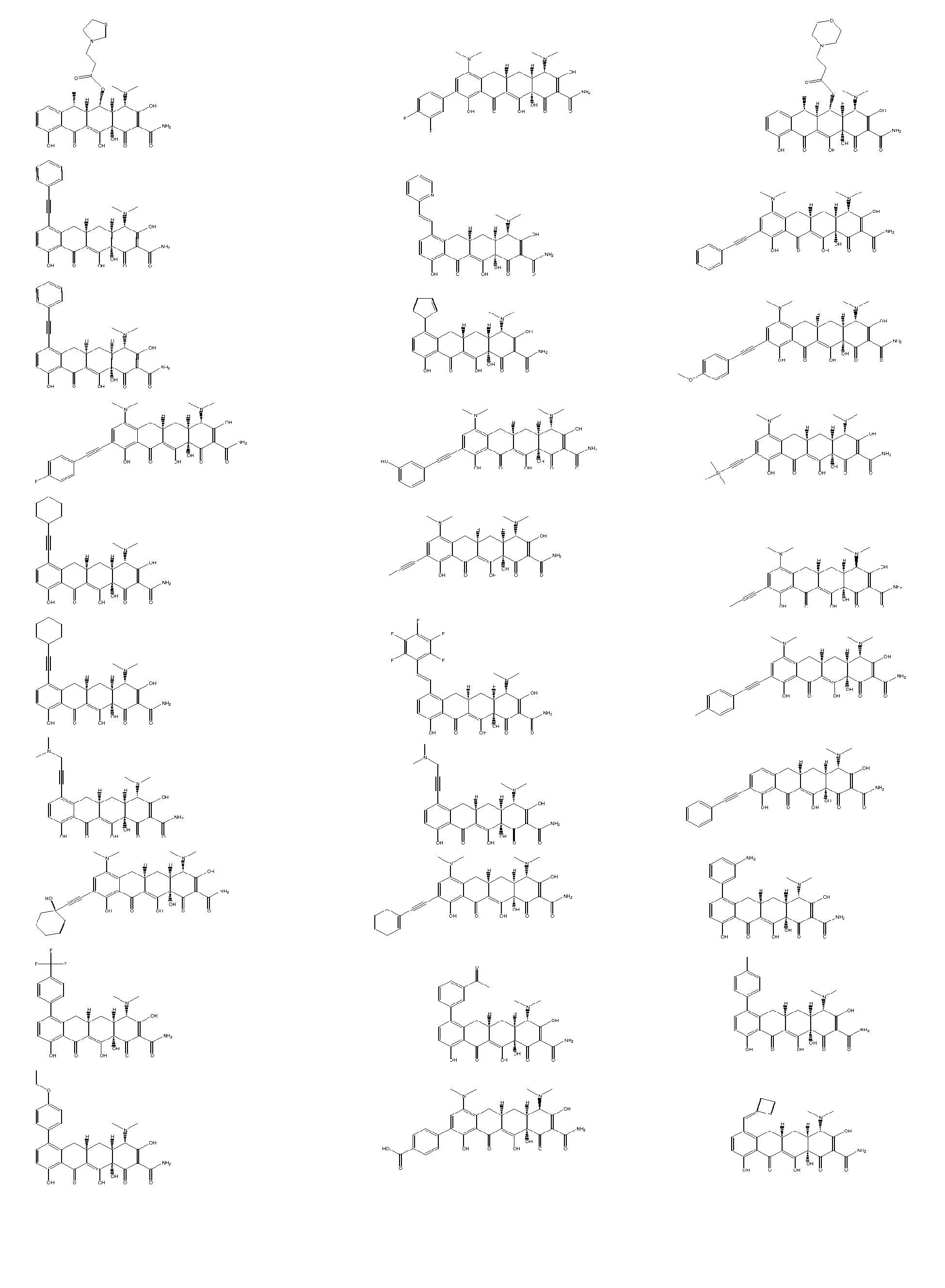

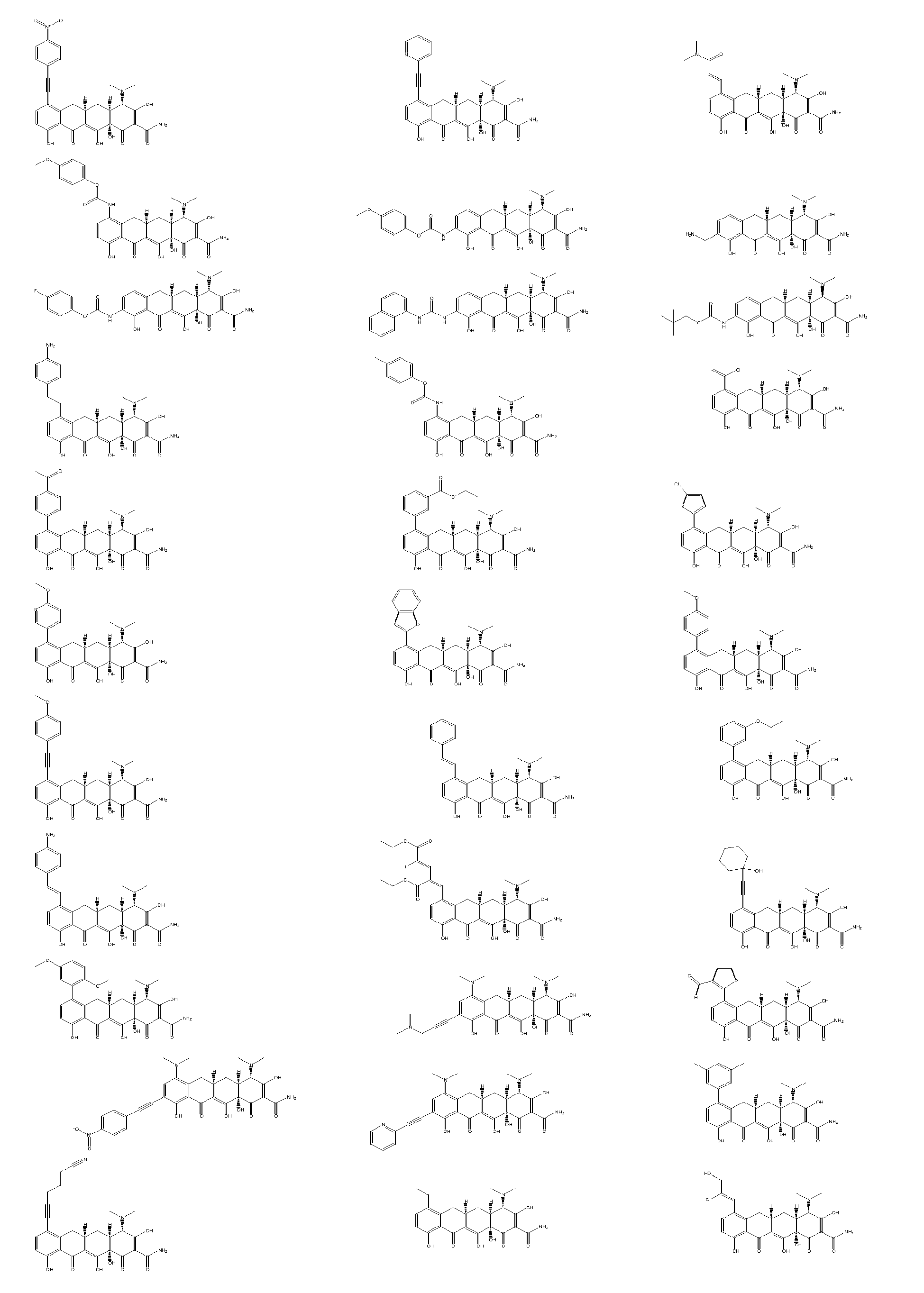

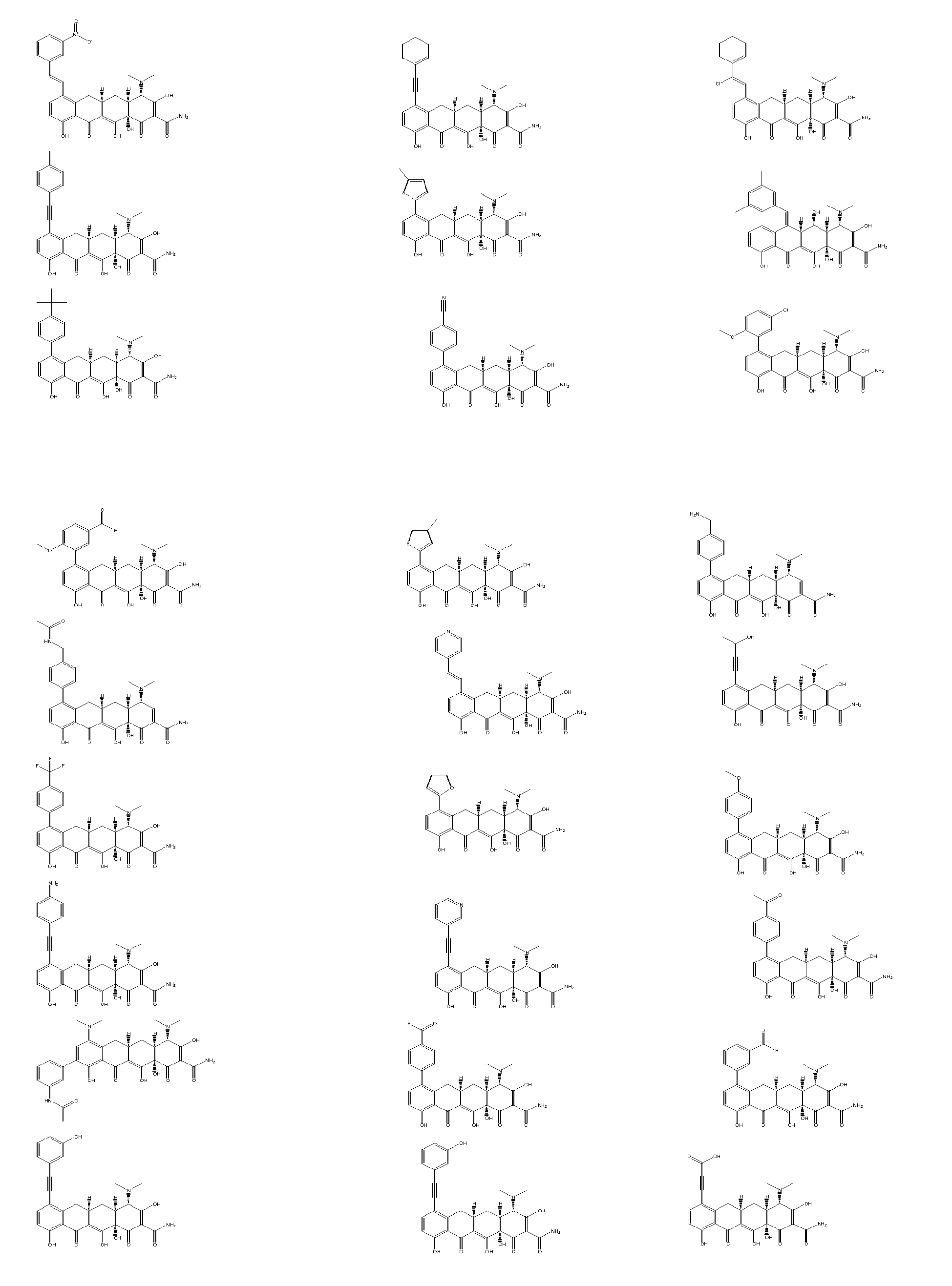

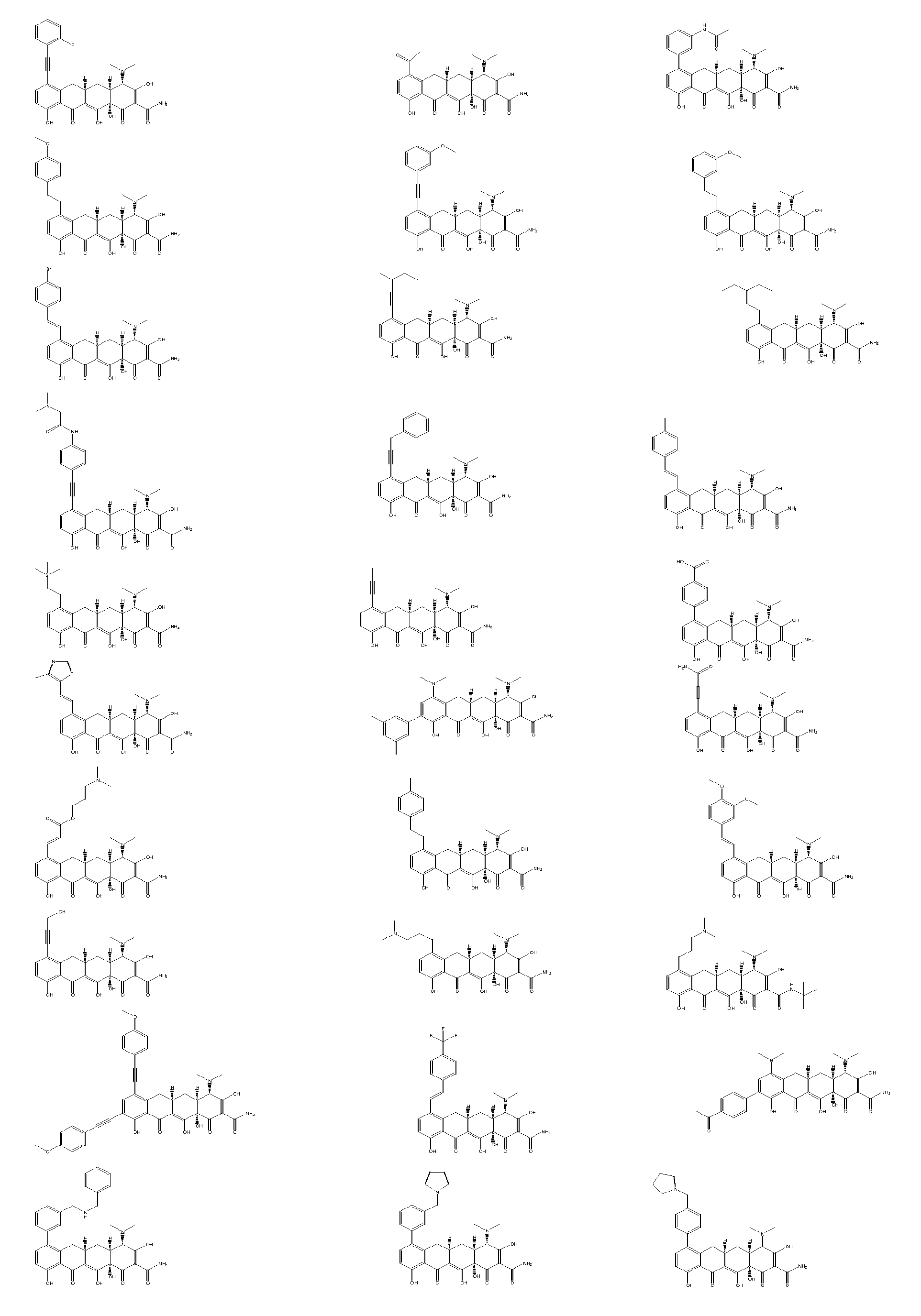

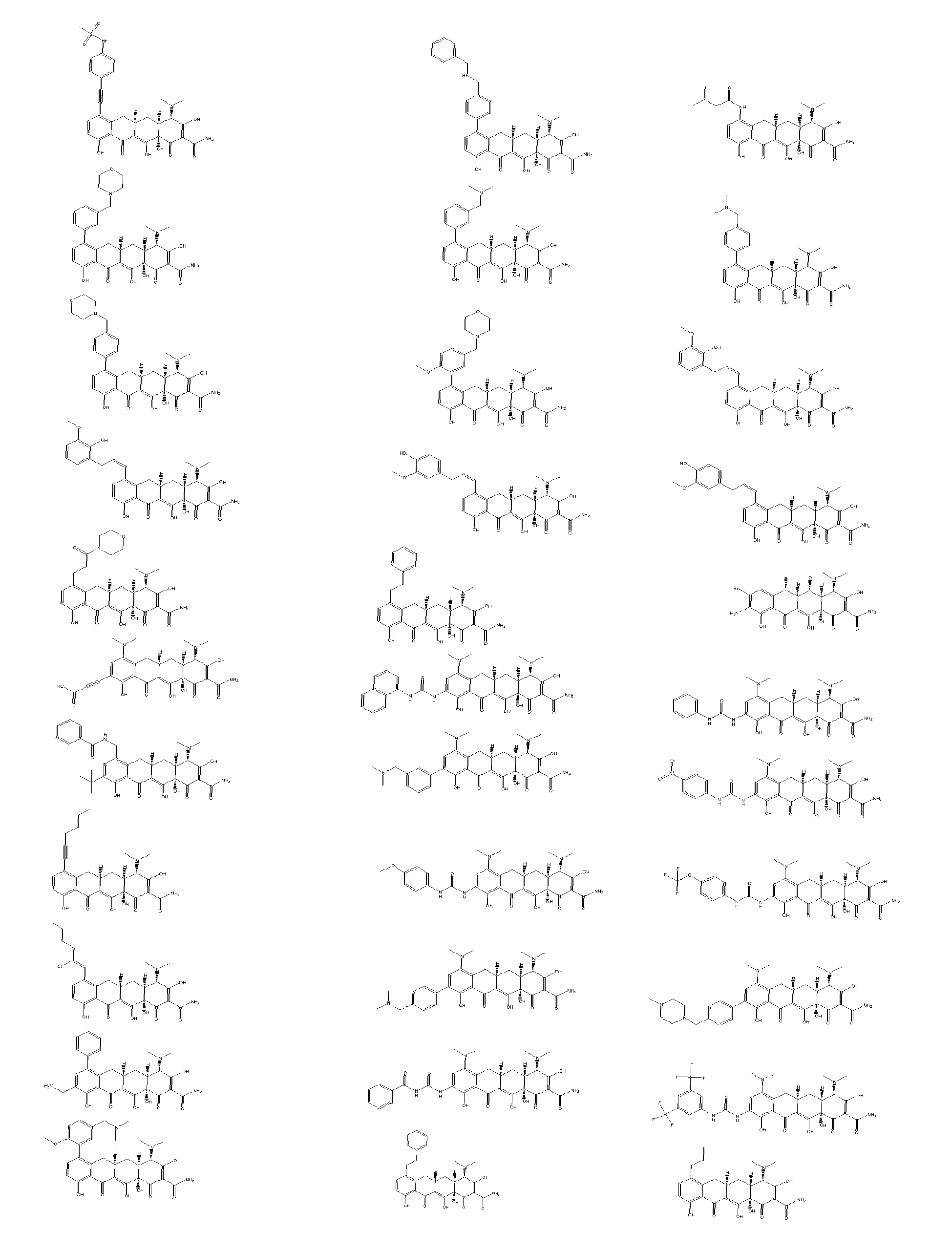

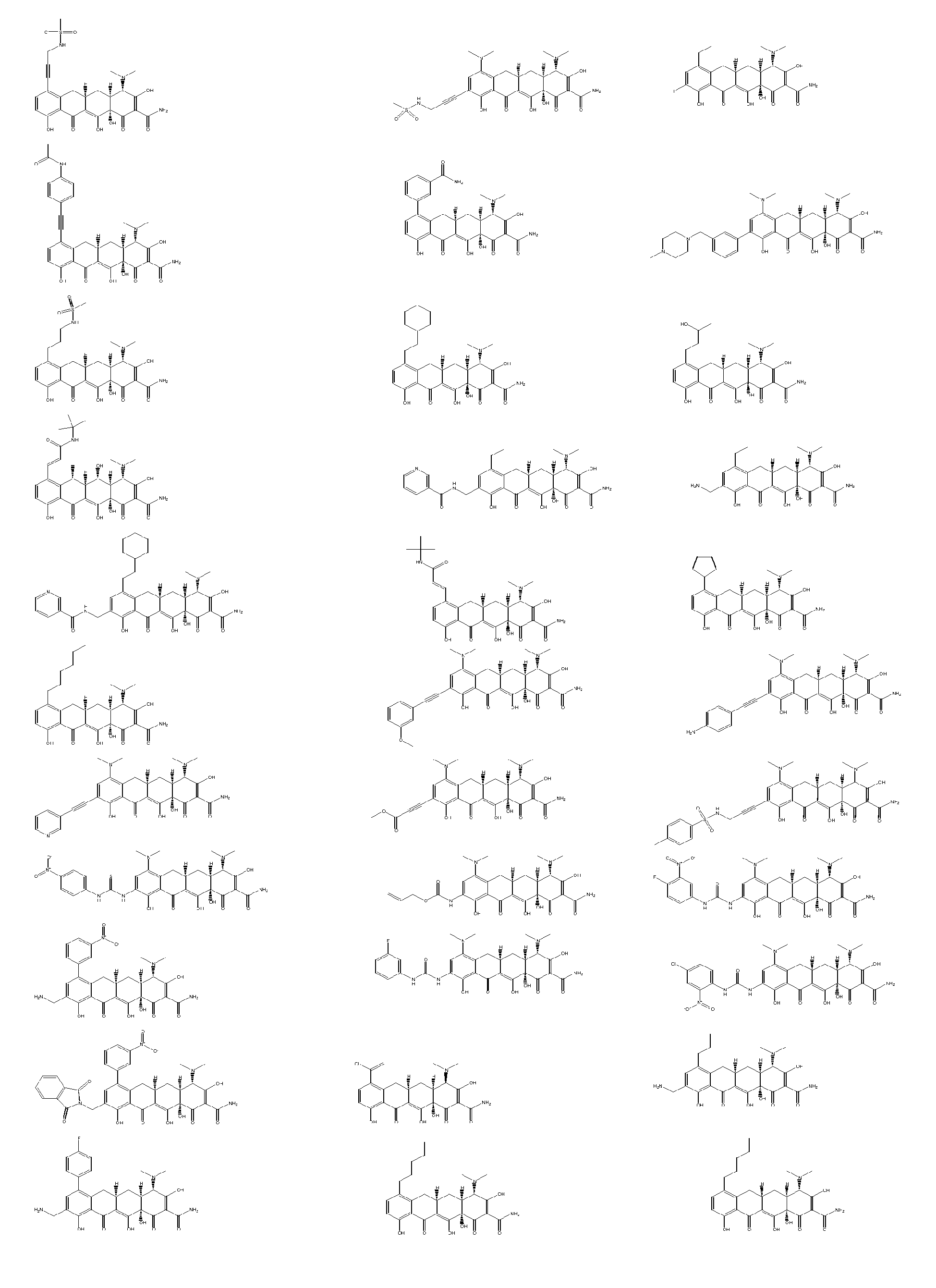

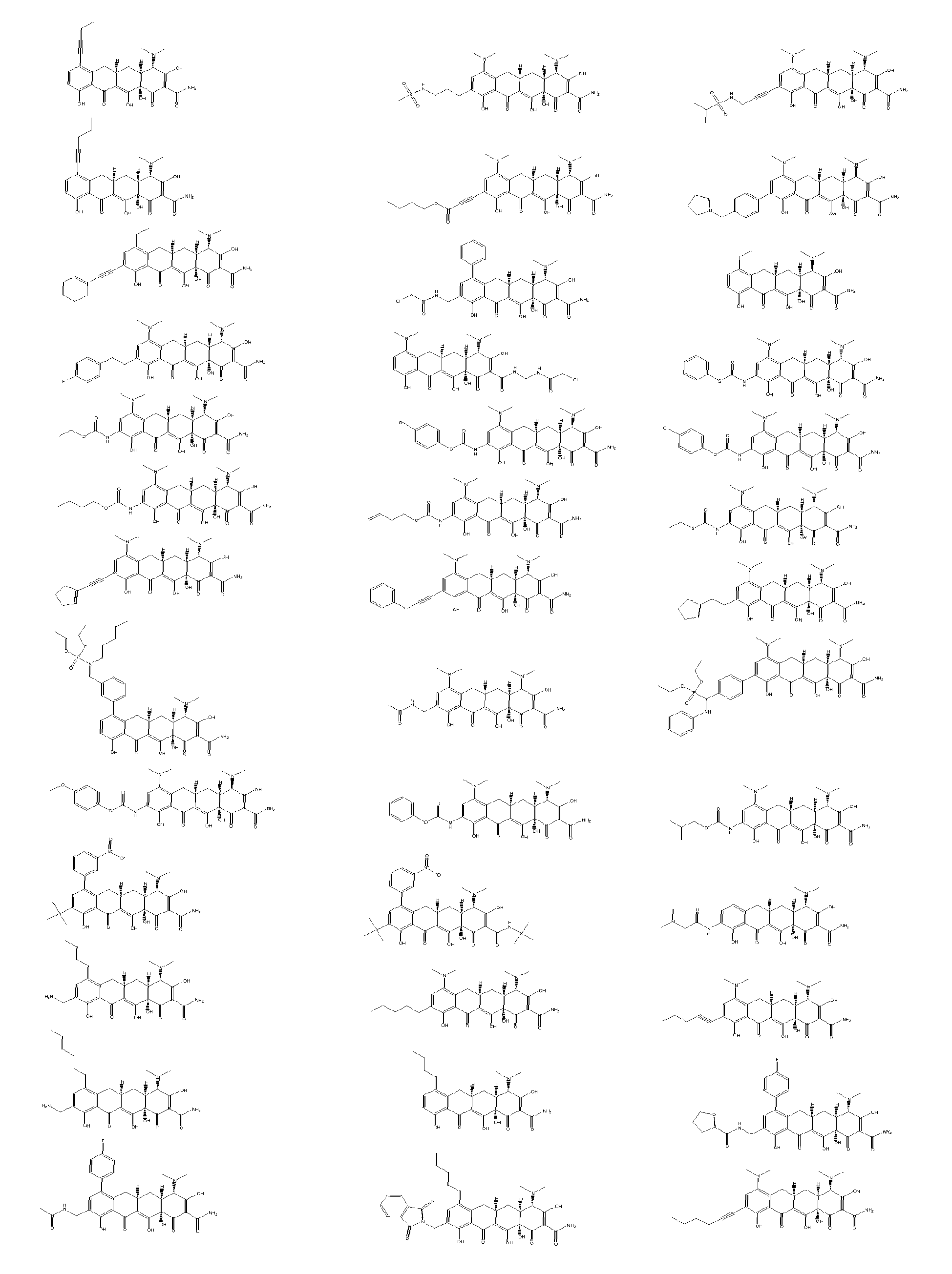

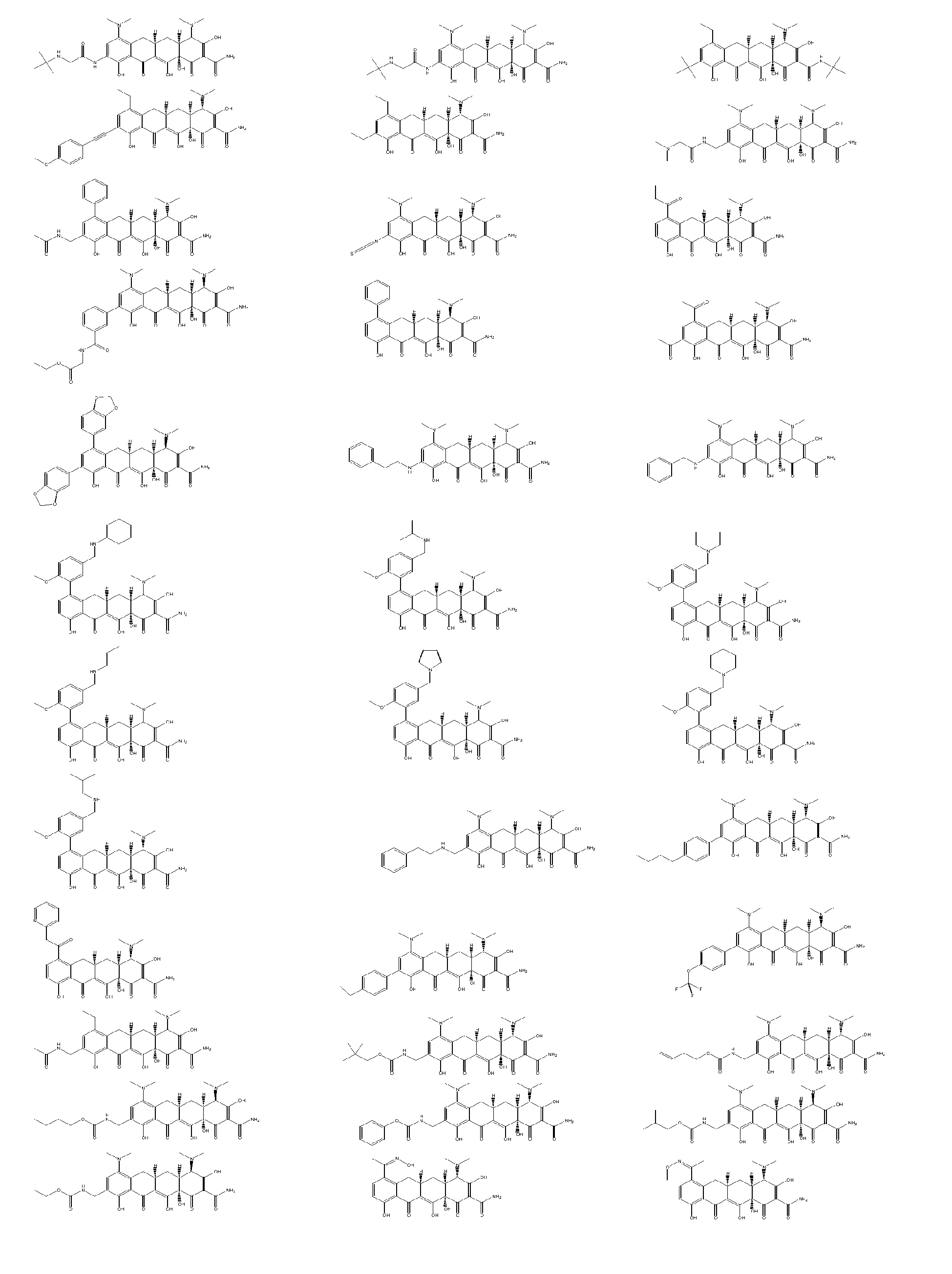

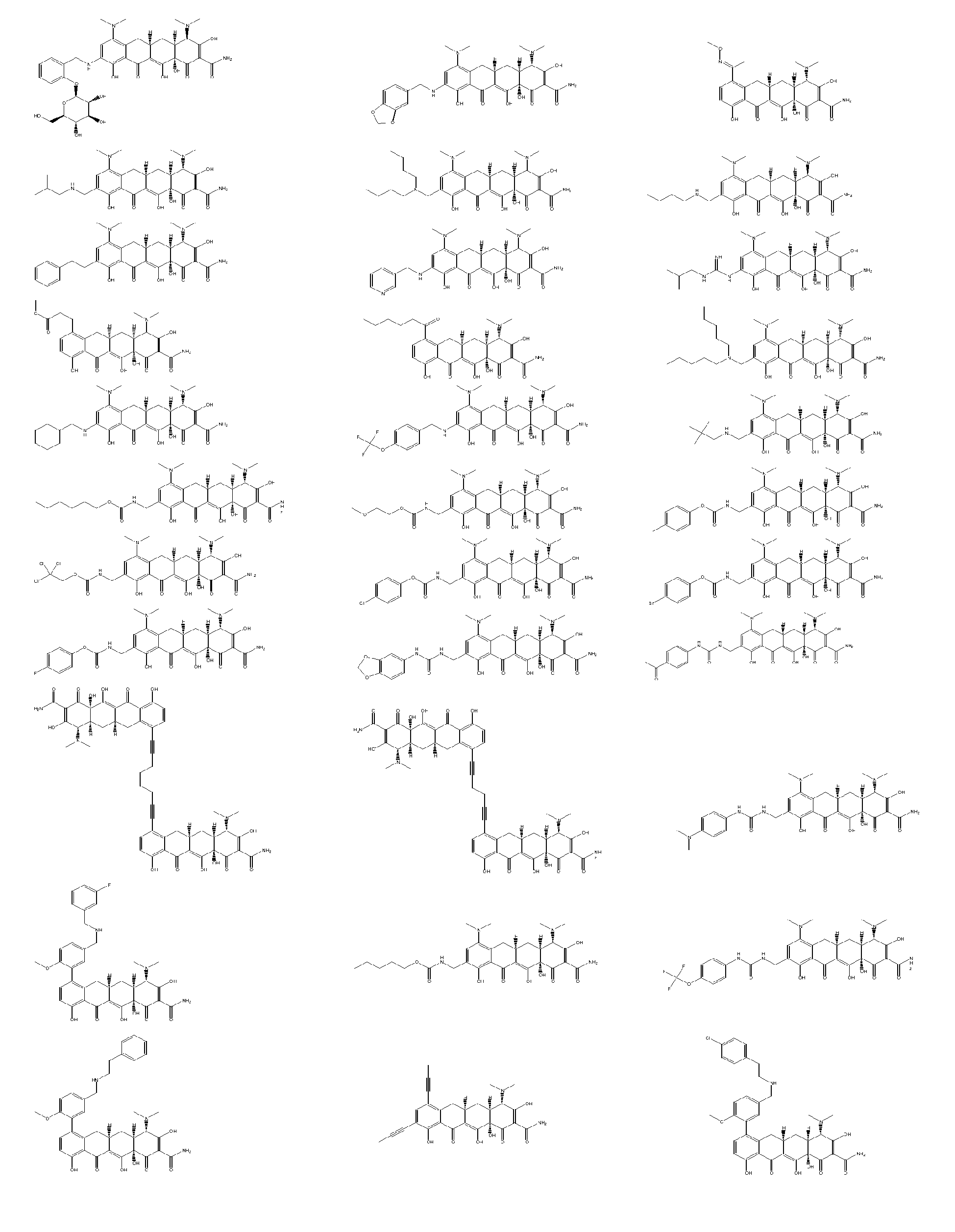

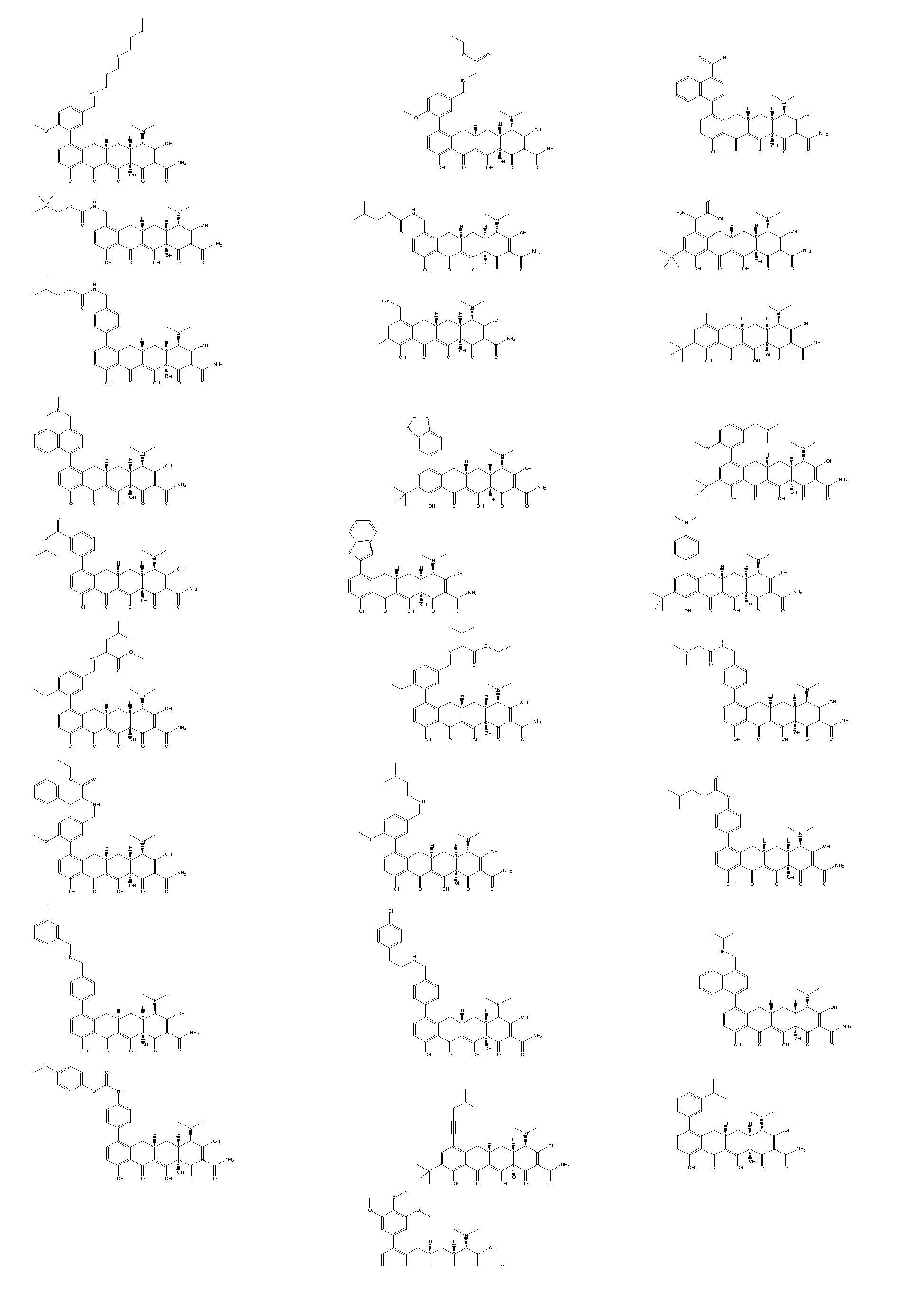

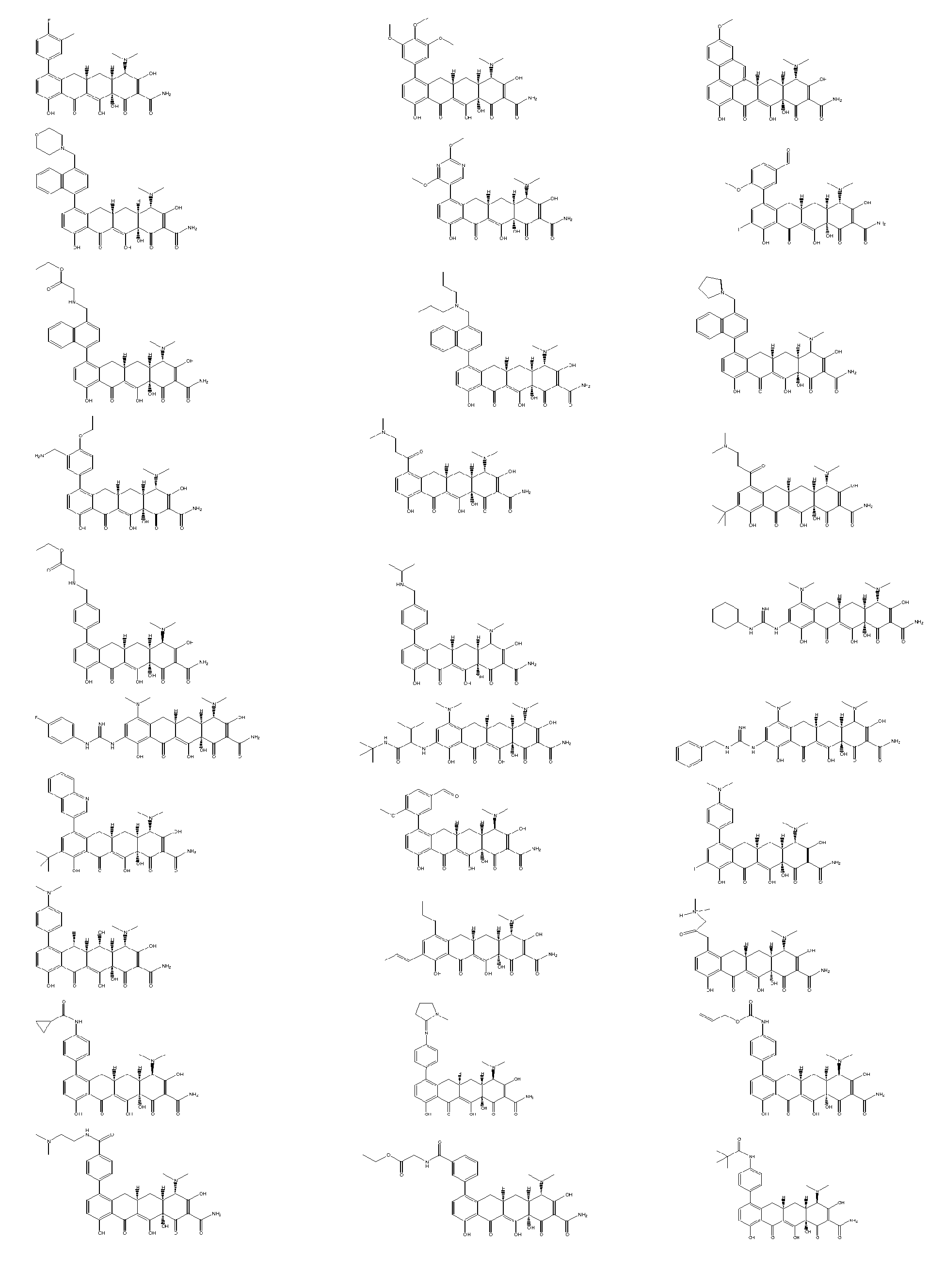

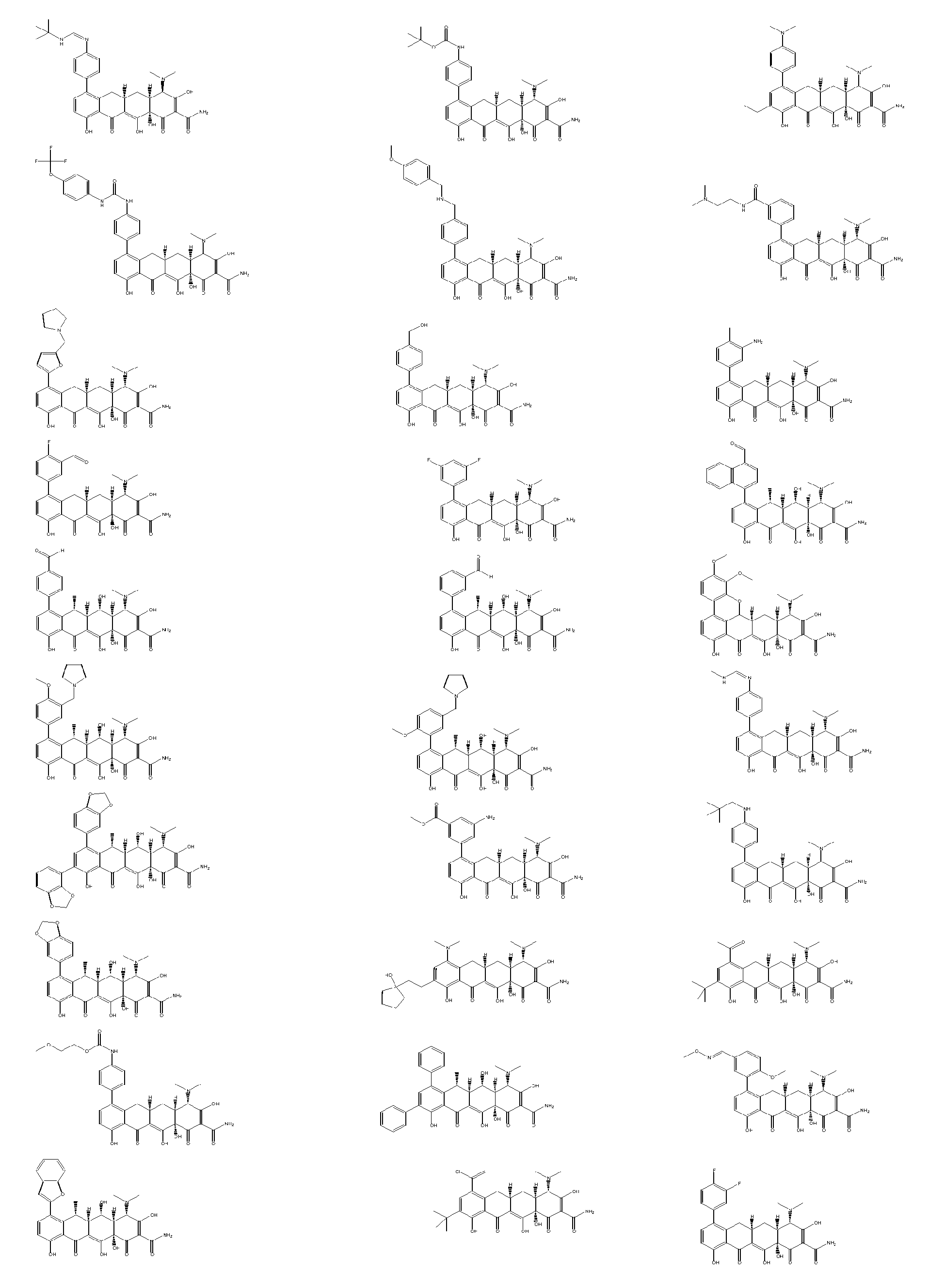

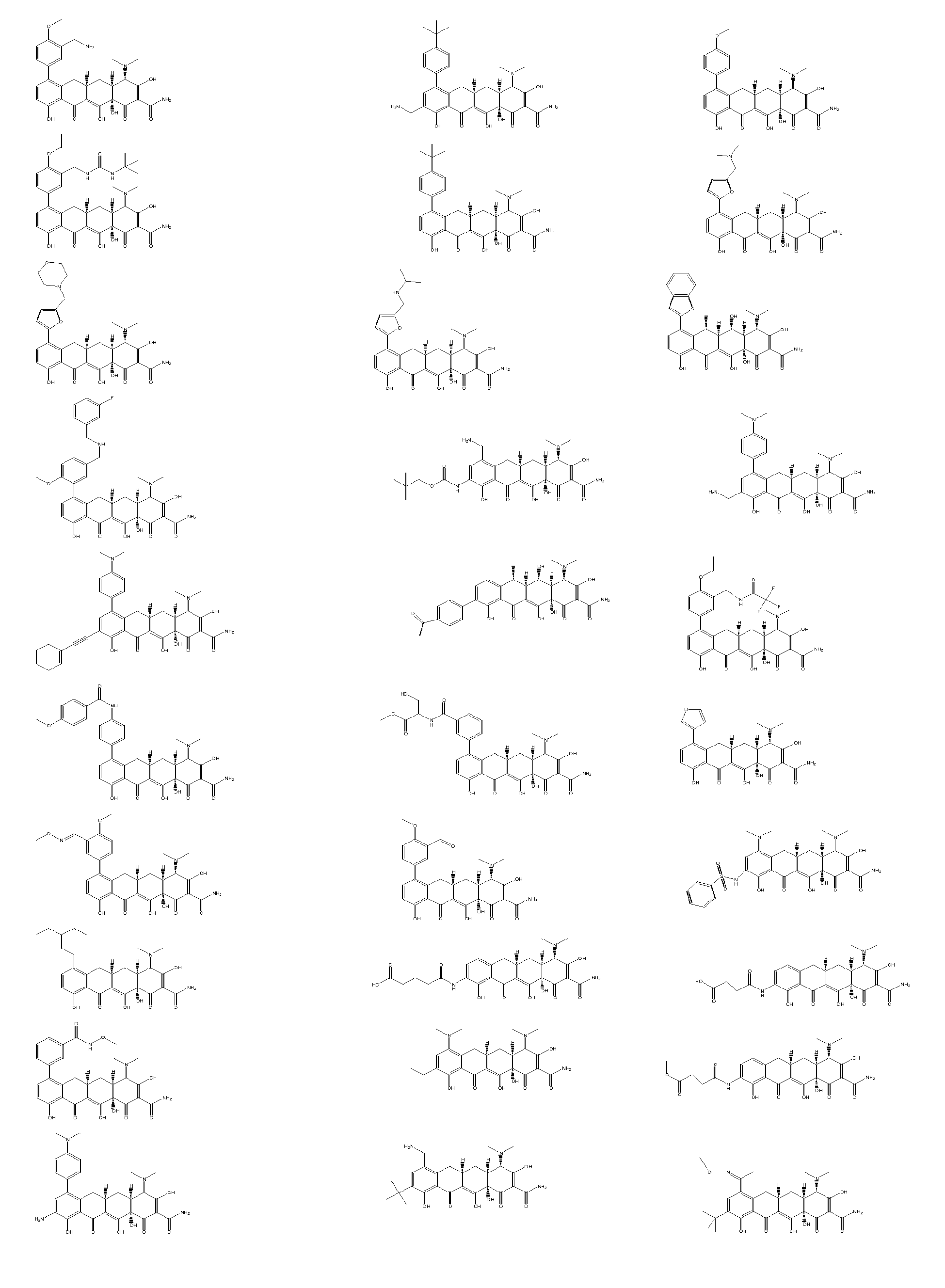

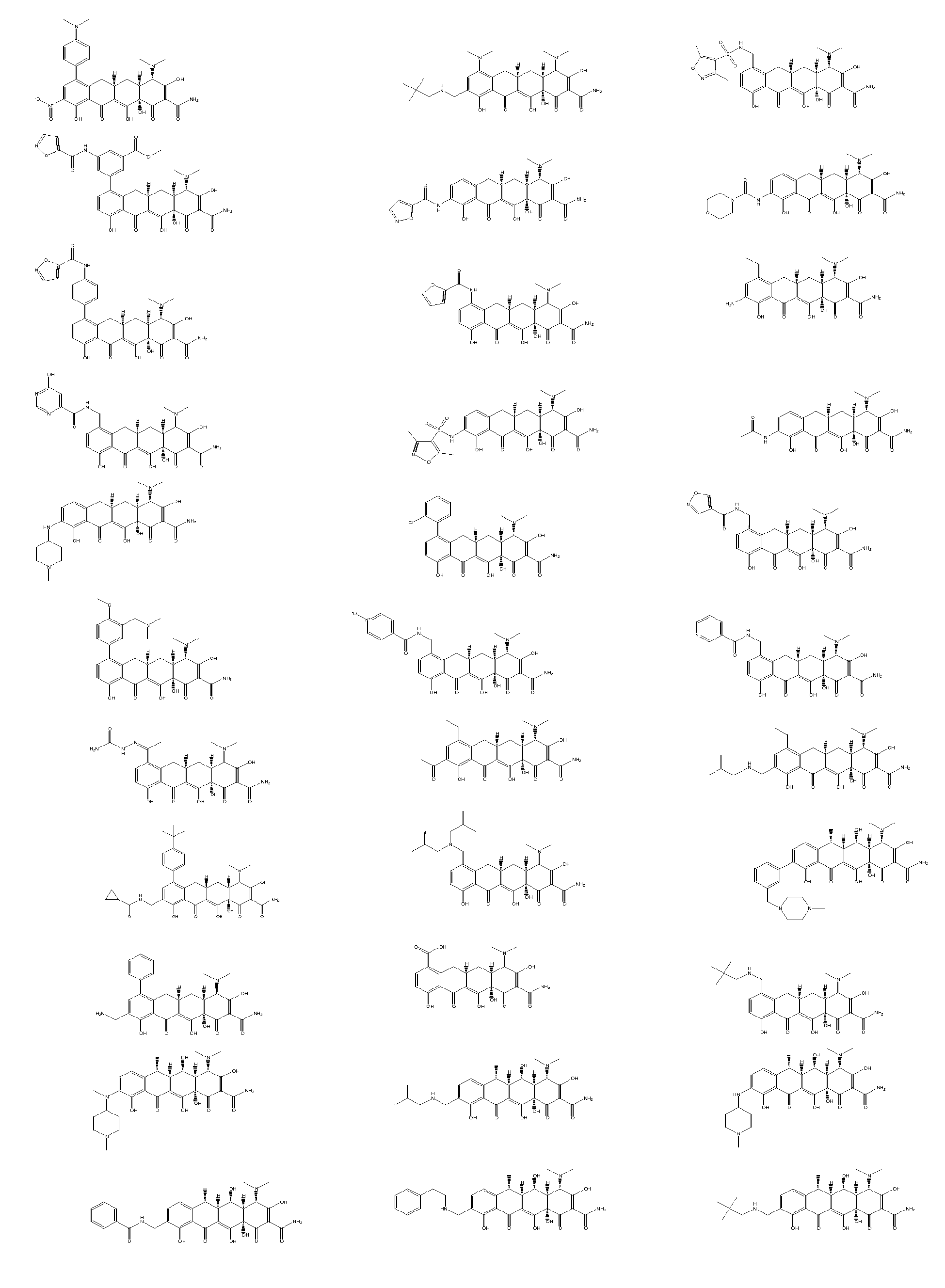

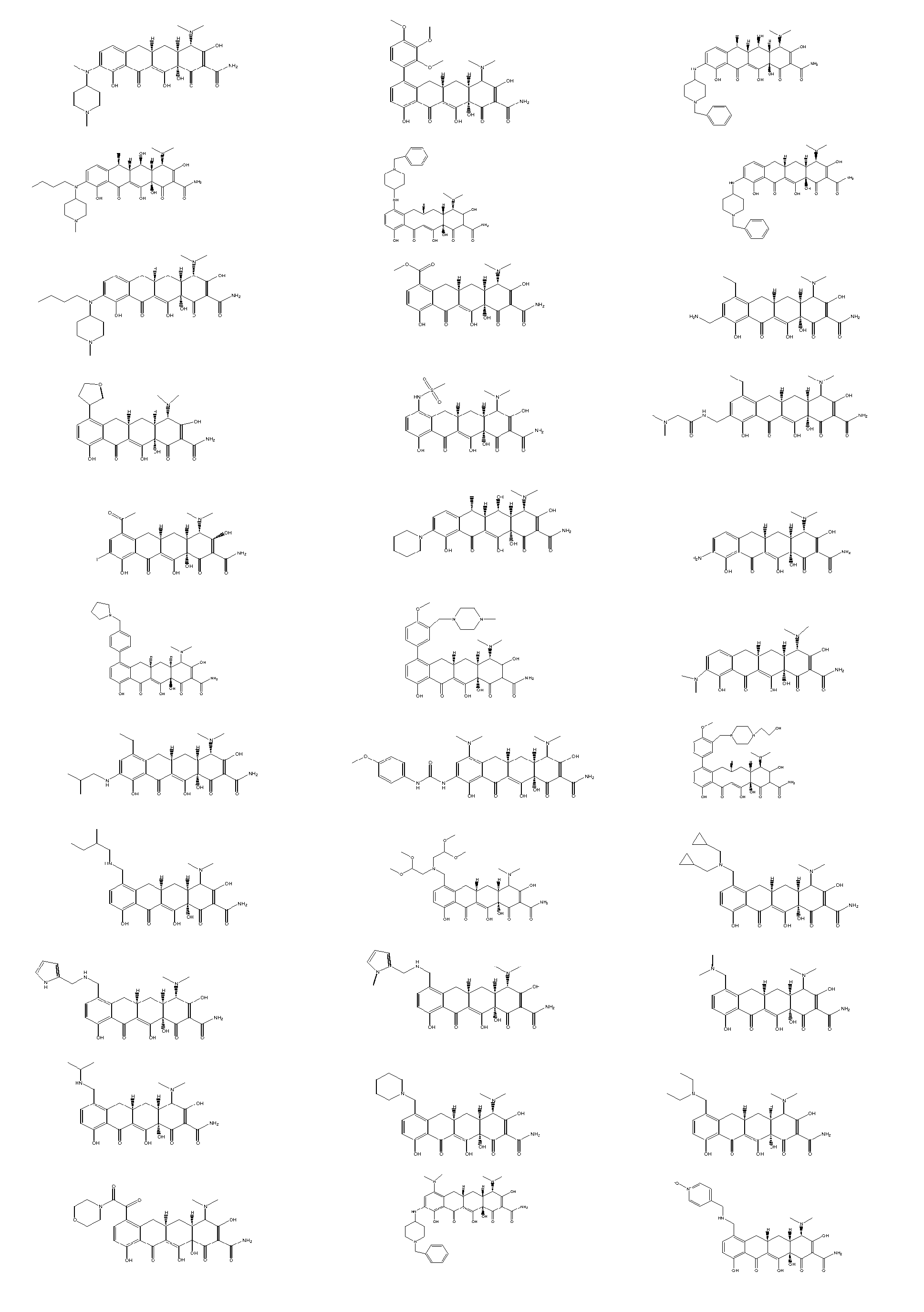

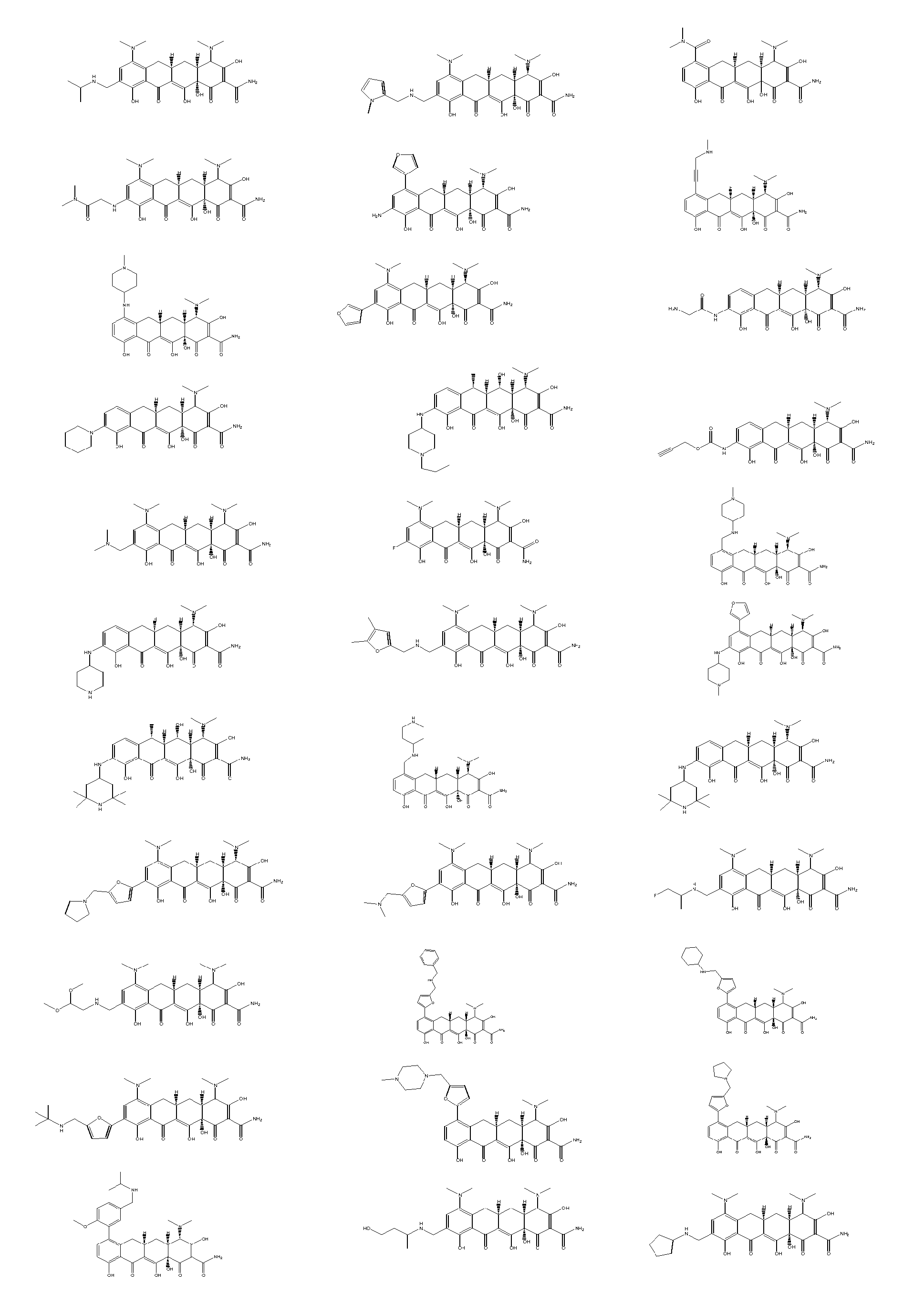

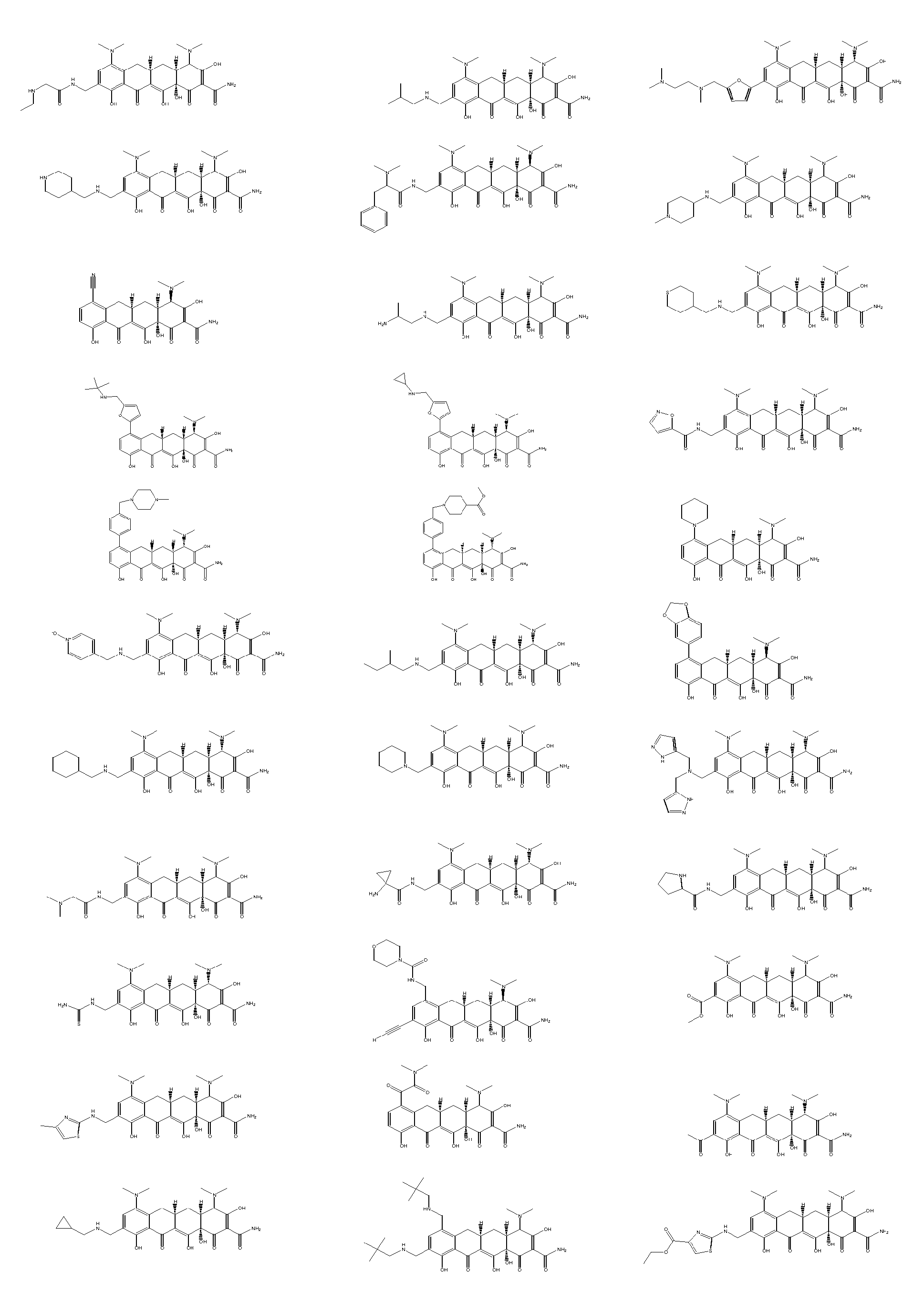

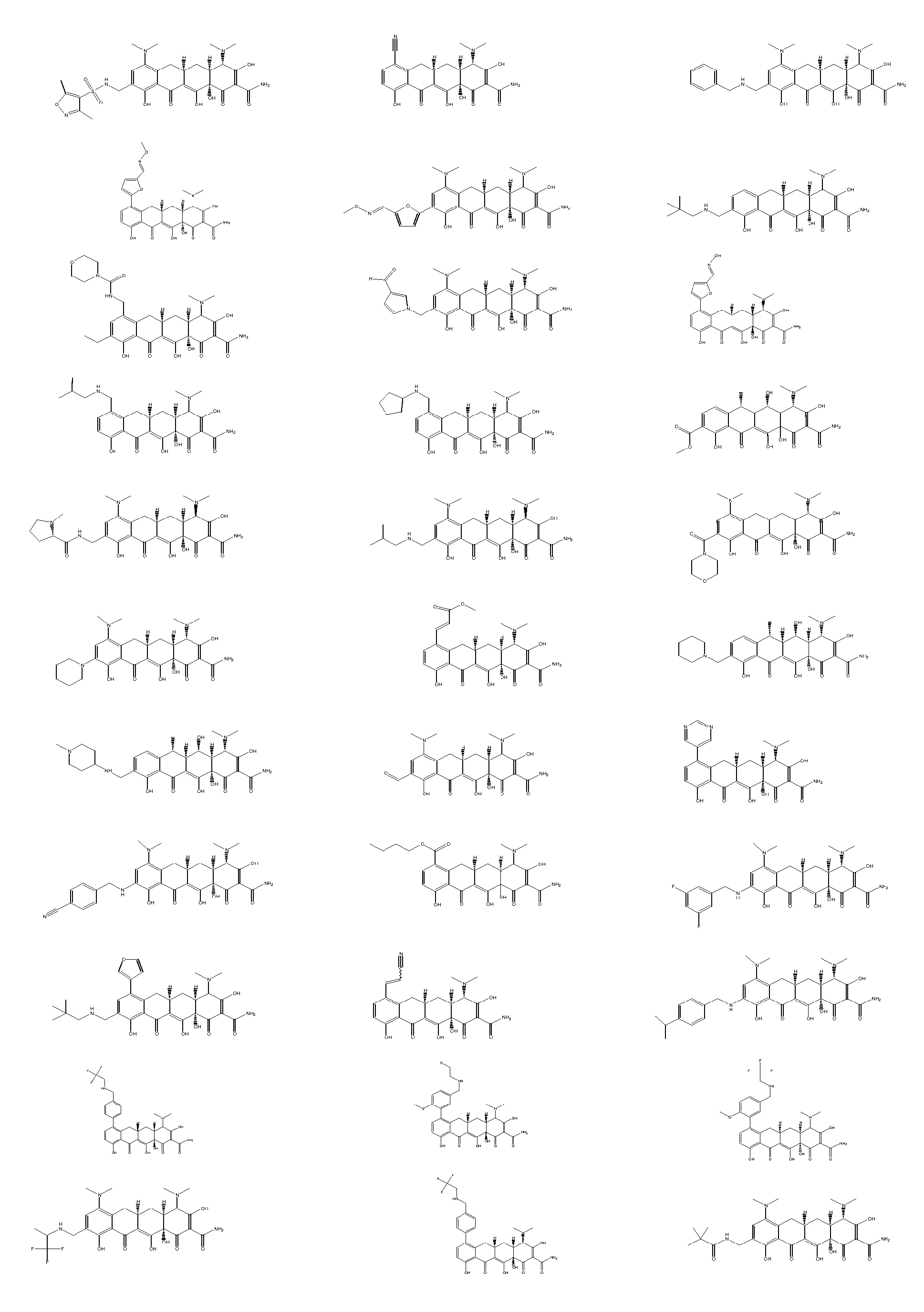

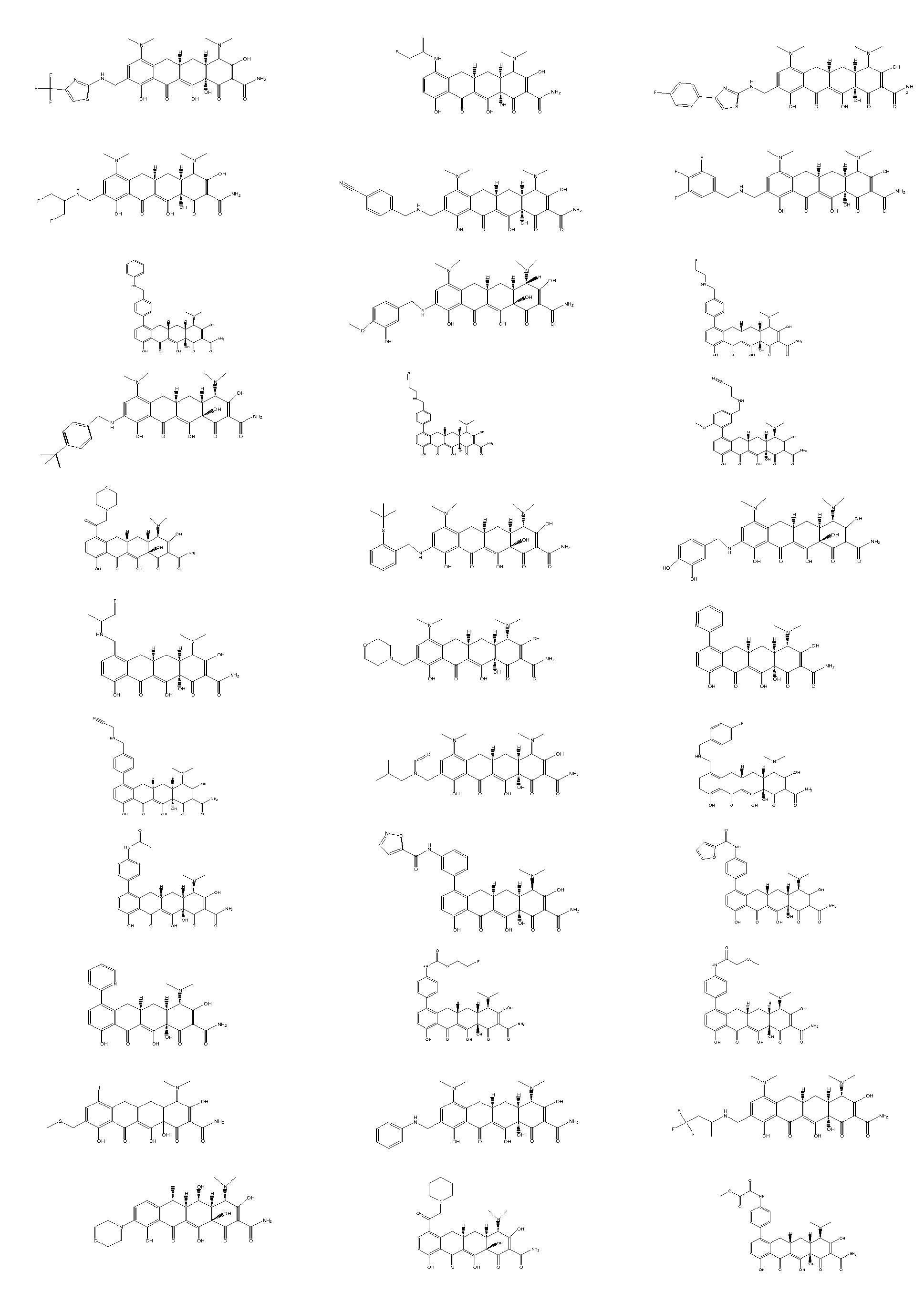

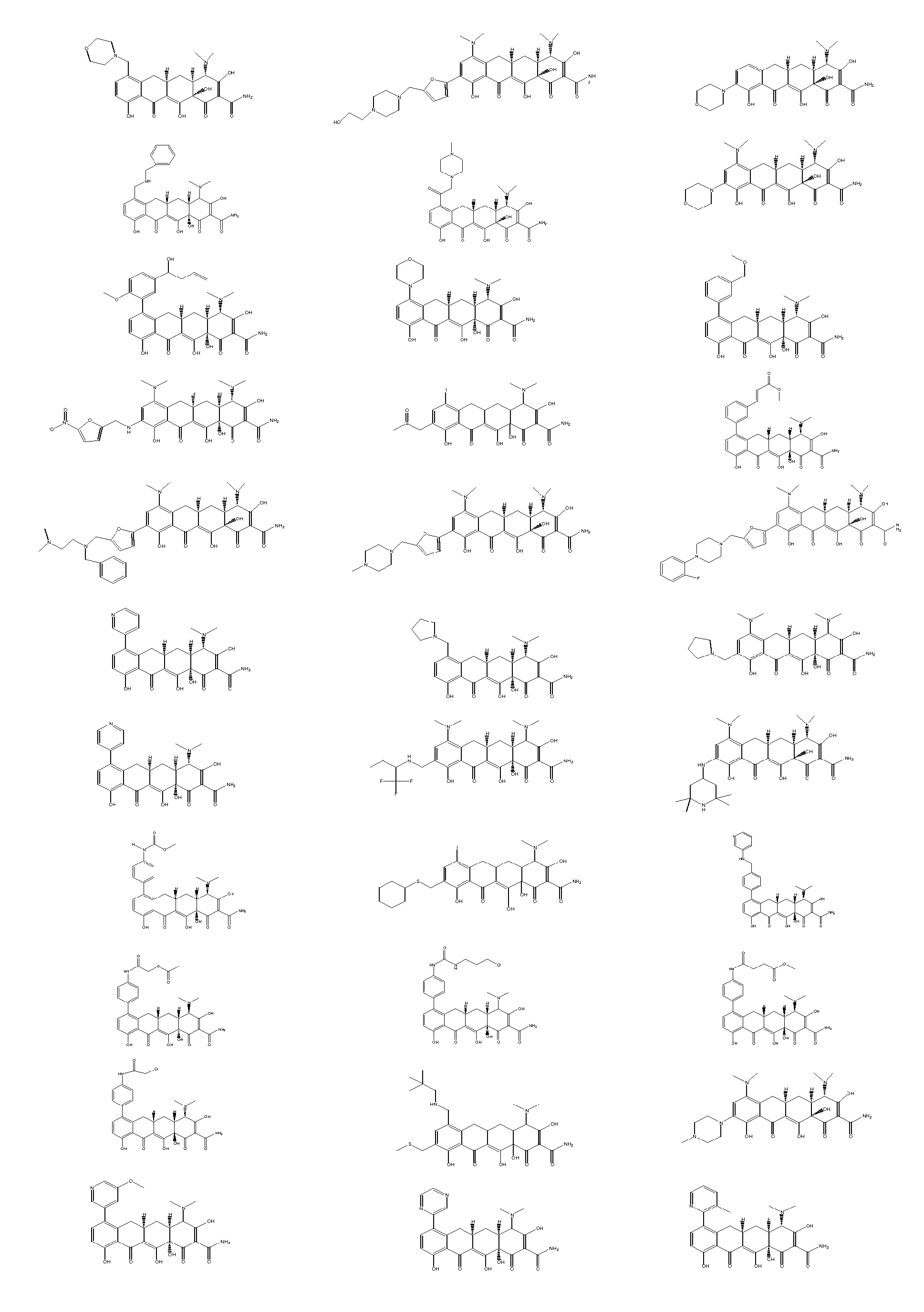

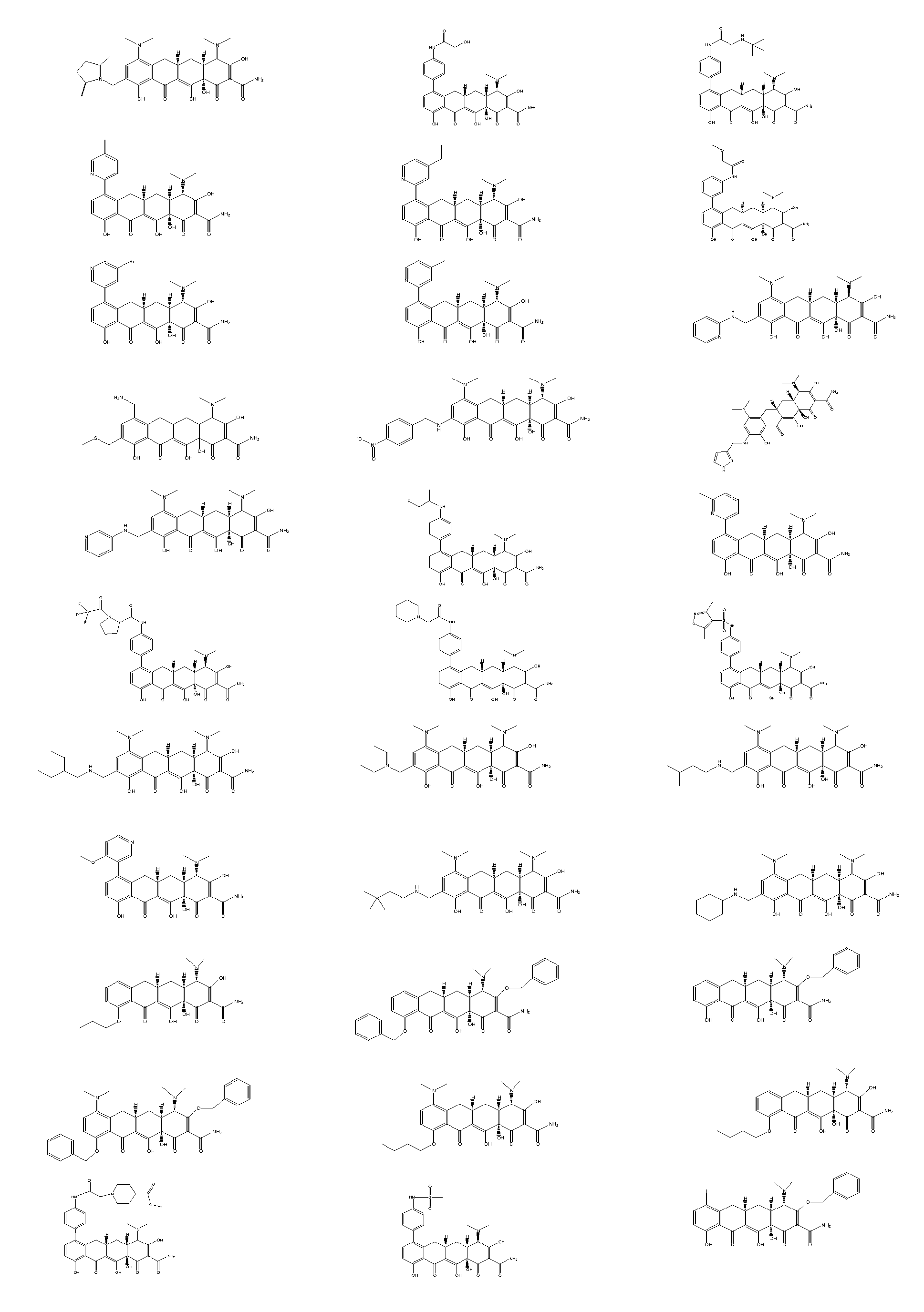

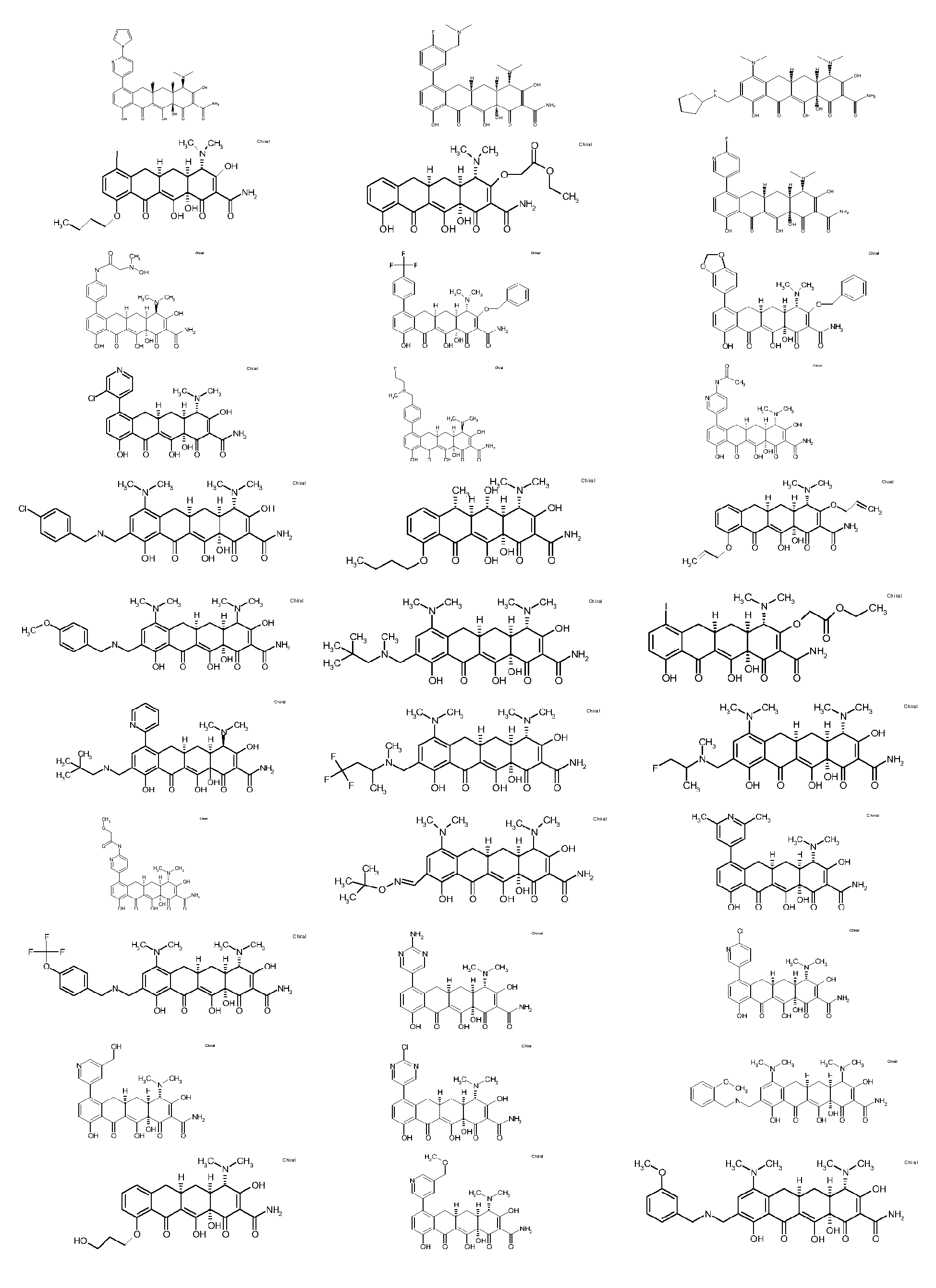

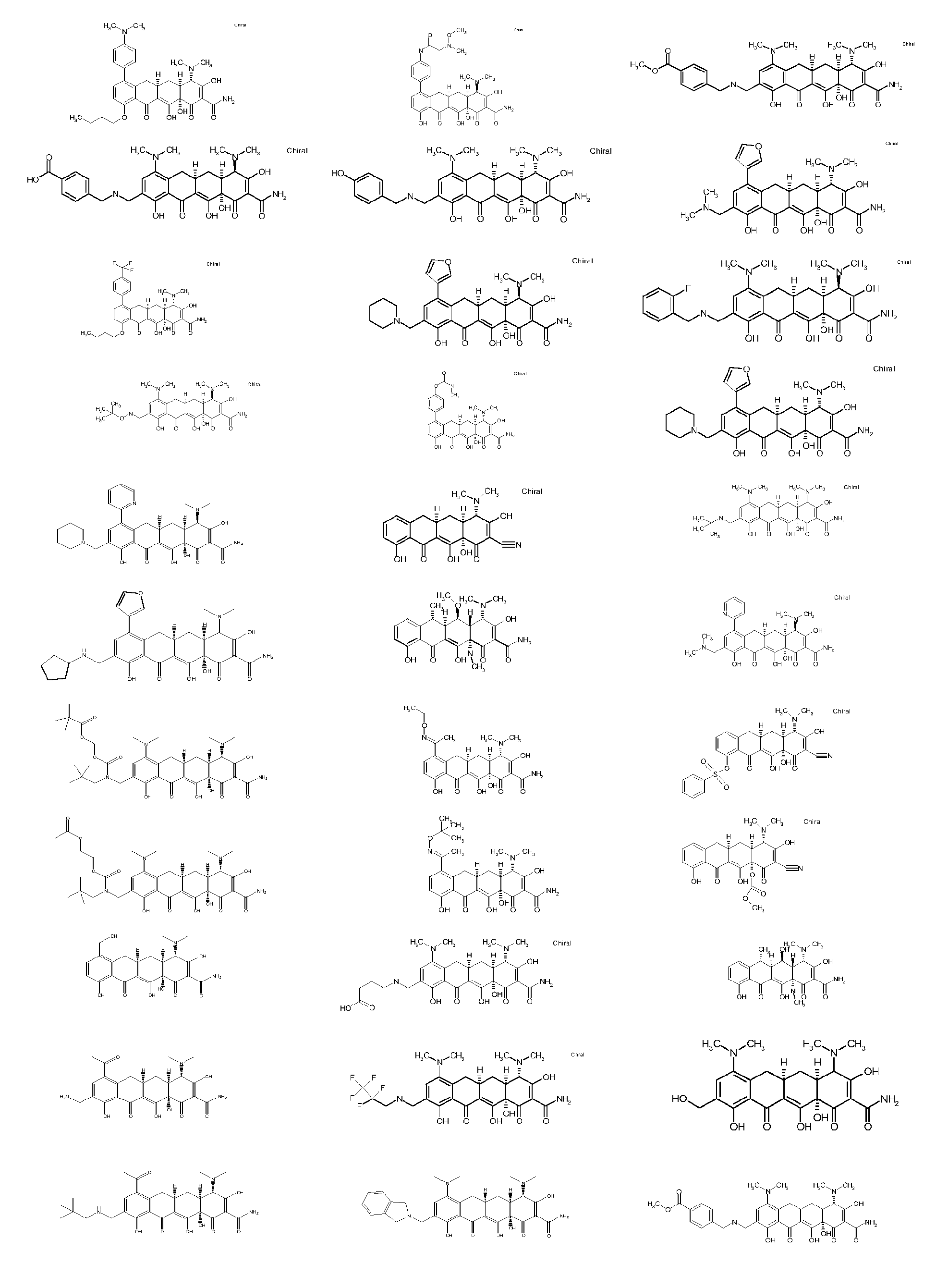

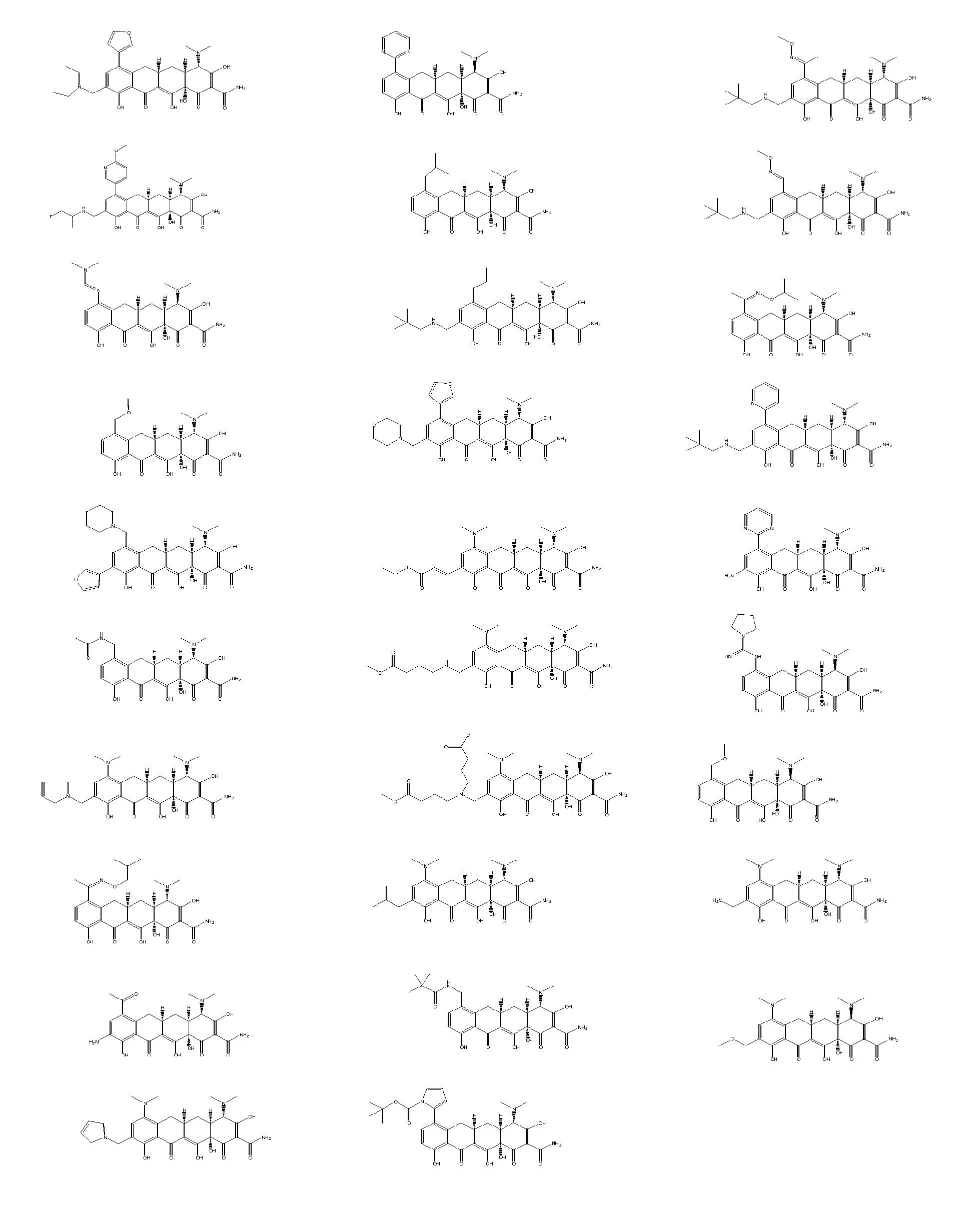

The substituted tetracycline compounds of the invention can be synthesized using the methods described in Example 1, in the following schemes and/or by using art recognized techniques. All novel substituted tetracycline compounds described herein are included in the invention as compounds.

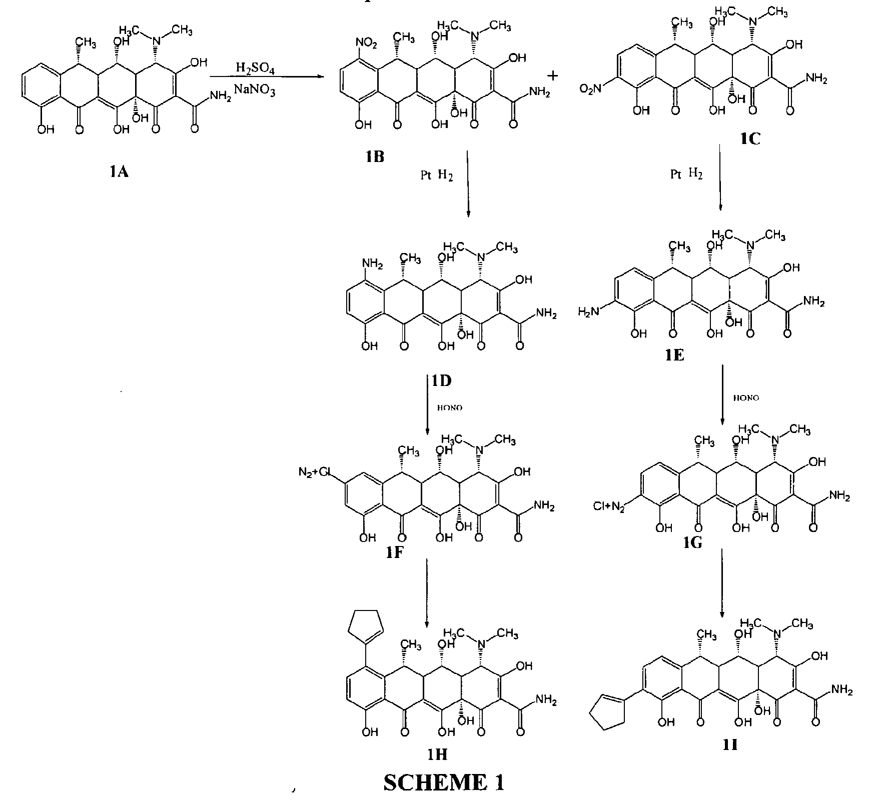

9- and 7- substituted tetracyclines can be synthesized by the method shown in Scheme 1. As shown in Scheme 1, 9- and 7-substituted tetracycline compounds can be synthesized by treating a tetracycline compound (*e.g.*, doxycycline, 1A), with sulfuric acid and sodium nitrate. The resulting product is a mixture of the 7-nitro and 9-nitro isomers (1B and 1C, respectively). The 7-nitro (1B) and 9- nitro (1C) derivatives are treated by hydrogenation using hydrogen gas and a platinum catalyst to yield amines 1 D and 1E. The isomers are separated at this time by conventional methods. To synthesize 7- or 9-substituted alkenyl derivatives, the 7- or 9-amino tetracycline compound (1E and 1F, respectively) is treated with HONO, to yield the diazonium salt (1G and 1H). The salt (1G and 1H) is treated with an appropriate reactive reagent to yield the desired compound(e.g., in Scheme 1, 7-cyclopent-1-enyl doxycycline (1H) and 9-cyclopent-1-enyl doxycycline (1I)).

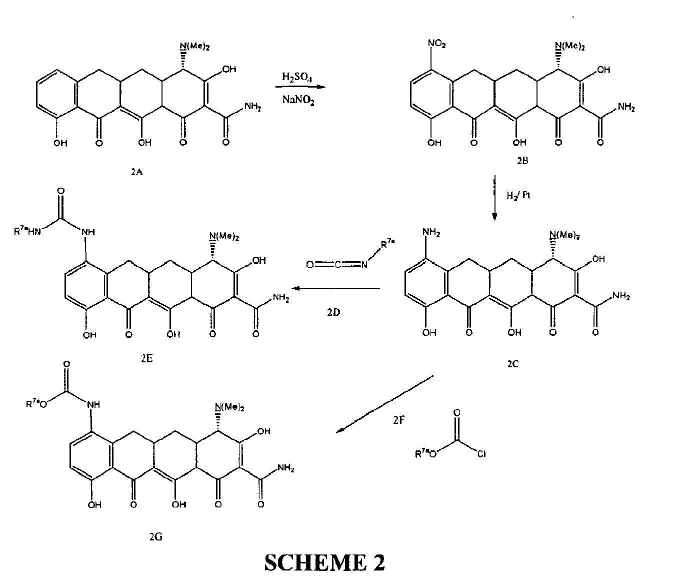

As shown in Scheme 2, tetracycline compounds of the invention wherein R⁷ is a carbamate or a urea derivative can be synthesized using the following protocol. Sancycline (2A) is treated with NaNO₂ under acidic conditions forming 7-nitro sancycline (2B) in a mixture of positional isomers. 7-nitrosancycline (2B) is then treated with H₂ gas and a platinum catalyst to form the 7-amino sancycline derivative (2C). To form the urea derivative (2E), isocyanate (2D) is reacted with the 7-amino sancycline derivative (2C). To form the carbamate (2G), the appropriate acid chloride ester (2F) is reacted with 2C.

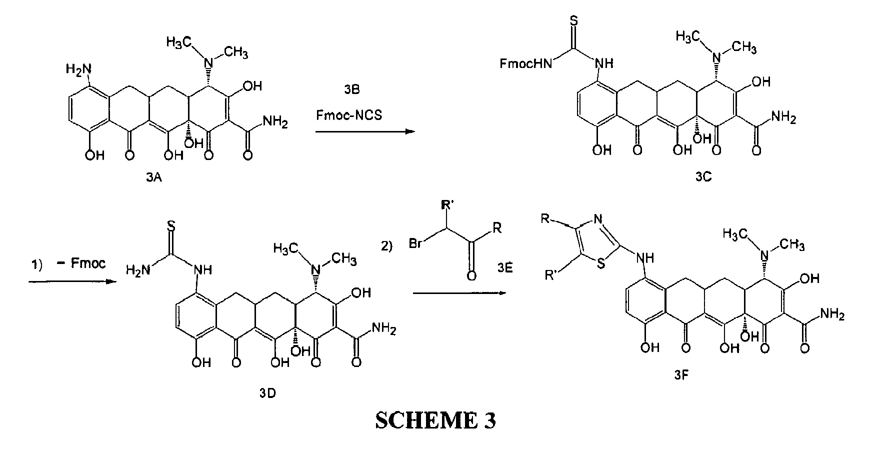

As shown in Scheme 3, tetracycline compounds of the invention, wherein R⁷ is a heterocyclic (i.e. thiazole) substituted amino group can be synthesized using the above protocol. 7-amino sancycline (3A) is reacted with Fmoc-isothiocyanate (3B) to produce the protected thiourea (3C). The protected thiourea (3C) is then deprotected yielding the active sancycline thiourea (3D) compound. The sancycline thiourea (3D) is reacted with an α-haloketone (3E) to produce a thiazole substituted 7-amino sancycline (3F).

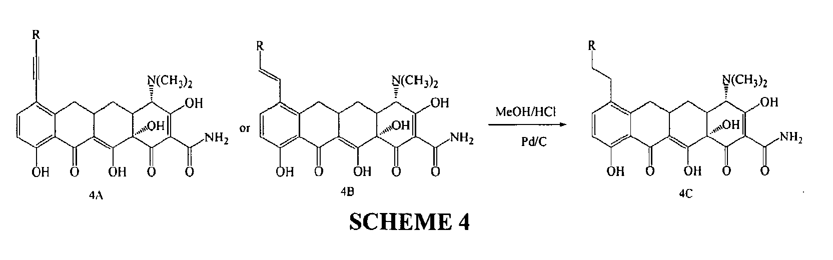

7- alkenyl tetracycline compounds, such as 7-alkynyl sancycline (4A) and 7-alkenyl sancycline (4B), can be hydrogenated to form 7-alkyl substituted tetracycline compounds (e.g., 7-alkyl sancycline, 4C). Scheme 4 depicts the selective hydrogenation of the 7- position double or triple bond, in saturated methanol and hydrochloric acid solution with a palladium/carbon catalyst under pressure, to yield the product.

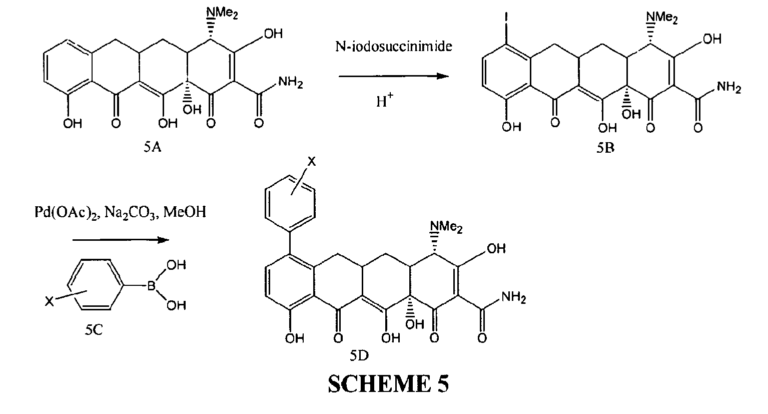

In Scheme 5, a general synthetic scheme for synthesizing 7-position aryl derivatives is shown. A Suzuki coupling of an aryl boronic acid with an iodosancycline compound is shown. An iodo sancycline compound (5B) can be synthesized from sancycline by treating sancycline (5A) with at least one equivalent N-iodosuccinimide (NIS) under acidic conditions. The reaction is quenched, and the resulting 7-iodo sancycline (5B) can then be purified using standard techniques known in the art. To form the aryl derivative, 7-iodo sancycline (5B) is treated with an aqueous base (e.g., Na₂CO₃) and an appropriate boronic acid (5C) and under an inert atmosphere. The reaction is catalyzed with a palladium catalyst (e.g., Pd(OAc)₂). The product (5D) can be purified by methods known in the art (such as HPLC). Other 7-aryl, alkenyl, and alkynyl tetracycline compounds can be synthesized using similar protocols.

The 7-substituted tetracycline compounds of the invention can also be synthesized using Stille cross couplings. Stille cross couplings can be performed using an appropriate tin reagent (e.g., R-SnBu₃) and a halogenated tetracycline compound, (e.g., 7-iodosancycline). The tin reagent and the iodosancycline compound can be treated with a palladium catalyst (e.g., Pd(PPh₃)₂Cl₂ or Pd(AsPh₃)₂Cl₂) and, optionally, with an additional copper salt, e.g., CuI. The resulting compound can then be purified using techniques known in the art.

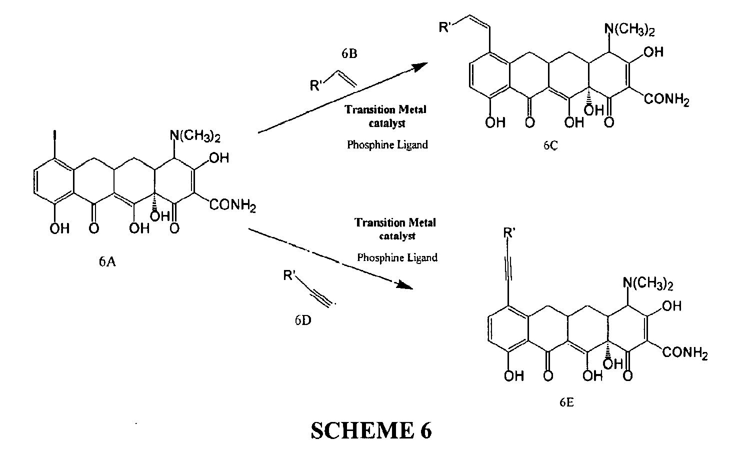

The compounds of the invention can also be synthesized using Heck-type cross coupling reactions. As shown in Scheme 6, Heck-type cross-couplings can be performed by suspending a halogenated tetracycline compound (e.g., 7-iodosancycline, 6A) and an appropriate palladium or other transition metal catalyst (e.g., Pd(OAc)₂ and CuI) in an appropriate solvent (e.g., degassed acetonitrile). The substrate, a reactive alkene (6B) or alkyne (6D), and triethylamine are then added and the mixture is heated for several hours, before being cooled to room temperature. The resulting 7-substituted alkenyl (6C) or 7-substituted alkynyl (6E) tetracycline compound can then be purified using techniques known in the art.

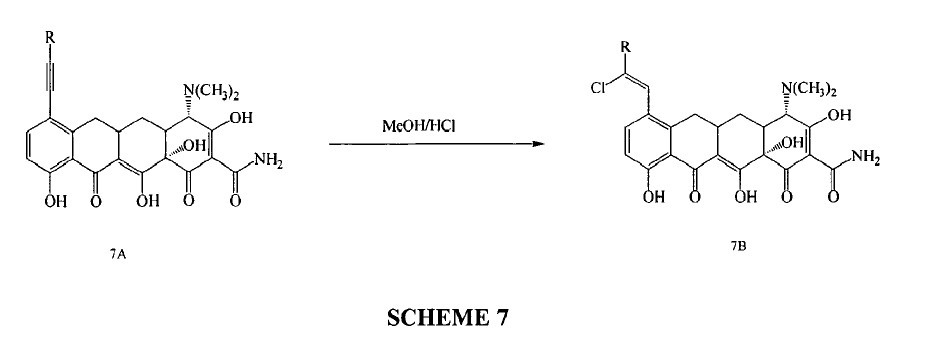

To prepare 7-(2'-Chloro-alkenyl)-tetracycline compounds, the appropriate 7-(alkynyl)-sancycline (7A) is dissolved in saturated methanol and hydrochloric acid and stirred. The solvent is then removed to yield the product (7B).

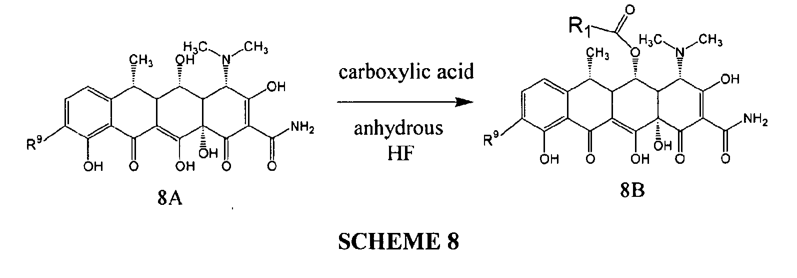

As depicted in Scheme 8, 5-esters of 9- substituted tetracycline compounds can be formed by dissolving the 9- substituted compounds (8A) in strong acid (e.g. HF, methanesulphonic acid, and trifluoromethanesulfonic acid) and adding the appropriate carboxylic acid to yield the corresponding esters (8B).

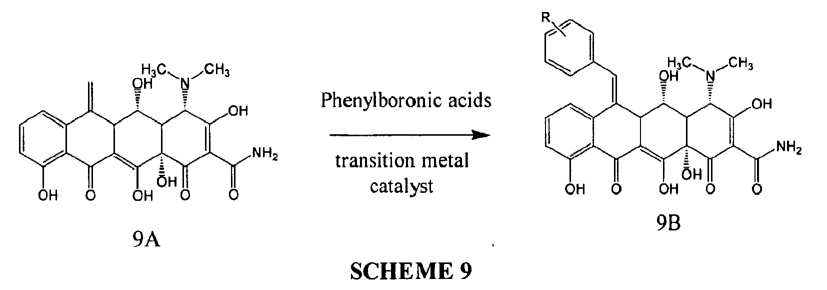

As shown in Scheme 9, methacycline (9A) can be reacted with a phenylboronic acid in the presence of a palladium catalyst such as Pd(OAc)₂ to form a 13 aryl substituted methacycline compound. The resulting compound can then be purified using techniques known in the art such as preparative HPLC and characterized.

As shown in Scheme 10 below, 7 and 9 aminomethyl tetracyclines may be synthesized using reagents such as hydroxymethyl-carbamic acid benzyl ester.

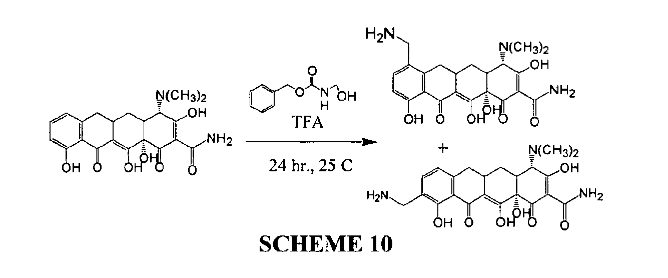

Substituted tetracycline compounds substituted at the 3, 10 or 12a position can be synthesized by contacting the tetracycline compound with a base to deprotonate the hydroxyl group. Examples of bases that can be used include potassium hydride and sodium hydroxide. The tetracyclines can then be further derivatized by using halides and other reactive species known in the art.

Other examples of chemical syntheses are described in provisional patent applications filed on July 12, 2002 entitled "Amino-Methyl Substituted Tetracycline Compounds," "3, 10, and 12a Substituted Tetracycline Compounds," and "Substituted Tetracycline Compounds," the entire contents of each of which are hereby incorporated herein by reference.

The term "alkyl" includes saturated aliphatic groups, including straight-chain alkyl groups (*e.g.*, methyl, ethyl, propyl, butyl, pentyl, hexyl, heptyl, octyl, nonyl, decyl, *etc.*)*,* branched-chain alkyl groups (*e.g.*, isopropyl, tert-butyl, isobutyl, *etc*.), cycloalkyl (alicyclic) groups (*e.g.*, cyclopropyl, cyclopentyl, cyclohexyl, cycloheptyl, cyclooctyl), alkyl substituted cycloalkyl groups, and cycloalkyl substituted alkyl groups. The term alkyl further includes alkyl groups, which can further include oxygen, nitrogen, sulfur or phosphorous atoms replacing one or more carbons of the hydrocarbon backbone. In certain embodiments, a straight chain or branched chain alkyl has 20 or fewer carbon atoms in its backbone (*e.g.*, C₁-C₂₀ for straight chain, C₃-C₂₀ for branched chain), and more preferably 4 or fewer. Cycloalkyls may have from 3-8 carbon atoms in their ring structure, and more preferably have 5 or 6 carbons in the ring structure. The term C₁-C₆ includes alkyl groups containing 1 to 6 carbon atoms.

Moreover, the term alkyl includes both "unsubstituted alkyls" and "substituted alkyls", the latter of which refers to alkyl moieties having substituents replacing a hydrogen on one or more carbons of the hydrocarbon backbone. Such substituents can include, for example, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Cycloalkyls can be further substituted, *e.g.*, with the substituents described above. An "alkylaryl" or an "arylalkyl" moiety is an alkyl substituted with an aryl (*e.g.*, phenylmethyl (benzyl)). The term "alkyl" also includes the side chains of natural and unnatural amino acids.

The term "aryl" includes groups, including 5- and 6-membered single-ring aromatic groups that may include from zero to four heteroatoms, for example, benzene, phenyl, pyrrole, furan, thiophene, thiazole, isothiazole, imidazole, triazole, tetrazole, pyrazole, oxazole, isooxazole, pyridine, pyrazine, pyridazine, and pyrimidine, and the like. Furthermore, the term "aryl" includes multicyclic aryl groups, *e.g.*, tricyclic, bicyclic, *e.g.*, naphthalene, benzoxazole, benzodioxazole, benzothiazole, benzoimidazole, benzothiophene, methylenedioxophenyl, quinoline, isoquinoline, naphthridine, indole, benzofuran, purine, benzofuran, deazapurine, or indolizine. Those aryl groups having heteroatoms in the ring structure may also be referred to as "aryl heterocycles", "heterocycles," "heteroaryls" or "heteroaromatics". The aromatic ring can be substituted at one or more ring positions with such substituents as described above, as for example, halogen, hydroxyl, alkoxy, alkylcarbonyloxy, arylcarbonyloxy, alkoxycarbonyloxy, aryloxycarbonyloxy, carboxylate, alkylcarbonyl, alkylaminocarbonyl, arylalkyl aminocarbonyl, alkenylaminocarbonyl, alkylcarbonyl, arylcarbonyl, arylalkylcarbonyl, alkenylcarbonyl, alkoxycarbonyl, aminocarbonyl, alkylthiocarbonyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Aryl groups can also be fused or bridged with alicyclic or heterocyclic rings which are not aromatic so as to form a polycycle (*e.g.*, tetralin).

The term "alkenyl" includes unsaturated aliphatic groups analogous in length and possible substitution to the alkyls described above, but that contain at least one double bond.

For example, the term "alkenyl" includes straight-chain alkenyl groups (*e.g.*, ethylenyl, propenyl, butenyl, pentenyl, hexenyl, heptenyl, octenyl, nonenyl, decenyl, *etc.*), branched-chain alkenyl groups, cycloalkenyl (alicyclic) groups (cyclopropenyl, cyclopentenyl, cyclohexenyl, cycloheptenyl, cyclooctenyl), alkyl or alkenyl substituted cycloalkenyl groups, and cycloalkyl or cycloalkenyl substituted alkenyl groups. The term alkenyl further includes alkenyl groups which include oxygen, nitrogen, sulfur or phosphorous atoms replacing one or more carbons of the hydrocarbon backbone. In certain embodiments, a straight chain or branched chain alkenyl group has 20 or fewer carbon atoms in its backbone (*e.g.*, C₂-C₂₀ for straight chain, C₃-C₂₀ for branched chain). Likewise, cycloalkenyl groups may have from 3-8 carbon atoms in their ring structure, and more preferably have 5 or 6 carbons in the ring structure. The term C₂-C₂₀ includes alkenyl groups containing 2 to 20 carbon atoms.

Moreover, the term alkenyl includes both "unsubstituted alkenyls" and "substituted alkenyls", the latter of which refers to alkenyl moieties having substituents replacing a hydrogen on one or more carbons of the hydrocarbon backbone. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

The term "alkynyl" includes unsaturated aliphatic groups analogous in length and possible substitution to the alkyls described above, but which contain at least one triple bond.

For example, the term "alkynyl" includes straight-chain alkynyl groups (*e.g.*, ethynyl, propynyl, butynyl, pentynyl, hexynyl, heptynyl, octynyl, nonynyl, decynyl, *etc.*), branched-chain alkynyl groups, and cycloalkyl or cycloalkenyl substituted alkynyl groups. The term alkynyl further includes alkynyl groups which include oxygen, nitrogen, sulfur or phosphorous atoms replacing one or more carbons of the hydrocarbon backbone. In certain embodiments, a straight chain or branched chain alkynyl group has 20 or fewer carbon atoms in its backbone (*e.g.*, C₂-C₂₀ for straight chain, C₃-C₂₀ for branched chain). The term C₂-C₆ includes alkynyl groups containing 2 to 6 carbon atoms.

Moreover, the term alkynyl includes both "unsubstituted alkynyls" and "substituted alkynyls", the latter of which refers to alkynyl moieties having substituents replacing a hydrogen on one or more carbons of the hydrocarbon backbone. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including, *e.g.*, alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

Unless the number of carbons is otherwise specified, "lower alkyl" as used herein means an alkyl group, as defined above, but having from one to five carbon atoms in its backbone structure. "Lower alkenyl" and "lower alkynyl" have chain lengths of, for example, 2-5 carbon atoms.

The term "acyl" includes compounds and moieties which contain the acyl radical (CH₃CO-) or a carbonyl group. The term "substituted acyl" includes acyl groups where one or more of the hydrogen atoms are replaced by for example, alkyl groups, alkenyl, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

The term "acylamino" includes moieties wherein an acyl moiety is bonded to an amino group. For example, the term includes alkylcarbonylamino, arylcarbonylamino, carbamoyl and ureido groups.

The term "alkoxy" includes substituted and unsubstituted alkyl, alkenyl, and alkynyl groups covalently linked to an oxygen atom. Examples of alkoxy groups include methoxy, ethoxy, isopropyloxy, propoxy, butoxy, and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy, trichloromethoxy, *etc.*

The terms "alkoxyalkyl", "alkylaminoalkyl" and "thioalkoxyalkyl" include alkyl groups, as described above, which further include oxygen, nitrogen or sulfur atoms replacing one or more carbons of the hydrocarbon backbone, *e.g.*, oxygen, nitrogen or sulfur atoms.

The term "amide" or "aminocarboxy" includes compounds or moieties which contain a nitrogen atom which is bound to the carbon of a carbonyl or a thiocarbonyl group. The term includes "alkaminocarboxy" groups which include alkyl, alkenyl, or alkynyl groups bound to an amino group bound to a carboxy group. It includes arylaminocarboxy groups which include aryl or heteroaryl moieties bound to an amino group which is bound to the carbon of a carbonyl or thiocarbonyl group. The terms "alkylaminocarboxy," "alkenylaminocarboxy," "alkynylaminocarboxy," and "arylaminocarboxy" include moieties wherein alkyl, alkenyl, alkynyl and aryl moieties, respectively, are bound to a nitrogen atom which is in turn bound to the carbon of a carbonyl group.

The term "amine" or "amino" includes compounds where a nitrogen atom is covalently bonded to at least one carbon or heteroatom. The term "alkyl amino" includes groups and compounds wherein the nitrogen is bound to at least one additional alkyl group. The term "dialkyl amino" includes groups wherein the nitrogen atom is bound to at least two additional alkyl groups. The term "arylamino" and "diarylamino" include groups wherein the nitrogen is bound to at least one or two aryl groups, respectively. The term "alkylarylamino," "alkylaminoaryl" or "arylaminoalkyl" refers to an amino group which is bound to at least one alkyl group and at least one aryl group. The term "alkaminoalkyl" refers to an alkyl, alkenyl, or alkynyl group bound to a nitrogen atom which is also bound to an alkyl group.

The term "aroyl" includes compounds and moieties with an aryl or heteroaromatic moiety bound to a carbonyl group. Examples of aroyl groups include phenylcarboxy, naphthyl carboxy, *etc.*

The term "carbonyl" or "carboxy" includes compounds and moieties which contain a carbon connected with a double bond to an oxygen atom. Examples of moieties which contain a carbonyl include aldehydes, ketones, carboxylic acids, amides, esters, anhydrides, *etc.*

The term "ester" includes compounds and moieties which contain a carbon or a heteroatom bound to an oxygen atom which is bonded to the carbon of a carbonyl group. The term "ester" includes alkoxycarboxy groups such as methoxycarbonyl, ethoxycarbonyl, propoxycarbonyl, butoxycarbonyl, pentoxycarbonyl, *etc.* The alkyl, alkenyl, or alkynyl groups are as defined above.

The term "ether" includes compounds or moieties which contain an oxygen bonded to two different carbon atoms or heteroatoms. For example, the term includes "alkoxyalkyl" which refers to an alkyl, alkenyl, or alkynyl group covalently bonded to an oxygen atom which is covalently bonded to another alkyl group.

The term "halogen" includes fluorine, bromine, chlorine, iodine, *etc.* The term "perhalogenated" generally refers to a moiety wherein all hydrogens are replaced by halogen atoms.

The term "heteroatom" includes atoms of any element other than carbon or hydrogen. Preferred heteroatoms are nitrogen, oxygen, sulfur and phosphorus.

The term "hydroxy" or "hydroxyl" includes groups with an -OH or -O⁻ X⁺, where X⁺ is a counterion.

The terms "polycyclyl" or "polycyclic radical" refer to two or more cyclic rings (*e.g.*, cycloalkyls, cycloalkenyls, cycloalkynyls, aryls and/or heterocyclyls) in which two or more carbons are common to two adjoining rings, *e.g.*, the rings are "fused rings". Rings that are joined through non-adjacent atoms are termed "bridged" rings. Each of the rings of the polycycle can be substituted with such substituents as described above, as for example, halogen, hydroxyl, alkylcarbonyloxy, arylcarbonyloxy, alkoxycarbonyloxy, aryloxycarbonyloxy, carboxylate, alkylcarbonyl, alkoxycarbonyl, alkylaminoacarbonyl, arylalkylaminocarbonyl, alkenylaminocarbonyl, alkylcarbonyl, arylcarbonyl, arylalkyl carbonyl, alkenylcarbonyl, aminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkyl, alkylaryl, or an aromatic or heteroaromatic moiety.

The term "thiocarbonyl" or "thiocarboxy" includes compounds and moieties which contain a carbon connected with a double bond to a sulfur atom.

The term "thioether" includes compounds and moieties which contain a sulfur atom bonded to two different carbon or hetero atoms. Examples of thioethers include, but are not limited to alkthioalkyls, alkthioalkenyls, and alkthioalkynyls. The term "alkthioalkyls" include compounds with an alkyl, alkenyl, or alkynyl group bonded to a sulfur atom which is bonded to an alkyl group. Similarly, the term "alkthioalkenyls" and alkthioalkynyls" refer to compounds or moieties wherein an alkyl, alkenyl, or alkynyl group is bonded to a sulfur atom which is covalently bonded to an alkynyl group.

The term "oximyl" includes moieties which comprise an oxime group.

The term "dimeric moiety" includes moieties which comprise a second tetracycline four ring structure. The dimeric moiety may be attached to the substituted tetracycline through a chain of from 1-30 atoms. The chain may be comprised of atoms covalently linked together through single, double and triple bonds. The tetracycline ring structure of the dimeric moiety may further be substituted or unsubstituted. It may be attached at the 1, 2, 3, 4, 5, 6, 7, 8, 9, 10, 11, 11a, 12, 12a, and/or 13 position. Examples of substituted tetracycline compounds with dimeric moieties are shown in Tables 2 and 3.

The term "prodrug moiety" includes moieties which can be metabolized *in vivo.* Generally, the prodrugs moieties are metabolized *in vivo* by esterases or by other mechanisms to hydroxyl groups or other advantageous groups. Examples of prodrugs and their uses are well known in the art (See, *e.g.*, Berge et al. (1977) "Pharmaceutical Salts", J. Pharm. Sci. 66:1-19). The prodrugs can be prepared *in situ* during the final isolation and purification of the compounds, or by separately reacting the purified compound in its free acid form or hydroxyl with a suitable esterifying agent. Hydroxyl groups can be converted into esters *via* treatment with a carboxylic acid. Examples of prodrug moieties include substituted and unsubstituted, branch or unbranched lower alkyl ester moieties, (*e.g*.*,* propionoic acid esters), lower alkenyl esters, di-lower alkylamino lower-alkyl esters (*e.g.*, dimethylaminoethyl ester), acylamino lower alkyl esters (*e.g.*, acetyloxymethyl ester), acyloxy lower alkyl esters (*e.g.,* pivaloyloxymethyl ester), aryl esters (phenyl ester), aryl-lower alkyl esters (*e.g.*, benzyl ester), substituted (*e.g.*, with methyl, halo, or methoxy substituents) aryl and aryl-lower alkyl esters, amides, lower-alkyl amides, di-lower alkyl amides, and hydroxy amides. Preferred prodrug moieties are propionoic acid esters and acyl esters. Prodrugs which are converted to active forms through other mechanisms *in vivo* are also included.

The structures of some of the substituted tetracycline compounds used in the methods and compositions of the invention include asymmetric carbon atoms. The isomers arising from the chiral atoms (*e.g.*, all enantiomers and diastereomers) are included within the scope of this invention, unless indicated otherwise. Such isomers can be obtained in substantially pure form by classical separation techniques and by stereochemically controlled synthesis. Furthermore, the structures and other compounds and moieties discussed in this application also include all tautomers thereof.

### 3. Pharmaceutical Compositions of the Invention

In an embodiment, the invention also pertains to pharmaceutical compositions comprising an effective amount of a substituted tetracycline compound (or pharmaceutically acceptable salt thereof) of the invention and a pharmaceutically acceptable carrier. The effective amount may be effective to treat any one of the dieseases described above, such as for example, IPAS, neurological disorders, or cancer. The pharmaceutical composition may further comprise a neuroprotective agent or a chemotherapeutic agent as described above.

The language "pharmaceutical composition" includes preparations suitable for administration to mammals, e.g., humans. When the compounds of the present invention are administered as pharmaceuticals to mammals, *e.g.,* humans, they can be given per se or as a pharmaceutical composition containing, for example, 0.1 to 99.5% (more preferably, 0.5 to 90%) of active ingredient in combination with a pharmaceutically acceptable carrier.

The phrase "pharmaceutically acceptable carrier" is art recognized and includes a pharmaceutically acceptable material, composition or vehicle, suitable for administering compounds of the present invention to mammals. The carriers include liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patent. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, α-tocopherol, and the like; and metal chelating agents, such as citric acid, ethylenediamine tetraacetic acid (EDTA), sorbitol, tartaric acid, phosphoric acid, and the like.

Formulations of the present invention include those suitable for oral, nasal, topical, transdermal, buccal, sublingual, rectal, vaginal, pulmonary and/or parenteral administration. The formulations may conveniently be presented in unit dosage form and may be prepared by any methods well known in the art of pharmacy. The amount of active ingredient which can be combined with a carrier material to produce a single dosage form will generally be that amount of the compound which produces a therapeutic effect. Generally, out of one hundred per cent, this amount will range from about 1 per cent to about ninety-nine percent of active ingredient, preferably from about 5 per cent to about 70 per cent, most preferably from about 10 per cent to about 30 per cent.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluent commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert dilutents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane. Sprays also can be delivered by mechanical, electrical, or by other methods known in the art.

Transdermal patches have the added advantage of providing controlled delivery of a compound of the present invention to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the active compound in a polymer matrix or gel.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial, antiparasitic and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility The rate of absorption of the drug then depends upon its rate of dissolution which, in turn, may depend upon crystal size and crystalline form. Alternatively, delayed absorption of a parenterally-administered drug form may be accomplished by dissolving or suspending the drug in an oil vehicle. The compositions also may be formulated such that its elimination is retarded by methods known in the art.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

The preparations of the present invention may be given orally, parenterally, topically, or rectally. They are of course given by forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administration or administration via inhalation is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually. Other methods for administration include via inhalation.

The tetracycline compounds of the invention may also be administered to a subject via stents. The compounds may be administered through the stent or be impregnated in the stent itself.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutics effect. Such an effective dose will generally depend upon the factors described above. Generally, intravenous and subcutaneous doses of the compounds of this invention tor a patient will range from about 0.0001 to about 100 mg per kilogram of body weight per day, more preferably from about 0.01 to about 50 mg per kg per day, and still more preferably from about 1.0 to about 100 mg per kg per day. An effective amount is that amount treats a target disease such as, for example, an IPAS, a neurological disorder, or cancer.

If desired, the effective daily dose of the active compound may be administered as two, three, four, five, six or more sub-doses administered separately at appropriate intervals throughout the day, optionally, in unit dosage forms.

While it is possible for a compound of the present invention to be administered alone, it is preferable to administer the compound as a pharmaceutical composition.

As set out above, certain embodiments of the present compounds can contain a basic functional group, such as amino or alkylamino, and are, thus, capable of forming pharmaceutically acceptable salts with pharmaceutically acceptable acids. The term "pharmaceutically acceptable salts" is art recognized and includes relatively non-toxic, inorganic and organic acid addition salts of compounds of the present invention. These salts can be prepared *in situ* during the final isolation and purification of the compounds of the invention, or by separately reacting a purified compound of the invention in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, *e.g.,* Berge et al. (1977) "Pharmaceutical Salts", J. Farm. SCI. 66:1-19).

In other cases, the compounds of the present invention may contain one or more acidic functional groups and, thus, are capable of forming pharmaceutically acceptable salts with pharmaceutically acceptable bases. The term "pharmaceutically acceptable salts" in these instances includes relatively non-toxic, inorganic and organic base addition salts of compounds of the present invention. These salts can likewise be prepared *in situ* during the final isolation and purification of the compounds, or by separately reacting the purified compound in its free acid form with a suitable base, such as the hydroxide, carbonate or bicarbonate of a pharmaceutically acceptable metal cation, with ammonia, or with a pharmaceutically acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like.

The term "pharmaceutically acceptable esters" refers to the relatively non-toxic, esterified products of the compounds of the present invention. These esters can be prepared *in situ* during the final isolation and purification of the compounds, or by separately reacting the purified compound in its free acid form or hydroxyl with a suitable esterifying agent. Carboxylic acids can be converted into esters *via* treatment with an alcohol in the presence of a catalyst. Hydroxyls can be converted into esters *via* treatment with an esterifying agent such as alkanoyl halides. The term also includes lower hydrocarbon groups capable of being solvated under physiological conditions, *e.g.*, alkyl esters, methyl, ethyl and propyl esters. (See, for example, Berge et al., *supra.*)

The invention also pertains, at least in part, to packaged compositions comprising the tetracycline compounds of the invention and instructions for using said compounds for the treatment of diseases which are treatable by the administration of a tetracycline compound having a target therapeutic activity.

The invention is further illustrated by the following examples, which should not be construed as further limiting. The contents of all references, pending patent applications and published patents, cited throughout this application are hereby expressly incorporated by reference. Art recognized animal models or *in vitro* assays for the inflammatory process associated states described herein are used to determine the efficacy of a particular tetracycline compound for a target disease such as an IPAS, neurological disorder, or cancer.

### Exemplification of the Invention:

### Example 1: Synthesis of Tetracycline Compounds

The following example discusses methods of synthesizing the tetracycline compounds of the invention. Other compounds of the invention can be synthesized using techniques discussed in the application and/or by using art recognized methods.

### Experimental

Melting points were taken on a Mel-Temp capillary melting point apparatus and are uncorrected. Nuclear magnetic resonance (¹H NMR) spectra were recorded at 300 MHz on a Bruker Avance spectrometer. The chemical shift values are expressed in δ values (ppm) relative to tetramethylsilane or 3-(trimethylsilyl)-1-propanesulfonic acid, sodium salt, as either an internal or external standard using CDCl₃, DMSO-*d*₆, or MeOH-*d*₄ as the solvent. Column chromatography was performed according to the method of Still using Baker "flash" grade silica gel (40 µm) that was treated with a saturated solution of Na₂EDTA, washed with water, filtered and dried in an oven at 130°C for three hours prior to use. Analytical TLC separations employed the use of 0.25 mm silica gel plates with florescence indicator obtained from J.T. Baker Chemical Co., Phillipsburg, NJ, that were pretreated by immersion into a saturated solution of Na₂EDTA for five minutes and reactivated at 130 °C for three hours. Solvent systems used were as follows: 50:50:5 CHCl₃/MeOH/5% Na₂EDTA (lower phase) (I), 65:20:5, CHCl₃/MeOH/Na₂EDTA (lower phase) (II). Visualization of TLC was accomplished by 0.5% aqueous Fast Blue BB salt and heating at 130 °C for 5 minutes. Analytical HPLC was performed on a Waters Bondapak C18 reverse phase column by using two Varian SD 100 HPLC pumps at a 1.6 mL/min flow rate controlled by software. Detection was by UV absorption with Model 441 absorbance detector operating at 280 nm. Mobile phases used followed a linear gradient from 30% to 100% methanol over 30 minutes at 1.6 mL/min flow rate followed by isocratic elution with MeOH; solvent system A: 0.02 M Na₂HPO₄ +0.001 M Na₂EDTA adjusted to pH 4.5 with H₃PO₃; solvent system B: 100% MeOH. Semipreparative HPLC separations used a Waters semipreparative C18 reverse-phase column at a flow rate of 6.4 mL/min. Low and high resolution mass spectra were performed on a PE Mariner spectrometer (Nelson et al., J. Med. Chem. (1993) 36(3):374).

### 7-Iodo-Sancycline

One gram of sancycline was dissolved in 25 mL of TFA (trifluoroacetic acid) that was cooled to 0 C (on ice). 1.2 equivalents of N-iodosuccinimide (NIS) was added to the reaction mixture and reacted for forty minutes. The reaction was removed from the ice bath and was allowed to react at room temperature for an additional five hours. The mixture was then analyzed by HPLC and TLC, was driven to completion by the stepwise addition of NIS. After completion of the reaction, the TFA was removed *in vacuo* and 3 mL of MeOH was added to dissolve the residue. The methanolic solution was the added slowly to a rapidly stirring solution of diethyl ether to form a greenish brown precipitate. The 7-iodo isomer of sancycline was purified by treating the 7-iodo product with activated charcoal, filtering through Celite, and subsequent removal of the solvent *in vacuo* to produce the 7-isomer compound as a pure yellow solid in 75% yield.
MS(M+H) (formic acid solvent) 541.3.
\Rt: Hypersil C18 BDS Column, 11.73
¹H NMR (Methanol d₄-300 MHz) δ 7.87-7.90 ( d, 1H), 6.66-6.69 (d, 1H), 4.06 (s, 1H), 2.98 (s, 6H), 2.42 (m, 1H), 2.19 (m, 1H), 1.62 (m, 4H), 0.99 (m, 2H)

### 13-(4'-Trifluoromethylphenyl) Methacycline

Methacycline (1.0 mmol), PdCl₂ (.14 mmol), and CuCl₂ (.90 mmol) were dissolved in 20 ml of MeOH and heated under nitrogen atmosphere. After 1 hour, the 4-trifluoromethylphenyl boronic acid (2.0 mmol) was added to it and the reaction mixture was heated for another 6-10 hours. The reactions was monitored by TLC, and analytical HPLC. The reaction mixture was then cooled down to the room temperature and was passed through a bed of Celite. Evaporation of the solvent gave a yellow-brown solid, which was purified using preparative HPLC (CH₃CN:MeOH:H₂O). Evaporation of the solvent from the fractions indicated the right peak for the expected product, gave a yellow solid, which was again dissolved in MeOH and purged with HCl gas. After evaporation of MeOH, the yellow material was dried under vacuum for several hours.

### 7-(3',4'-Dimethoxy-Phenyl) Sancycline

7-iodosancycline (0.28 mM), Pd(OAc)₂ and 1 0 mL of MeOH are added to a flask with a stir bar and the system degassed 3x using argon. Na₂CO₃ (0.8 mM) dissolved in water and argon degassed is added via syringe is added along with 2,5-dimethoxy phenylboronic acid (0.55 mM) in MeOH that was also degassed. The reaction was followed by HPLC for 2 hours and cooled to room temperature. The solution was filtered, and dried to produce a crude mixture. The solid was dissolved in dimethylformamide and injected onto a preparative HPLC system using C18 reverse-phase silica. The solvent was removed in vacuo to yield the product plus salts. The salts were removed by extraction into 50:25:25 water, butanol, ethyl acetate and dried *in vacuo.* This solid was dissolved in MeOH and the HCl salt made by bubbling in HCl gas.

### 7-(3'-aminophenyl) Sancycline

To a solution of 200 mg of 7-(3-nitrophenyl) sancycline in 50 mL methanol, 10 mg of 10% palladium on charcoal catalyst was added. The reaction mixture was shaken under 40 psi hydrogen pressure for 2 hours and was then filtered followed by concentration. The residue was further purified by preparative HPLC. 35 mg was isolated as the HCl salt and the structure was proved by NMR and LC-MS to be 7-(3-aminophenyl) sancycline.

### 1,8-Di-7-Sancyclinyl-1,8-Heptyne (Compound FM)

A flask was charged with 7-iodosancycline (3.0 g, 4.57 mmol,), Pd(OAc)₂ (0.102 g, 0.46 mmol), CuI (0.044 g, 0.23 mmol), and P(*o*-Tol)₃ (0.278 g, 0.91 mmol) and the contents were suspended in anhydrous acetonitrile. After purging this mixture with dinitrogen at 60 °C (bath temperature), 1,7-octadiyne (0.305 mL, 2.29 mmol) was added to it, followed by the addition of triethylamine. The dark colored solution was stirred at 60 °C for 3h, filtered through a bed of Celite and dried. A methanol: DMF: TFA (90:8:2) solution of the product (9C) was purified on preparative HPLC column. The compound was identified by HPLC, MS, and ¹H NMR spectroscopy.

### 7-(2', 4'-Difluorophenyl) Sancycline

7-iodosancycline, (0.3 mM), Pd(OAc)₂, and 10 mL of MeOH was added to a flask with a stir bar and the system degassed 3x using argon. Na₂CO₃ (1.1 mM) dissolved in water and argon degassed was added via syringe is added along with 2,4-difluoro-phenylboronic acid (0.7 mM) in MeOH that was also degassed. The reaction was followed by HPLC for 20 minutes and cooled to room temperature. The solution was filtered, and dried to produce a crude mixture. The solid was dissolved in dimethylformamide and injected onto a preparative HPLC system using C18 reverse-phase silica. The solvent was removed *in vacuo* to yield the product plus salts. The salts were removed by extraction into 50:25:25 water, butanol, ethyl acetate and dried *in vacuo.* This solid was dissolved in MeOH and the HCl salt made by bubbling in HCl gas. The solvent was removed to produce the product.

### 9-Cyclohexenylethynyl-Minocycline

To a solution of 9-iodo-minocycline (1.13 mmol), 50 mg tetrakis-triphenylphosphino-palladate, 50 mg copper(I) iodide, 10 mg palladium acetate and 3 ml triethylamine, 0.1 ml cyclohexenyl-acetylene was added. The reaction mixture was stirred at 60 °C for one hour, filtered through a Celite bed and concentrated. The dry material was dissolved in methanol and filtered. The solution was then concentrated and purified using preparative liquid chromatography. The preparative liquid chromatography used a C₁₈ stationary phase with eluent A: 0.1 % TFA in water and eluent B: 0.1% TFA in acetonitrile. The compound was identified by standard techniques.

### 7-(Propynyl)-Sancycline

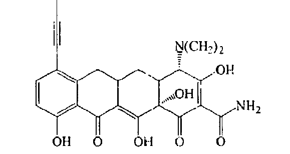

7-I-Sancycline (1 gm, 1.86 mmol), taken in 25 mL of acetonitrile was degassed and purged with nitrogen (three times). To this suspension Pd(OAc)₂ (20 mg, .089 mmol), CuI (10 mg, .053 mmol), (o-tolyl)₃P (56 mg, 0.183 mmol) were added and purged with nitrogen for few minutes. Propyne (3.72 mmol) and triethylamine (1 mL) were added to the suspension. It was turned into a brown solution upon addition of Et₃N. The reaction mixture was then heated to 70 °C for 3 hours. Progress of the reaction was monitored by HPLC. It was then cooled down to room temperature and was filtered through Celite. Evaporation of the solvent gave a brown solid, which was then purified on preparative HPLC to give a yellow solid. The structure of this compound has been characterized using 1H NMR, HPLC, and MS.

### 7-(2-Methylphenylethyl)-Sancycline

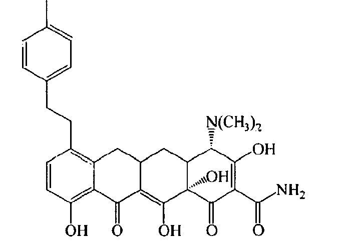

7-(2-Methylphenylethynyl)-sancycline (1mmol) was taken in saturated solution of MeOH/HCl. To this solution 10% Pd/C was added and was subjected to hydrogenation at 50 psi for 12 hrs. It was then filtered through Celite. The solvent was evaporated to give a yellow powder. Finally, it was precipitated from MeOH/diethylether. The structure of this compound has been characterized using 1H NMR, HPLC, and MS.

### 9-(4'-Acetyl phenyl) Minocycline

In a clean, dry reaction vessel, was placed 9-iodominocycline (0.762mmoles) bis HCl salt, palladium (II) acetate (0.076mmoles) along with 10ml of reagent grade methanol. The solution was immediately purged, with stirring, with a stream of argon gas for approximately 5 minutes. The reaction vessel was brought to reflux and to it was sequentially added via syringe 2M potassium carbonate solution, followed by a solution of p-acetylphenyl boronic acid (1.53mmoles) in 5ml of reagent DMF. Both of these solutions were previously degassed with argon gas for approximately 5 minutes. The reaction was heated for 45 minutes, the progress was monitored via reverse phase HPLC. The reaction was suctioned filtered through a pad of diatomaceous earth and the pad was washed with DMF. The filtrates were reduced to an oil under vacuum and residue treated with t-butylmethyl ether. Crude material was purified via reverse phase HPLC on DVB utilizing a gradient of water and methanol/acetonitrile containing 1.0% trifluoroacetic acid.

### 7-(n-Propyl)-Sancycline

7-propynyl sancycline was dissolved in a saturated methanol hydrochloric acid solvent. The mixture was placed in a hydrogenator under 50 psi hydrogen pressure. The reaction was completed in ∼8 hours. The catalyst was filtered off, and the resulting solution was concentrated. The crude product was purified by preparative liquid chromatography using a C₁₈ stationary phase with eluent A: 0.1% TFA in water and eluent B: 0.1% TFA in acetonitrile. The combined clean fractions are concentrated and hydrochloric acid saturated isopropanol added. The pure product is precipitated by addition of diethylether and filtered off.

### N-Benzyl-9'-minocyclinyl guanidine

To a stirred solution of 9-aminominocycline (1.6 mmol) in 30 mL of acetonitrile, benzylcyanimide (6.0 mmol) was added in one portion. The reaction mixture was first heated to refluxed at 60 °C for several hours, and continued at room temperature for 4-5 days. The guanidino product was subsequently isolated, and identified using MS, NMR and HPLC.

### 7-(para-tert-butyl phenyl)-9-aminomethyl sancycline

7-para-tert-butyl phenyl sancycline (5.0 g) was dissolved in trifluoroacetic acid (300 mL). Three equivalents of HMBC was added and the reaction was stirred at room temperature. After 72 hours, HPLC indicated that the reaction was complete. The reaction mixture was filtered to give a brown liquid which was subsequently dissolved in methanol and precipitated in diethyl ether. The solid was then purified using HPLC and the product was identified using NMR and mass spectra.

### 7-Furanyl Sancycline

7-iodo sancycline (1.3 mg) and Pd(OAc)₂ were taken in 100 mL of methanol and purged with argon for five minutes at 70 °C. To this solution was added a solution of sodium carbonate (44 mg) in water (previously purged with argon). A yellow precipitate was obtained and the mixture was heated for another ten minutes. 3-Furanyl boronic acid (333 mg, solution in DMF, purged with argon) was then added and the mixture was heated for another two hours at 70 °C. The reaction was monitored by HPLC/MS. When the reaction was complete, the mixture was filtered through Celite and the solvent was removed to give a crude material. The crude material was purified by precipitating it with ether (200 ml). The yellow precipitate was filtered and purified using preparative HPLC. The hydrochloride salt was made by dissolving the material in MeOH/HCl and evaporating to dryness. The identity of the resulting solid was confirmed using HPLC, MS, and NMR.

### 9-(2'phenyl ethyl amino methyl)-Doxycycline

Under a N₂ atmosphere, a stirred solution of 9-aminomethyldoxycycline dihydrochloride (1.21 g, 2.21 mmol) in DMF (10 mL) was treated with InCl₃ (0.076 g, 0.34 mmol) and phenylacetaldehyde (0.511 mL, 4.4 mmol). HPLC and LC-MS monitoring of the reaction indicated the complete consumption of the starting material over the course of twelve hours; the products being both mono- (major) and bis- (minor) substituted aminomethyldoxycycline. Methanol (10 mL) was added to quench this reaction. The reaction mixture was filtered through a bed of Celite. The Celite bed was subsequently washed with 5 mL of methanol twice. The combined organic washes were concentrated to about 7-8 mL and diluted with ether. The resulting amorphous solid was filtered, washed with ether (6 x 15 mL) and dried under vacuum to afford a red powder, which was purified by preparative HPLC. The final product, Compound RR, was characterized by HPLC, MS, and ¹H NMR spectroscopic methods. MS(*m*/*z*): Theor. 577.24; Found: 578.17 (M+1).

### 7-Ethyl-9-(Iso-butyl amino) Sancycline

7-ethyl-9-amino sancycline (390 mg) was dissolved in 10 mL of DMF. Triethylamine (237 µL), isobutyraldehyde (77 µL), and InCl₃ (19 mg) were then added and the reaction mixture was stirred for several minutes at room temperature. Then, NaBH(OAc)₃ (360 mg) was added and the reaction was continued at room temperature. LC-MS showed that the reaction was completed after two hours. The reaction was quenched with methanol and dried. The resulting solid was redissolved in methanol and purified. The product was then converted to the HCl salt. The identity of the product was confirmed using NMR, HPLC, and MS.

### 7-Furanyl-9-nitro-Sancycline

500 milligrams of 9-NO₂ sancycline was taken in 20 mL of TFA and cooled down in an ice bath. To this solution, NIS (300 mg) was added in portions and stirred at room temperature for three hours. Once the reaction was completed, 7-iodo-9-NO₂ sancycline was precipitated in diethyl ether. The yellow powder was then filtered and dried *in vacuo.*

7-Iodo-9-nitro-sancycline (585 mg) and Pd(OAc)₂ (22 mg) were taken in 20 mL of methanol and purged with argon for five minutes. To this solution, Na₂CO₃ (420 mg, solution in 5 mL H₂O, purged with argon), was added and a yellow precipitate was obtained. The solution was stirred at 55-60 °C for five minutes. To this solution, 3-furanyl boronic acid (160 mg in 5 mL of DMF, purged with argon) was added and the reaction mixture was heated at 70 °C for three hours. The reaction mixture was then passed through Celite. Evaporation of the solvent gave a brown solid, which was then recrystallized using a mixture of methanol and ether to yield 7-furanyl 9-nitro sancycline.

7-Furanyl 9-nitro sancycline (500 mg) was taken in 30 ml of methanol. To this solution, PtO₂ (15 mg) was added and hydrogenated at 40 psi for three hours. It was then filtered through Celite. The crude material was purified using preparative HPLC to yield 7-furanyl 9-amino sancycline.

### 9-Minocycline methyl ester

In the Parr apparatus were placed: 9-iodosancycline trifluoroacetic acid salt (0.8 g, 1.17 mmol), NaOAc (0.64g, 4 eq.), Pd(dppf)₂Cl₂, and CH₂Cl₂ (48mg, 5%). The apparatus was closed, purged with CO, and then filled with CO under 450psi. The reaction mixture was stirred for four hours at 80 °C. It was then acidified with TFA and concentrated *in vacuo.* The product was purified by HPLC. A mixture of 3 : 1 epimers was obtained. The yield was 188 mg of product.

### 7-Cyano Sancycline

7-iodo sancycline (1.3 g) was dissolved in NMP (15 mL) and CuCN (344 mg) was added. The reaction mixture was stirred at 80 °C for 15/16 hours overnight. The reaction mixture was diluted with methanol and centrifuged to yield a grey white precipitate. The reaction mixture was then passed through Celite and washed with additional methanol. The filtrate was then concentrated and precipitated with ether. The solid obtained was then purified using preparative HPLC to yield 7-cyano sancycline in a 50/50 mixture of epimers. The structure of the product was confirmed using mass spectra and NMR.

### 9-(N-piperdinyl)-minocycline

Concentrated H₂SO₄ (2 mL) was added slowly to a stirred solution of gluteraldehyde (1 mL). Water (0.8 g) was added and stirred at room temperature for eighteen hours and heater to 70 °C for two hours. The mixture was then cooled to room temperature. The solution was then transferred to a solution of 9-amino minocycline in DMF (5 ml) and stirred at room temperature for two days until all starting material was consumed, as indicated by HPLC. The product was isolated and purified using standard techniques. The structure of the product was confirmed by NMR and mass spec.

### 2-[4-(5-Minocyclin-9-yl-furan-2-ylmethyl)-piperazin-1-yl]-ethanol

Na₂CO₃ (0.64 g) in water (5 mL) was added to a degassed solution of 9-iodominocycline hydrochloride (1 g) and Pd(OAc)₂ (100 mg) in methanol (10mL). The reaction was stirred for five minutes at 60 °C. 2-Formyl furan-5-boronic acid (0.3 g) in methanol (10 mL) was then added, and the reaction was allowed to proceed for four hours. The mixture was then filtered and concentrated to give a brown solid (9-(2'formyl furanyl)-minocycline).

The brown solid (9-(2'formyl furanyl)-minocycline, 1 g) was dissolved in 20 mL of methanol and acetic acid (2 mL) and hydroxyethyl piperazine (1 mL) was added and stirred for ten minutes at room temperature. The reaction was quenched with ether (200 mL), and the organic layer was then washed and concentrated to yield a brown oil. The brown oil was the dissolved in methanol (10 mL) and water. The mixture was the chromatographed using a CH₃CN gradient to yield the product, 2-[4-(9-Minocyclin-2-yl-furan-2-ylmethyl)-piperazin-1-yl]-ethanol. The product was confirmed using MS, NMR, and HPLC.

### 9-N-morpholinyl minocycline

NaCNBH₃ (200 mg) was added to a stirred solution of 9-amino minocycline H₂SO₄ (1 g) in methanol (4.9 mL) and acetic acid 91 mL) and stirred for five minutes at room temperature. (2-Oxo-ethoxy)-acetaldehyde (10 mL) was added dropwise and stirred for fifteen minutes at room temperature. The reaction mixture was concentrated with out heat and the residue was dissolved in 20 mL of methanol and TFA (0.5 mL). The product was obtained using preparative HPLC and converted to the HCl salt. The product was confirmed using mass spectra and NMR.

### N-Benzyl-N',N'-dimethyl-N-(5-minocyclin-9-yl-furan-2-ylmethyl)-ethane-1,2-diamine

Na₂CO₃ (0.64 g) in water (5 mL) was added to a degassed solution of 9-iodo-minocycline hydrochloride (1 g) and Pd(OAc)₂ (100 mg) in methanol (10mL). The reaction was stirred for five minutes at 60 °C. 2-Formyl furan-5-boronic acid (0.3 g) in methanol (10 mL) was then added, and the reaction was allowed to proceed for four hours. The mixture was then filtered and concentrated to give a brown solid (9-(2'formyl furanyl)-minocycline).

The brown solid (9-(2'formyl furanyl)-minocycline, 1 g) was dissolved in 20 mL of methanol and acetic acid (2 mL) and N'-benzyl-N,N-dimethyl ethylenediamine (1 mL) was added and stirred for ten minutes at room temperature. The reaction was quenched with ether (200 mL), and the organic layer was then washed and concentrated to yield a brown oil. The brown oil was the dissolved in methanol (10 mL) and water. The mixture was the chromatographed using a CH₃CN gradient to yield the product, N-Benzyl-N',N'-dimethyl-N-(5-minocyclin-9-yl-furan-2-ylmethyl)-ethane-1,2-diamine. The product was confirmed using MS, NMR, and HPLC.

### 3-Benzyloxysancycline

60% NaH in a mineral oil dispersion (100 mg, 2.5 mmol) was added in small portions to a stirred solution of sancycline (0.5 g, 1.20 mmol) in DMF (5 mL) at room temperature. The resulting suspension was stirred at room temperature for 5 minutes. Benzyl bromide (0.143 mL, 1.2 mmol) was added and heated at 60 °C for 16 hours. The reaction mixture was then cooled to room temperature and quenched with ether (100 mL). The ether was decanted and the remaining solid was dissolved in MeOH/water. The product was purified by preparative HPLC and converted to the HCl salt, yielding 3-benzyloxysancycline as a light yellow solid.

### 3, 10 -Dibenzyloxysancycline

60% NaH in a mineral oil dispersion (192 mg, 4.8 mmol) was added in small portions to a stirred solution of sancycline (0.5 g, 1.20 mmol) in DMF (5 mL) at room temperature. The resulting suspension was stirred at room temperature for 5 minutes. Benzyl bromide (0.43 mL, 3.6 mmol) was added and the reaction mixture was heated at 60 °C for 1 hour. The reaction mixture was subsequently cooled to room temperature and quenched with ether (100 mL). The ether was the removed by decanting and the remaining solid was dissolved in MeOH/water. The product was purified by preparative HPLC and converted to the HCl salt to yield 3, 10-dibenzyloxysancycline as a light yellow solid.

### 10-Butyloxyminocycline

60% NaH in a mineral oil dispersion (152 mg, 3.8 mmol) was added in small portions to a stirred solution of minocycline HCl salt (0.5 g, 0.95 mmol) in DMF (5 mL) at room temperature. The resulting suspension was stirred at room temperature for 5 minutes. Iodobutane (0.325 mL, 2.85 mmol) was added and heated at 60 °C for 1 hour. The reaction mixture was cooled to room temperature and quenched with ether (100 mL). The ether was subsequently decanted and the remaining solid was dissolved in MeOH/water. The product was purified by preparative HPLC and converted to the HCl salt to give 10-butyloxyminocycline as an olive green solid.

### 3-Benzyloxy-7-iodosancycline

60% NaH (121 mg, 3.04 mmol) was added in small portions to a stirred solution of 7-iodosancycline TFA salt (0.5 g, 0.76 mmol) in DMF (10 mL) at room temperature. The resulting suspension was stirred at room temperature for 5 minutes. Benzyl bromide ( 0.277 mL, 2.28 mmol) was added and heated at 60 °C for 30 minutes. The reaction mixture was then cooled to room temperature and quenched with ether (100 mL). The ether was decanted and the remaining solid was dissolved in MeOH. The product was purified by preparative HPLC and converted to the HCl salt to give 3-benzyloxy-7-iodosancycline as a yellow solid.

### 3-Benzyloxy-7-(3'-trifluoromethylphenyl)sancycline

A solution of sodium carbonate (670 mg, 6.32 mmol) in water (5 mL) was added to a stirred suspension of 7-iodo-3-benzyloxysancycline (1.00 g, 1.58 mmol) and Pd(OAc)₂ (100 mg, 0.44 mmol) in methanol (10 mL) at 60 °C under nitrogen. The resulting suspension was stirred at 60 °C for 10 min. 4-Trifluoromethylphenyl boronic acid ( 0.6 g, 3.16 mmol) in methanol (10 mL) was then added and the reaction mixture was heated at 60 °C for 3 hours under nitrogen. The warm reaction mixture was filtered and concentrated. The crude product was purified by preparative HPLC and converted to the HCl salt to give 3-benzyloxy-7-(3'-trifluoromethylphenyl)sancycline as a pale brown solid.

### Example 2: Mammalian Cytotoxicity Assay

COS-1 and CHO-K1 cell suspensions were prepared, seeded into 96-well tissue culture treated black-walled microtiter plates (density determined by cell line), and incubated overnight at 37°C, in 5% CO₂ and approximately 95% humidity. The following day, serial dilutions of drug were prepared under sterile conditions and transferred to cell plates. Cell/Drug plates were incubated under the above conditions for 24 hours. Following the incubation period, media/drug was aspirated and 50 µl of Resazurin (0.042 mg/ml in PBS w/Ca and Mg) was added. The plates were then incubated under the above conditions for 2 hours and then in the dark at room temperature for an additional 30 minutes. Fluorescence measurements were taken (excitation 535 nm, emission 590 nm). The IC₅₀ (concentration of drug causing 50% growth inhibition) was then calculated. The cytotoxicity of both unsubstituted minocycline and doxycycline were found to be greater than 25. Each of the compounds shown in Table 3 were found to have acceptable cytotoxicities.

### Example 3: In vitro Anti-Bacterial Activity Assay

The following assay was used to determine the efficacy of the tetracycline compounds against gram positive (*S*. *aureus* RN450) and gram negative (*E*. *coli* ML308 225) bacteria. 2 mg of each compound was dissolved in 100 µl of DMSO. The solution was then added to cation-adjusted Mueller Hinton broth (CAMHB), which resulted in a final compound concentration of 200 µg per ml. The tetracycline compound solutions were diluted to 50 µL volumes, with a test compound concentration of .098 µg/ml. Optical density (OD) determinations were made from fresh log-phase broth cultures of the test strains. Dilutions were made to achieve a final cell density of 1x10⁶ CFU/ml. At OD=1, cell densities for different genera were approximately:

| | |
|---|---|
| *E*. *coli* | 1x10⁹ CFU/ml |
| *S. aureus* | 5x10⁸ CFU/ml |

50 µl of the cell suspensions were added to each well of microtiter plates. The final cell density was approximately 5x10⁵ CFU/ml. These plates were incubated at 35°C in an ambient air incubator for approximately 18 hours. The plates were read with a microplate reader and were visually inspected when necessary. The MIC was defined as the lowest concentration of the tetracycline compound that inhibits growth. For illustrative purposes and not to be construed as limiting, in Table 3, compounds with MIC of greater than 4 µg/ml are indicated with *, and compounds with MIC less than or equal to 4 µg/ml are indicated with **.

### Example 4: In vitro Anti-Inflammatory Assay: Lipopolysaccharide Stimulation of Macrophage Assay (LSM Assay)

This assay was used to determine the anti-inflammatory effect of tetracycline compounds of the invention by determining the modulation of nitric oxide, interleukin-10 and interleukin-12 synthesis in the J774 cell line, according to a literature procedure (D'Agostino P. et al. Int Immunopharmacol. 2001 Sep;1(9-10):1765-76). J774.2 cells were stimulated with 100 ng/ml lipopolysaccharide (LPS). Nitrite, the spontaneous degradation product of nitric oxide, is measured in cell supernatants using the Greiss Reaction. In the experimental conditions, test tetracycline compounds were added 30 minutes prior to LPS stimulation. Cytotoxicity is determined using Resazurin metabolism. On the first day, a 96-well black-walled plate (except for the bottom row) was seeded with 100 µl of a 2.5 x 10⁶ cells /ml suspension and incubated for two hours at 37 °C and 5% CO₂. Towards the end of the two hour incubation period, test compounds were prepared at a concentration of 139 µg/ml, in 1.25% DMSO, a 2.5x concentration ready for addition to the cells.

At the end of the two hour incubation period, 80 µl of each of the test tetracycline compound solutions were added to the cell plates to make a final concentration of 56 µg/ml. Next, 80 µl of 1.25% DMSO in media was added to the negative control wells. Then, 80 µl of media was added to the positive control wells. The plate was then incubated for half an hour at 37°C, 5% CO₂. A 10X working solution of LPS was prepared at a concentration of 1 µg/ml LPS.

After the half hour incubation period, 20 µl of LPS (1 µg/ml) in media was added to half of the test and control wells to give a final concentration of 100 ng/ml. Next, 20 µl of media was added to other half of the wells. The plate was then incubated for 24 hours at 37°C, 5% CO₂. 100 µl of the supernatant from each well was collected for nitrite testing. 60 µl of the supernatant was collected for cytokine testing using an enzyme linked immunosorbant assay. The plates are then stored after being covered with sealer and frozen.

For the testing toxicity, the remaining media was blotted from the cell plates and 50 µl of Resazurin was added to each well (0.042 mg/ml in PBS w/Ca and Mg). The plate were then incubated for 45 mins, 37°C, 5% CO₂, and for 30 minutes at room temperature. The plate was then read for Resazurin fluorescence.

50 µl/well of sulfanilamide solution (1% sulfanilamide in 5% H₂PO₄) was added to supernatant plates. The plates were then incubated for 10 minutes at room temperature in the dark. Next, 50 µl/well NED solution (0.1 % N-1-naphthylethylene diamine dihydrochloride in water) was added and incubated for 10 minutes at room temperature in the dark. Plates are then read for nitrite measurement and compared to a standard curve generated from the control wells.

The data from the inhibition of nitric oxide assay are shown in Table 3. In Table 3, compounds with very good nitric oxide synthesis inhibition are indicated with ***, those with good inhibition are indicated with **, those with satisfactory inhibition of nitric oxide synthesis are indicated with *, and those with some inhibition at concentrations higher than 56 µg/ml are indicated with "o."

**TABLE 3**

| **ID** | **STRUCTURE** | Nitrite | AntibioticActivity Gram - | Antibiotic Activity Gram + |
|---|---|---|---|---|
| A | 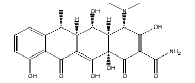 | ** | ** | ** |
| B | 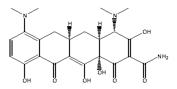 | ** | ** | ** |
| C | 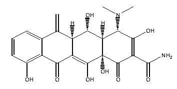 | ** | * | * |
| D | 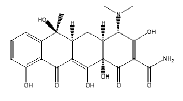 | * | ** | ** |
| E | 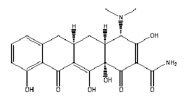 | * | ** | ** |
| F | 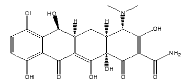 | *** | NT | NT |
| G | 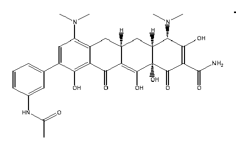 | o | * | * |
| H | 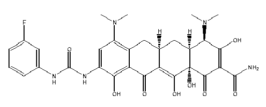 | ** | * | ** |
| I | 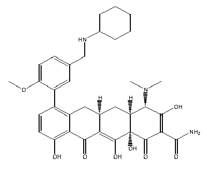 | * | ** | ** |
| J | 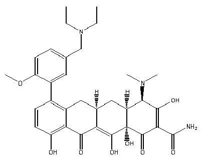 | * | ** | ** |
| K | 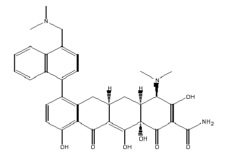 | *** | ** | ** |
| L | 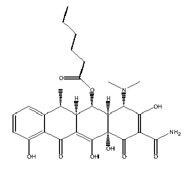 | * | * | ** |
| M | 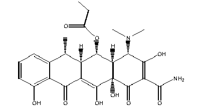 | ** | * | ** |
| N | 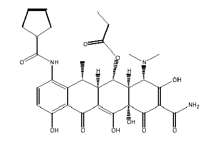 | * | NT | NT |
| O | 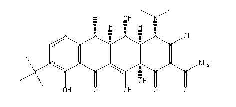 | *** | * | ** |
| P | 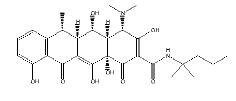 | * | NT | ** |
| Q | 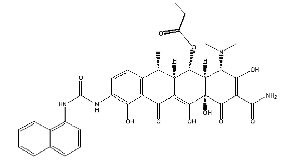 | o | * | ** |
| R | 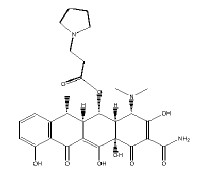 | ** | ** | ** |
| S | 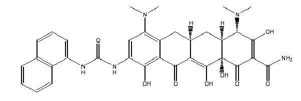 | ** | * | ** |
| T | 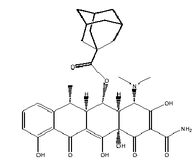 | ** | * | ** |
| V | 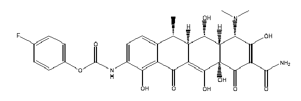 | * | * | * |
| W | 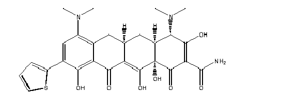 | *** | * | ** |
| X | 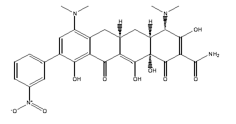 | *** | * | ** |
| Y | 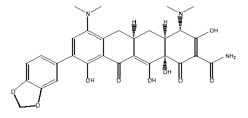 | ** | * | ** |
| Z | 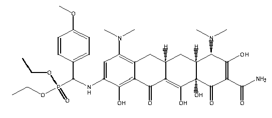 | * | NT | ** |
| AA | 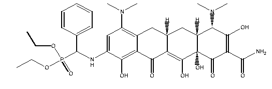 | * | * | * |
| AB | 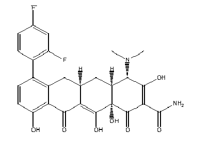 | *** | * | ** |
| AC | 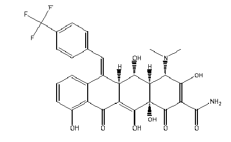 | *** | * | ** |
| AD | 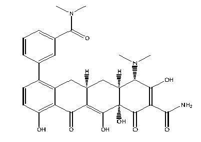 | * | * | ** |
| AE | 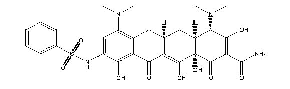 | * | ** | ** |
| AF | 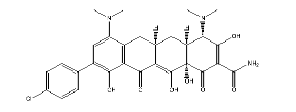 | ** | * | ** |
| AG | 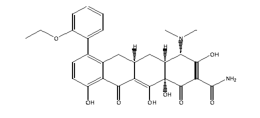 | ** | * | ** |
| AH | 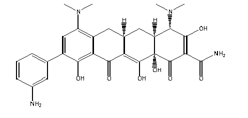 | ** | * | ** |
| AI | 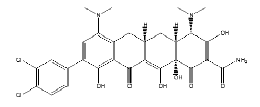 | *** | * | ** |
| AJ | 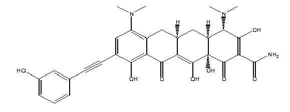 | ** | * | ** |
| AL | 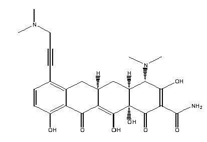 | *** | ** | ** |
| AM | 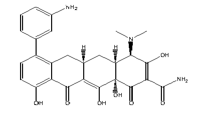 | *** | ** | ** |
| AN | 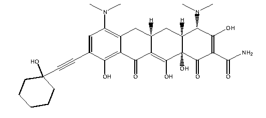 | * | * | * |
| AO | 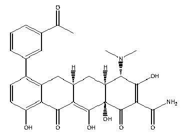 | ** | * | ** |
| AP | 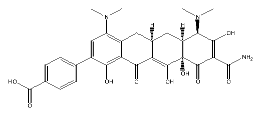 | * | * | * |
| AQ | 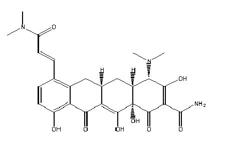 | ** | ** | ** |
| AR | 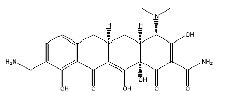 | ** | ** | ** |
| AS | 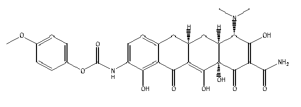 | ** | * | ** |
| AU | 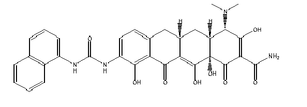 | * | * | ** |
| AV | 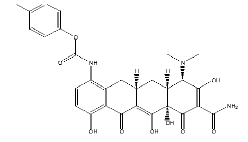 | * | ** | ** |
| AW | 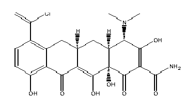 | ** | ** | ** |
| AX | 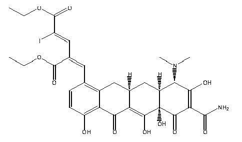 | *** | * | * |
| AY | 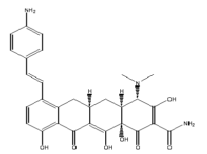 | *** | * | ** |
| BA | 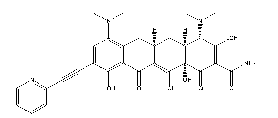 | ** | * | * |
| BB | 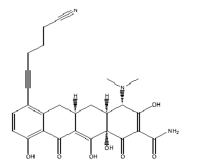 | * | ** | ** |
| BC | 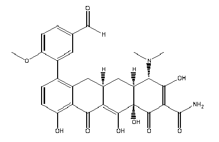 | * | ** | ** |
| BD | 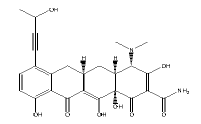 | * | ** | ** |
| BE | 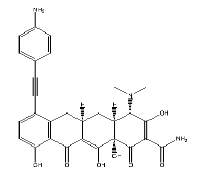 | *** | * | ** |
| BF | 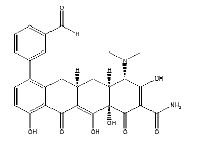 | * | ** | ** |
| BH | 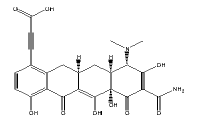 | * | * | * |
| BI | 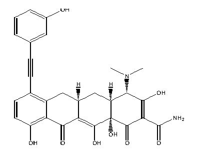 | *** | * | ** |
| BJ | 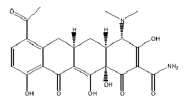 | ** | ** | ** |
| BK | 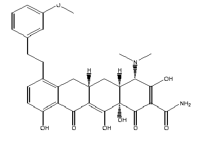 | *** | * | ** |
| BL | 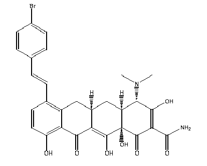 | *** | * | ** |
| BM | 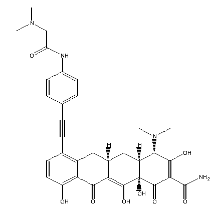 | *** | ** | ** |
| BN | 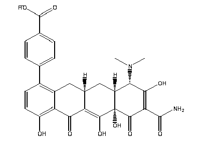 | * | * | ** |
| BO | 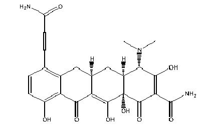 | *** | * | * |
| BP | 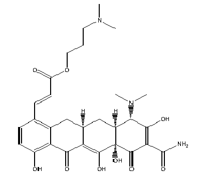 | *** | ** | ** |
| BQ | 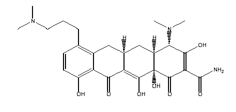 | * | * | ** |
| BS | 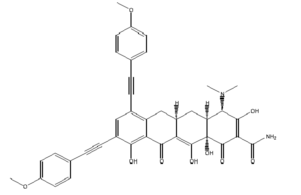 | * | * | * |
| BT | 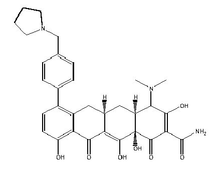 | *** | ** | ** |
| BU | 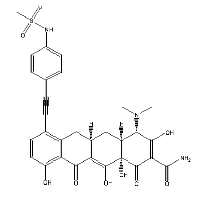 | * | * | ** |
| BV | 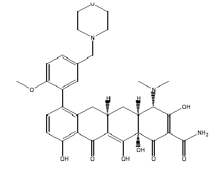 | *** | * | ** |
| BW | 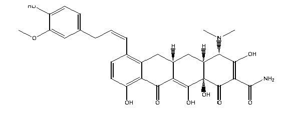 | * | * | ** |
| BY | 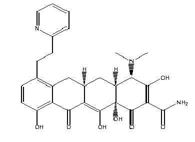 | ** | * | ** |
| CA | 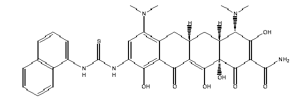 | * | * | ** |
| CB | 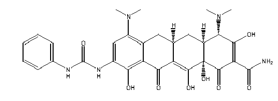 | * | * | ** |
| CD | 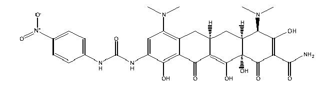 | * | * | ** |
| CE | 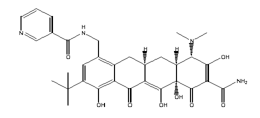 | * | * | * |
| CG | 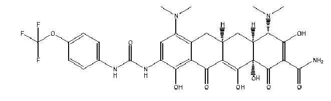 | ** | * | ** |
| CH | 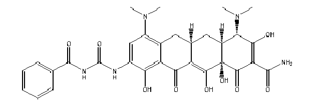 | * | * | ** |
| CI | 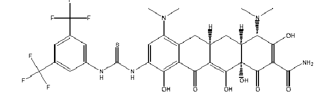 | ** | * | ** |
| CJ | 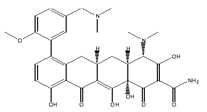 | *** | ** | ** |
| CM | 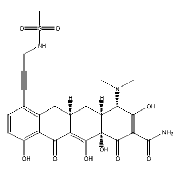 | ** | * | * |
| CN | 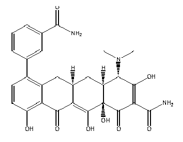 | * | ** | ** |
| CO | 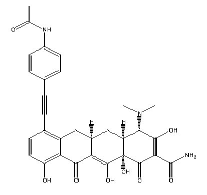 | *** | * | ** |
| CQ | 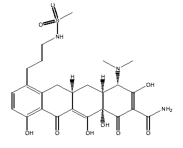 | * | * | ** |
| CR | 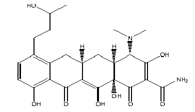 | *** | * | ** |
| CS | 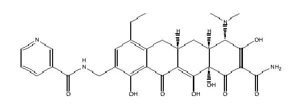 | * | * | * |
| CT | 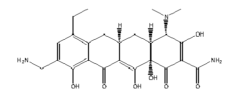 | * | ** | * |
| CU | 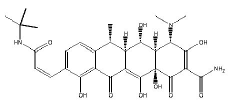 | * | * | * |
| CV | 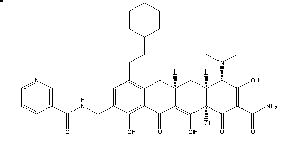 | *** | * | ** |
| CW | 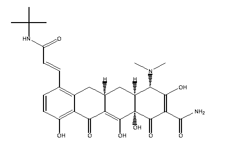 | *** | * | ** |
| CX | 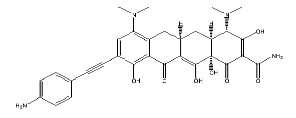 | *** | * | * |
| CY | 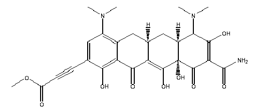 | *** | * | ** |
| CZ | 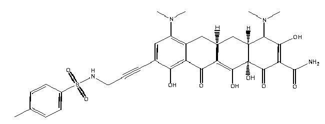 | * | * | * |
| DA | 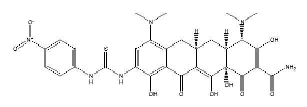 | * | * | ** |
| DB | 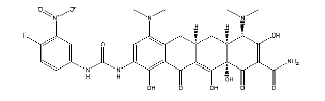 | * | * | ** |
| DC | 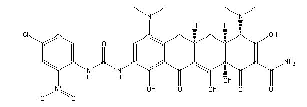 | ** | * | ** |
| DD | 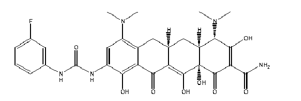 | * | * | ** |
| DE | 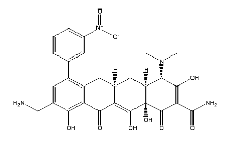 | *** | * | ** |
| DF | 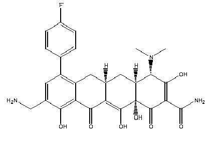 | *** | * | ** |
| DG | 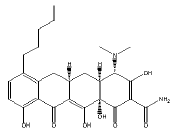 | *** | * | ** |
| DI | 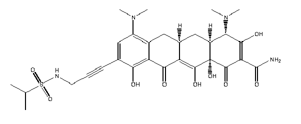 | * | * | * |
| DK | 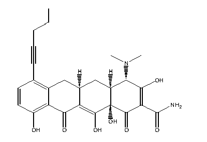 | *** | * | * |
| DL | 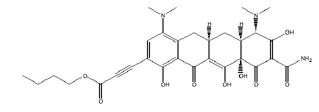 | *** | * | * |
| DM | 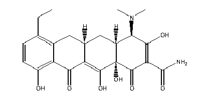 | *** | * | ** |
| DN | 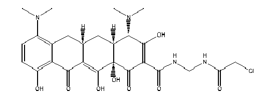 | ** | * | ** |
| DP | 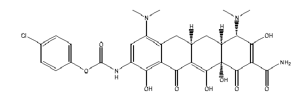 | * | * | * |
| DQ | 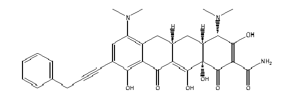 | * | * | ** |
| DR | 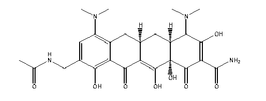 | * | * | ** |
| DS | 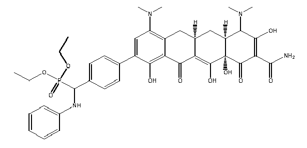 | * | * | ** |
| DT | 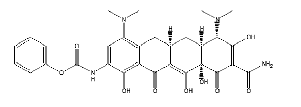 | * | * | * |
| DU | 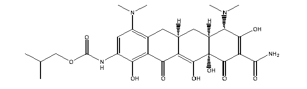 | ** | * | ** |
| DV | 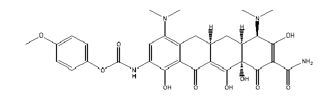 | ** | * | * |
| DW | 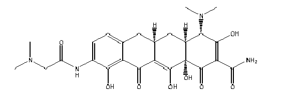 | ** | ** | ** |
| DX | 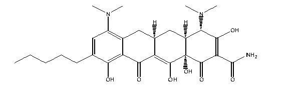 | ** | * | ** |
| DY | 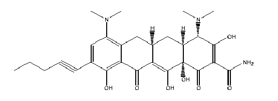 | ** | * | ** |
| DZ | 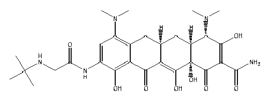 | * | ** | ** |
| EA | 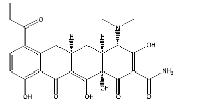 | *** | ** | ** |
| EB | 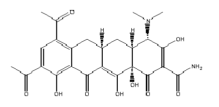 | * | * | ** |
| EC | 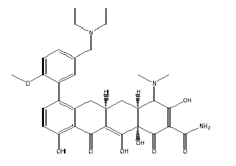 | * | ** | ** |
| ED | 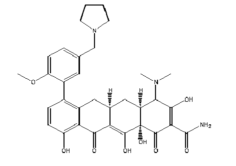 | ** | ** | ** |
| EF | 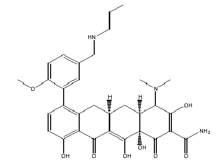 | ** | ** | ** |
| EG | 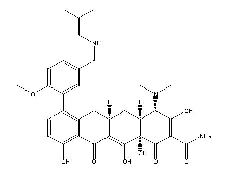 | *** | ** | ** |
| EH | 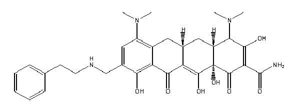 | ** | ** | ** |
| EI | 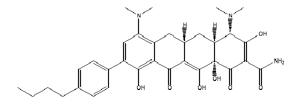 | *** | * | * |
| EJ | 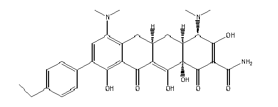 | * | * | ** |
| EK | 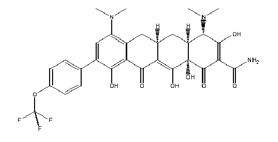 | ** | * | ** |
| EL | 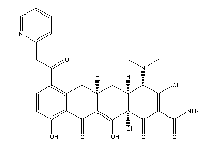 | *** | ** | ** |
| EM | 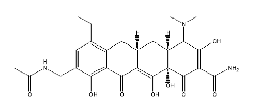 | * | * | ** |
| EN | 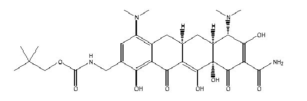 | ** | * | ** |
| EO | 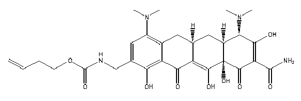 | * | * | ** |
| EP | 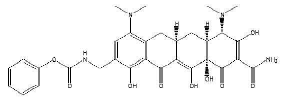 | * | * | ** |
| EQ | 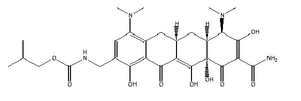 | ** | * | ** |
| ER | 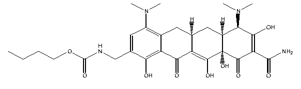 | ** | * | ** |
| ES | 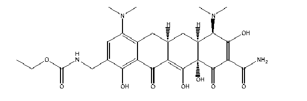 | * | * | ** |
| ET | 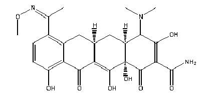 | ** | ** | ** |
| EU | 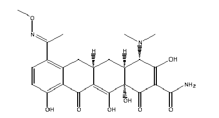 | ** | ** | ** |
| EV | 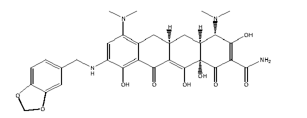 | ** | * | * |
| EZ | 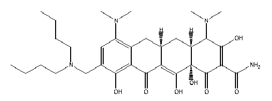 | ** | * | ** |
| FA | 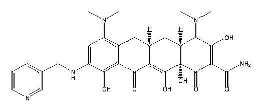 | * | * | * |
| FB | 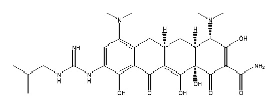 | * | * | * |
| FC | 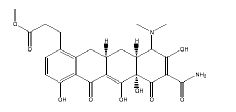 | * | * | ** |
| FD | 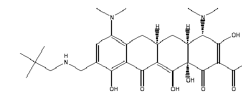 | * | ** | ** |
| FE | 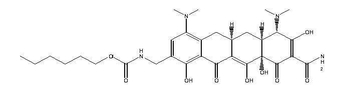 | ** | * | ** |
| FG | 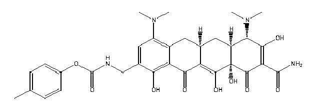 | * | * | ** |
| FH | 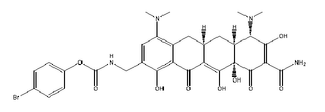 | *** | * | ** |
| FI | 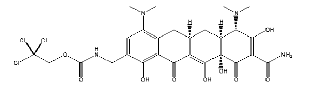 | *** | * | ** |
| FJ | 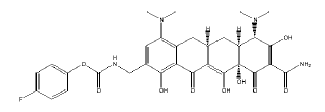 | * | * | ** |
| FL | 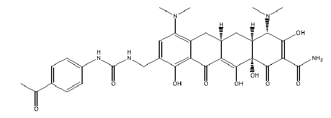 | * | * | ** |
| FM | 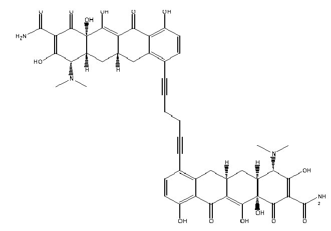 | *** | * | * |
| FN | 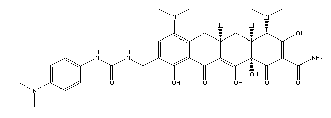 | ** | * | ** |
| FO | 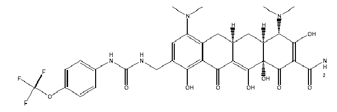 | *** | * | ** |
| FR | 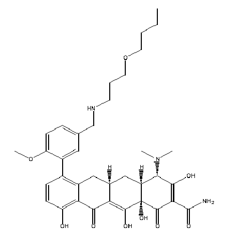 | *** | ** | ** |
| FS | 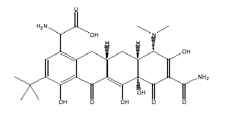 | * | * | * |
| FT | 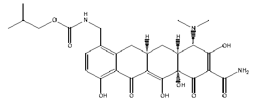 | * | * | ** |
| FU | 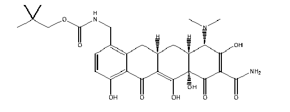 | *** | * | ** |
| FV | 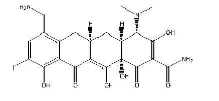 | * | * | * |
| FX | 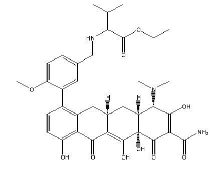 | *** | * | ** |
| FY | 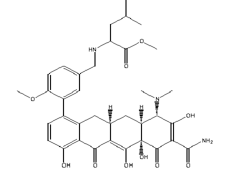 | *** | * | ** |
| FZ | 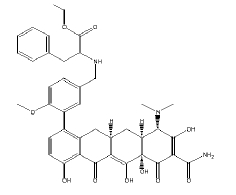 | ** | * | ** |
| GA | 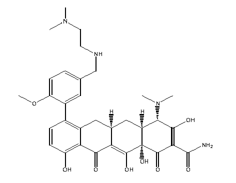 | * | * | ** |
| GB | 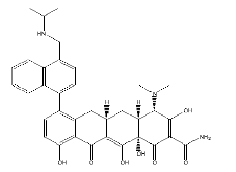 | *** | ** | ** |
| GC | 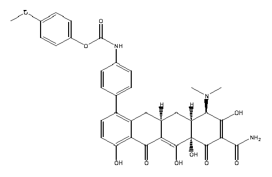 | *** | * | * |
| GD | 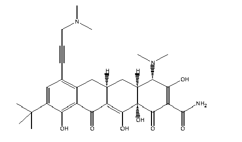 | *** | * | ** |
| GE | 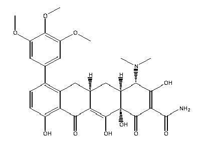 | ** | * | ** |
| GF | 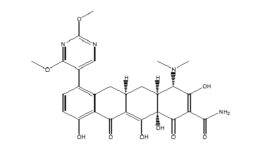 | ** | ** | ** |
| GH | 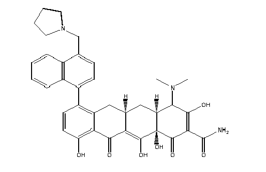 | ** | ** | ** |
| GJ | 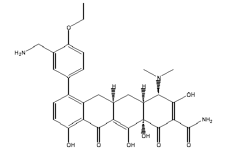 | ** | ** | ** |
| GK | 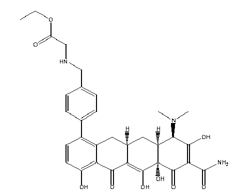 | * | * | ** |
| GL | 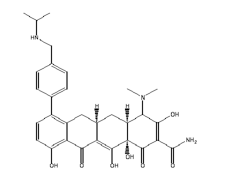 | ** | ** | ** |
| GM | 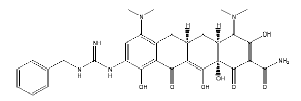 | * | * | * |
| GO | 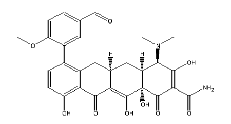 | * | * | ** |
| GP | 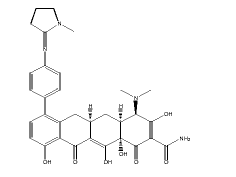 | * | * | ** |
| GQ | 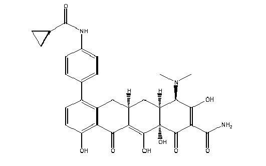 | *** | * | ** |
| GS | 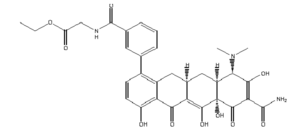 | * | * | ** |
| GT | 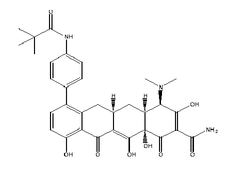 | * | * | ** |
| GU | 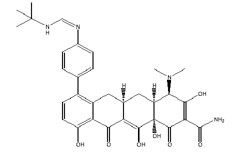 | * | ** | ** |
| GV | 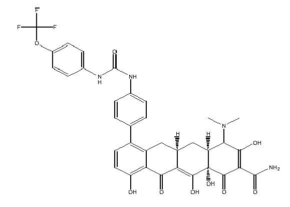 | ** | * | * |
| GX | 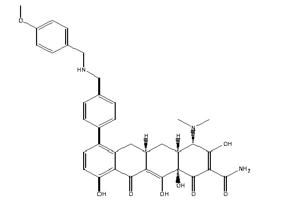 | ** | ** | ** |
| GY | 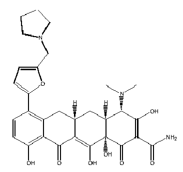 | *** | ** | ** |
| HC | 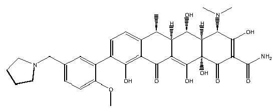 | * | * | * |
| HE | 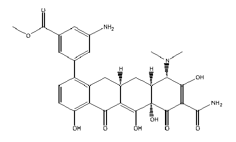 | ** | * | ** |
| HF | 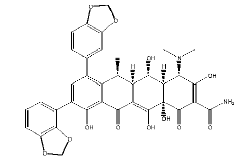 | * | * | ** |
| HH | 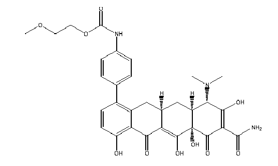 | * | * | ** |
| HI | 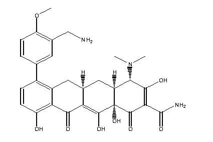 | *** | * | ** |
| HJ | 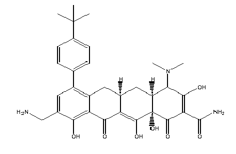 | *** | ** | ** |
| HK | 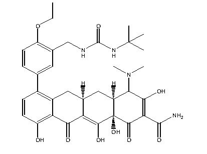 | ** | * | ** |
| HL | 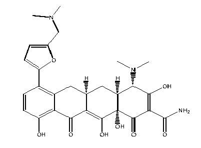 | *** | ** | ** |
| HM | 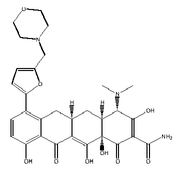 | ** | ** | ** |
| HN | 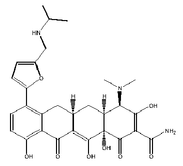 | *** | ** | ** |
| HO | 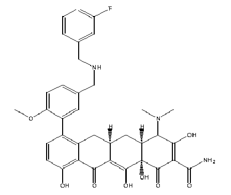 | ** | ** | ** |
| HP | 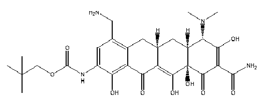 | *** | * | * |
| HQ | 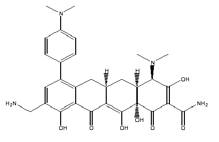 | *** | * | ** |
| HR | 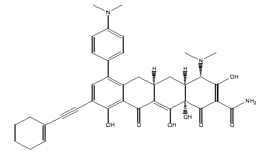 | ** | * | * |
| HS | 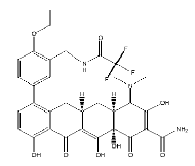 | ** | * | ** |
| HT | 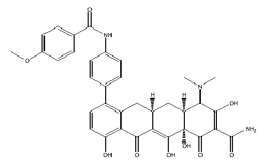 | *** | * | ** |
| HV | 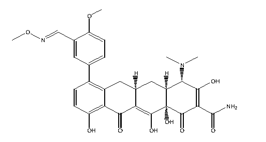 | *** | * | ** |
| HW | 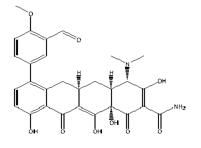 | ** | ** | ** |
| HX | 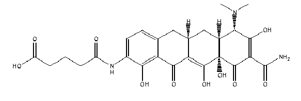 | * | * | * |
| IA | 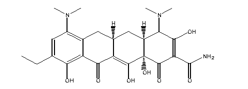 | ** | * | ** |
| IB | 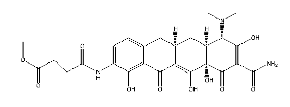 | * | * | * |
| IC | 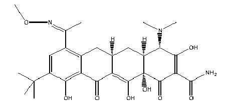 | ** | * | ** |
| ID | 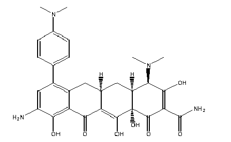 | * | * | ** |
| IE | 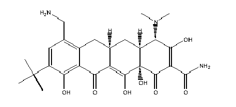 | *** | * | * |
| IG | 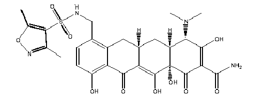 | * | * | ** |
| IH | 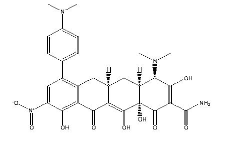 | ** | * | ** |
| IJ | 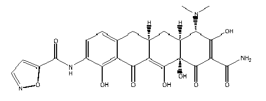 | * | * | ** |
| IK | 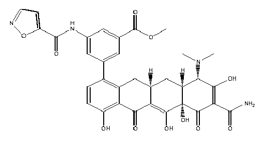 | ** | * | ** |
| IL | 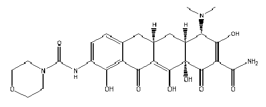 | * | * | * |
| IM | 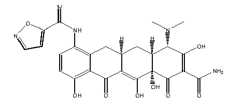 | * | * | ** |
| IN | 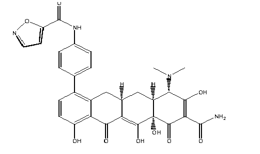 | ** | * | ** |
| IP | 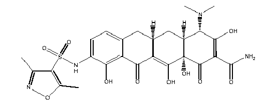 | * | * | ** |
| IT | 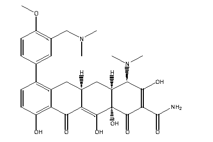 | *** | ** | ** |
| IY | 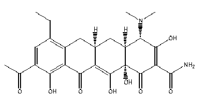 | * | * | ** |
| IZ | 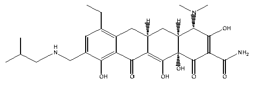 | ** | ** | ** |
| JA | 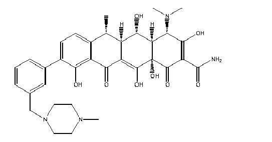 | *** | * | * |
| JC | 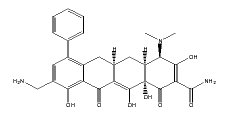 | *** | * | * |
| JD | 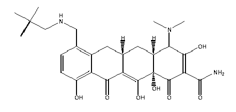 | *** | ** | ** |
| JF | 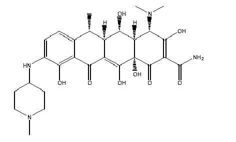 | * | ** | ** |
| JG | 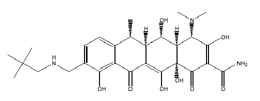 | *** | ** | ** |
| JH | 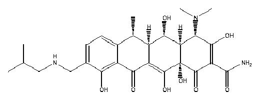 | * | ** | ** |
| JI | 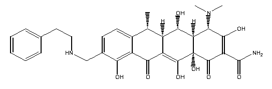 | * | ** | ** |
| JK | 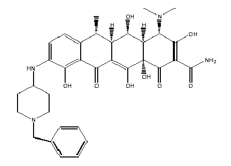 | *** | ** | ** |
| JL | 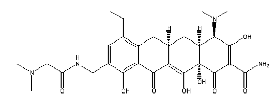 | * | * | ** |
| JM | 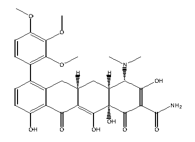 | * | * | ** |
| JN | 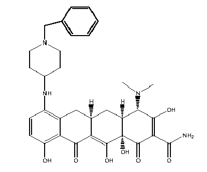 | ** | ** | ** |
| JO | 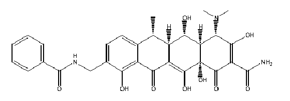 | * | * | ** |
| JR | 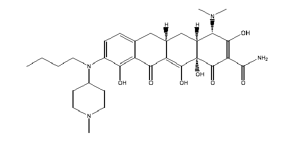 | * | * | * |
| JS | 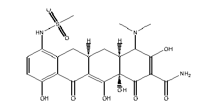 | * | * | ** |
| JU | 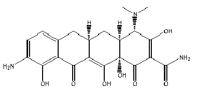 | * | ** | ** |
| JV | 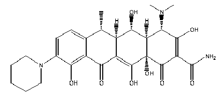 | *** | * | ** |
| JW | 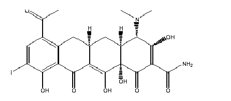 | * | * | * |
| JX | 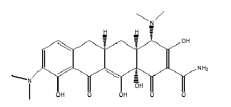 | *** | * | ** |
| JY | 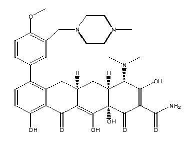 | *** | * | ** |
| JZ | 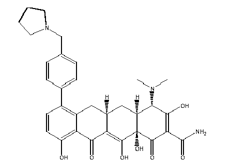 | *** | ** | ** |
| KA | 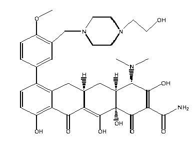 | * | * | ** |
| KB | 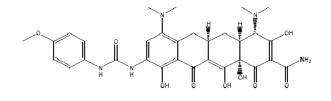 | * | * | ** |
| KC | 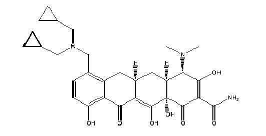 | *** | ** | ** |
| KD | 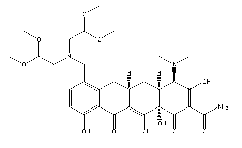 | * | * | ** |
| KE | 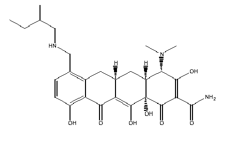 | *** | ** | ** |
| KF | 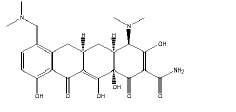 | *** | ** | ** |
| KG | 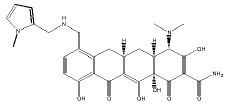 | *** | ** | ** |
| KH | 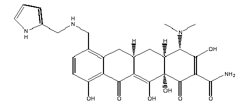 | *** | ** | ** |
| KI | 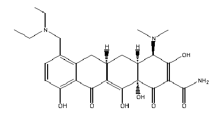 | *** | ** | ** |
| KJ | 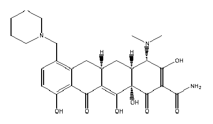 | *** | ** | ** |
| KK | 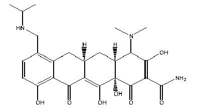 | ** | ** | ** |
| KM | 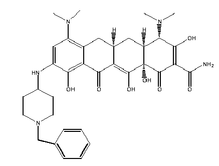 | ** | * | * |
| KR | 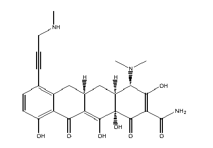 | *** | ** | ** |
| KT | 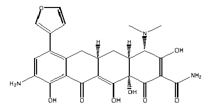 | * | ** | ** |
| KU | 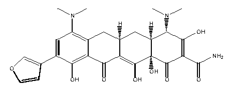 | * | * | ** |
| KX | 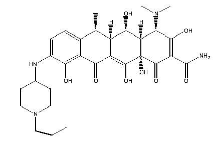 | ** | ** | ** |
| KY | 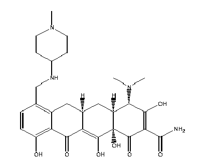 | * | * | * |
| KZ | 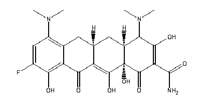 | * | ** | ** |
| LC | 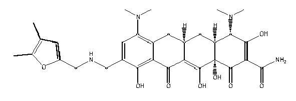 | ** | ** | ** |
| LD | 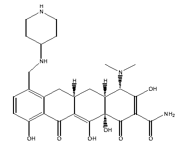 | * | * | * |
| LE | 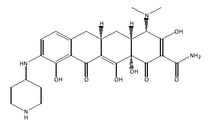 | * | * | * |
| LF | 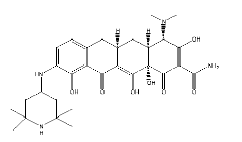 | * | * | ** |
| LI | 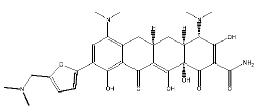 | * | * | ** |
| LJ | 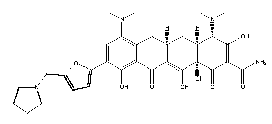 | ° | * | * |
| LK | 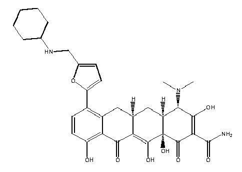 | ** | ** | ** |
| LL | 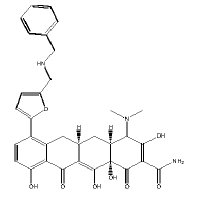 | *** | ** | ** |
| LM | 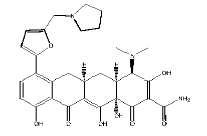 | *** | ** | ** |
| LN | 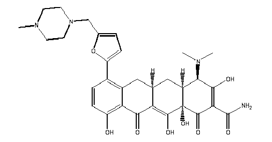 | ** | ** | ** |
| LR | 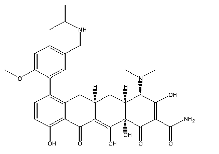 | * | ** | ** |
| MB | 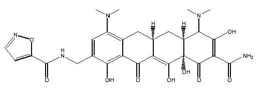 | *** | * | ** |
| MD | 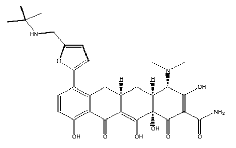 | *** | ** | ** |
| MF | 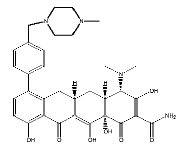 | *** | ** | ** |
| MK | 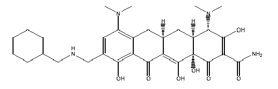 | ** | ** | ** |
| MO | 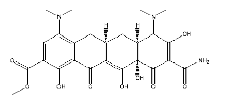 | * | * | * |
| MQ | 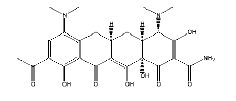 | * | * | * |
| MR | 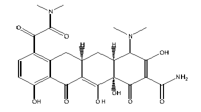 | * | ** | ** |
| MS | 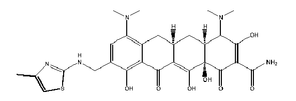 | * | * | ** |
| MT | 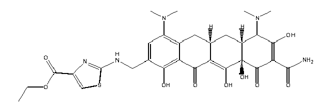 | * | * | * |
| MU | 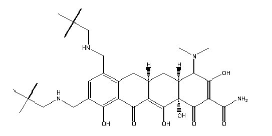 | ** | ** | ** |
| MW | 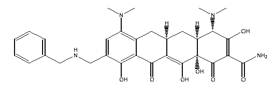 | * | ** | ** |
| MZ | 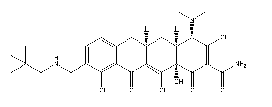 | *** | ** | ** |
| NA | 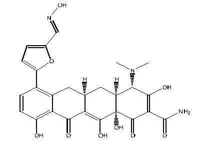 | *** | ** | ** |
| NE | 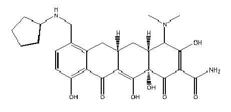 | ** | ** | ** |
| NF | 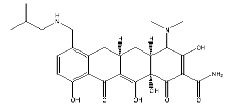 | *** | ** | ** |
| NJ | 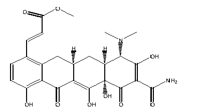 | * | * | ** |
| NK | 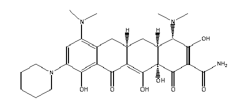 | ** | * | * |
| NL | 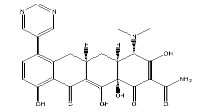 | * | ** | ** |
| NM | 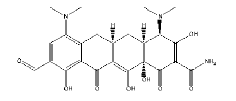 | * | ** | ** |
| NN | 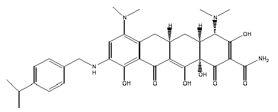 | ** | * | ** |
| NO | 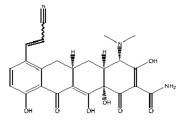 | *** | ** | ** |
| NP | 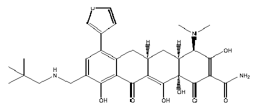 | *** | ** | ** |
| NQ | 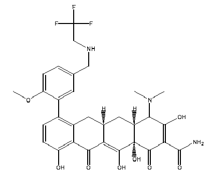 | * | * | ** |
| NT | 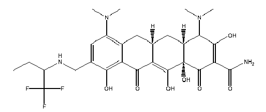 | * | * | ** |
| NZ | 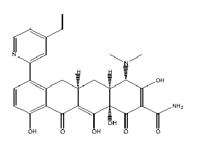 | * | ** | ** |
| OH | 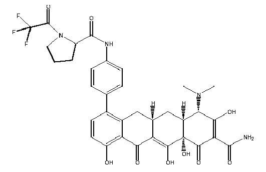 | * | * | ** |
| OJ | 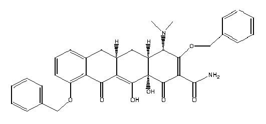 | ** | * | * |
| OM | 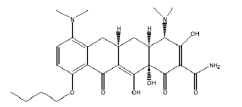 | ° | * | * |
| ON | 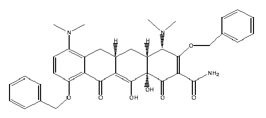 | * | * | * |
| OQ | 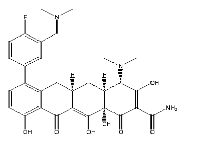 | ** | ** | ** |
| OR | 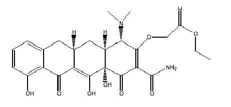 | * | * | * |
| OS | 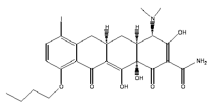 | * | * | * |
| OT | 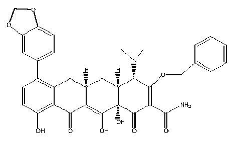 | * | NT | NT |
| OU | 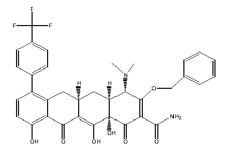 | *** | * | ** |
| OY | 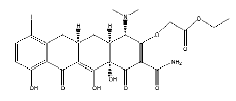 | * | * | ** |
| PD | 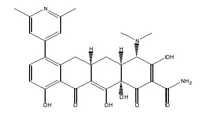 | ** | ** | ** |
| PF | 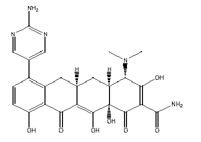 | * | ** | ** |
| PJ | 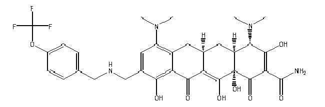 | * | * | ** |
| PN | 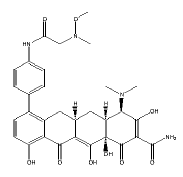 | ** | * | ** |
| PQ | 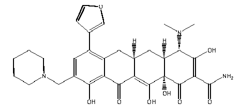 | * | ** | ** |
| PX | 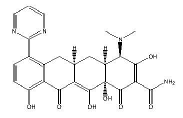 | * | * | ** |
| QA | 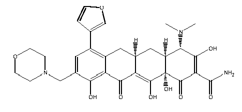 | * | ** | ** |
| QC | 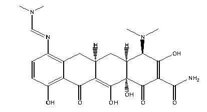 | * | ** | ** |

### Example 5: In vitro Neuroprotection Assay: Protection of Cultured Cortical Neurons from Excitotoxic Injury Induced by NMDA (NE Assay)

This assay shows the ability of tetracycline derivatives to protect cultured murine cortical neurons from excitotoxic injury induced by NMDA exposure.

Primary cortical astrocyte cultures were prepared from one-day old mice, as described in the literature procedure (Tikka, TM et al. J Immunol. 2001 Jun 15;166(12):7527-33). After decapitation, the forebrain cortices were collected, cleaned from meninges and, after mincing, dissociated by incubation in papain/Dnase solution followed by trituration. The dissociated cells were suspended in a culture medium consisting of Eagle's minimal essential medium (MEM) with 10% heat-inactivated fetal bovine serum and glutamine (2mM) and plated in 75 cm² cell culture flasks. The medium was replaced with fresh culture medium at day 7. At confluence (day 12-15), the astrocytes were trypsinized and re-plated into 24-well culture vessels. On day 7-8, the neurons were cultured on top of these confluent astrocyte monolayers.

### Cortical Neurons

Cortical neuron suspensions were prepared from embryonic day 17 mice. The cortices were collected and meninges removed. The tissue was minced into small pieces and incubated in trypsin solution. The tissue was suspended using a pipette and after centrifugation resuspended and plated on top of astrocyte cultures at a density of 250,000 cells / well to 24-well culture vessel in Eagle's minimal essential medium (MEM, Earle's salts) supplemented with 20 mM glucose, 2 mM glutamine, 10 % fetal bovine serum, and 10 % heat-inactivated horse serum (HS). Medium was changed after 5 days to MEM containing 20 mM glucose, 2 mM glutamine, and 10% HS, as well as cytosine arabinoside (final concentration 10 µM) to inhibit cell division, and incubated for 2 days. Subsequently, cultures were fed twice weekly with MEM supplemented with 20 mM glucose, 2 mM glutamine and 10 % HS, and used at day 12-15.

### Excitotoxicity

On the day of experiment, the culture medium was replaced with 1) MEM, 2) MEM containing the test tetracycline compounds. Positive control drug included 10 µM MK-801 (Sigma). Thirty minutes later NMDA (with a final concentration of 62.5 µM) was added to the wells (causing 50-75 % cell death). As a control for total neuronal death, a 24 hour incubation with 500 µM NMDA was used (100 % cell death control).

### Assessment of Cell Death and Results

The cell viability was assessed by measuring lactate dehydrogenase (LDH) release 24 hours after starting the exposure. The LDH release to the culture medium was measured from cell-free medium using Sigma LDH reagent employing kinetic measurement of conversion of lactate to pyruvate using NADH as a cofactor. The rate of increase in absorbance at 340 nm was directly proportional to the activity of LDH in the sample, and was measured with a Labsystems Multiskan ELISA reader.

The compounds for which demonstrated good neuroprotection in this assay include Compounds C, D, G, H, M, Q, BP, CD, CW, EV, IE, JC, JD, KF, LJ, and OM from Table 2.

### Example 6: In vitro Neuroprotection Assay: Neuroprotection of an SH-SY5Y Neuroblastoma Cell Line (NSN Assay)

In this example, the ability of tetracycline compounds to protect human neuroblastoma cells from oxidative stress is determined.

Human SH-SY5Y neuroblastoma cells are maintained in Dulbecco's modified Eagle's medium supplemented with 10% fetal bovine serum and 2% penicillin-streptomycin, and incubated at 37°C in a humidified atmosphere with 5% CO₂ according to a literature procedure (Zhu, S et al. Nature. 2002 May 2;417(6884):74-8). The cells are routinely sub-cultured using 0.05% trypsin-EDTA solution. The cells are seeded at 10³ cells/well in 96-well plates and grown until each well is 75-80 % confluent. To induce oxidant injury, the SHSY-5Y cells are incubated with various doses of hydrogen peroxide for 24 hours in order to identify a dose for screening that provides a model for a chronic injury to the cells. An optimal dose should result in approximately 70% loss in cell survival as compared to the control after 24 hours. The final concentration of H₂O₂ to be used in the assay will be less than 300uM.

For tetracycline compound inhibition of SH-SY5Y cell death, human neuroblastoma SH-SY5Y cells are preincubated with media containing drug for 24 hours at 37 °C, and later exposed to 6 mM H₂O₂ for 4 hours. Cells are then incubated with calcein-AM (1mM, Molecular Probes) in PBS for 40 min at 37 °C. Cell viability is determined using a fluorescence reader. Viability is converted to cell death ratio. Cells can be exposed to other agents such as thapsigargin (THG) to induce cell death. For example, cells can be preincubated with the tetracycline for 1 hour and then exposed to 15mM THG. After 12 hours, cell death is evaluated by MTT assay or by using calcein-AM as described above.

### Example 7: In vitro Parkinson's Disease Assay: Protection of Dopaminergic Cell Assay (PDC Assay)

In this example, the ability of the tetracycline compounds of the invention to protect dopaminergic cells is used to predict the ability of the tetracycline compounds to treat Parkinson's Disease.

Different groups of tetracycline compounds are tested in the *in vitro* model of dopaminergic neuron injury, in which MES23.5 cells or primary cultures of embryonic rat mesencephalon are cocultured with purified rat microglia and treated with lipopolysaccharide, PD IgG or dopa-quinone-modified MES 23.5 cell membranes to induce microglia-mediated injury according to literature procedure (Le W. et al., J Neurosci. 2001 Nov 1;21(21):8447-55). Detailed dosage and temporal response of tetracycline derivatives treatment is carried out. The neuroprotection is examined in the cultures by quantitatively counting the number of tyrosine hydroxylase (TH)-positive cells or by biochemical determination of TH activity in a blind fashion. Each compound will be tested three times in a triplicate manner. To determine the inhibitory effects of the tested tetracycline derivatives on microglia, the culture media is collected for measuring the levels of TNF-a released from microglia, a biochemical marker of microglial activation. The observed biological activity, neuroprotection of dopaminergic cells and inhibition of microglial activation is used to develop a structure activity relationship (SAR).

### Example 8: In vitro Neuroprotection Assay: Cytochrome C Release Assay (CCR Assay)

This example shows the ability of tetracycline compounds of the invention to inhibit cytochrome C release.

Mouse liver mitochondria are prepared as described by Luo et al. (Cell 94,481-490 (1998)) and resuspended in MRM buffer (250 mM sucrose, 10 mM HEPES, pH 7.5, 1 mM ATP, 5 mM sodium succinate, 80mM ADP, 2 mM K2 HPO4 ) at a concentration of 0.5 mg/mL, according to a literature procedure (Zhu S. et al. Nature. 2002 May 2;417(6884):74-8). Rat liver mitochondria are isolated from 4-6-month-old Fischer 344 X brown Norway F1 rats by differential centrifugation as described. Non-synaptosomal rat brain mitochondria are prepared from forebrains of 8 week old rats by ficoll gradient purification.
To assay the effects of the tetracycline compounds on cytochrome C release, a 25 µl aliquot of 0.5mg/mL mitochondrial extract preparation is preincubated with the test substituted tetracycline compounds for 5 minutes in MRM buffer. To this CaCl₂ or other inducers of cytochrome C release, such as purified Bid protein, are added. The mixtures are incubated for 30 minutes to 1 hour at 37 °C. The mixes are then centrifuged at 10,000g at 4 °C for 10 minutes and the supernatant is evaluated for release of cytochrome C by Western blot.

### Example 9: In vivo Amylotropic Lateral Sclerosis Mouse Model

In this example, the tetracycline compounds of the invention are tested *in vivo* for the treatment of amylotropic lateral sclerosis using a mouse model.

ALS mice (Jackson Laboratories) are injected intraperitoneally daily with saline as a control or the test tetracycline compounds according to a literature procedure (Zhu S et al. Nature. 2002 May 2; 417 (6884):74-8). The tetracycline compounds may be given by other routes including oral administration. Strength and coordination are evaluated weekly by a standard Rotarod test. The disease is defined as the first day a mouse can not remain on the Rotarod for 10 minutes at 15 r.p.m. Mortality is scored as age of death or age when the mouse is unable to right itself within 30 seconds. Tissues from animals can be evaluated for cytochrome C release, caspase activation and iNOS protein levels using Western blots and histochemical staining. Additionally, methods of detecting transcript levels (ie. Northern blots, quantitative PCR or microarrays) are used to evaluate message levels.

### Example 10: In vivo Huntington's Disease Mouse Model

In this example, the tetracycline compounds of the invention are tested *in vivo* for the treatment of Huntington's disease using a mouse model.

R6/2 mice (Jackson Laboratories, Bar Harbor, Maine) are randomly assigned to three groups, according to a literature procedure (Chen M et al. Nat Med. 2000 Jul;6(7):797-801). At 6 weeks of age, mice are treated with the test tetracycline compounds. The test tetracycline compounds can be given by a number of routes. Motor performance is evaluated weekly from 5 to 13 weeks on a Rotarod), at 5 and 15 rpm. If the mouse remains on the rod for 10 minutes the test is completed and scored as 10 minutes. Tissues from animal are evaluated for cytochrome C release, caspase activation and iNOS protein levels using Western blots and histochemical staining. Additionally, methods of detecting transcript levels (i.e., Northern blots, quantitative PCR or microarrays) are used to evaluate message levels.

### Example 11: In vivo Parkinson's Disease Model

In this example, a mouse model is used to determine the ability of the tetracycline compounds to treat Parkinson's Disease. Other models which can be used are described in Wu D.C. et al. J Neurosci. 2002 Mar 1;22(5):1763-71 and Du Y. et al. PNAS 2001 Dec 4;98(25):14669-74.

Eight-week-old male C57BLy6 are used in the example. Mice (5-7 per group) are administered the test tetracycline compounds by any of a number or routes including oral gavage before, during, and after 1-methyl-4-phenyl-1,2,3,6-tetrahydropyridine (MPTP) administration. An untreated control group and MPTP-only group are included. The MPTP-treated groups receive four injections of MPTP-HCl (20 mg/kg, i.p.) in saline at 2-h intervals in a single day (four injections total) and are killed 7 days after the last injection. Tissues from each animal are evaluated for cytochrome C release, caspase activation, tyrosine hydroxylase and iNOS protein levels using Western blots and histochemical staining. Additionally, methods of detecting transcript levels, such as Northern blots, quantitative PCR and microarrays, are used to evaluate message levels.

### Example 12: In vivo Multiple Sclerosis Rat Model

In this example, a rat model is used to determine the ability of the tetracycline compounds to treat Multiple Sclerosis. Other models which can be used are described in Brundula V. et al. Brain 2002 Jun;125(Pt 6):1297-308 and Popovic N. et al. Ann Neurol. 2002 Feb;51(2):215-23.

This example is performed on 6- to 8-week-old female DA rats. The recombinant extracellular immunoglobulin domain of myelin oligodendrocyte protein (MOG) is expressed and purified from *E. coli.* The rats are immunized subcutaneously (s.c.) at the base of the tail with either 10 µg MOG in complete Freund's adjuvant or 100 µg MOG emulsified in incomplete Freund's adjuvant in a total volume of 100 µl. The animals are weighed and examined daily for clinical signs of experimental autoimmune encephalomyelitis (EAE). The test tetracycline compounds are freshly dissolved in distilled water or phosphate-buffered saline (PBS) and are administered daily by intraperitoneal (i.p.) injections at a dosage of 45 mg/kg rat body weight. The animals are scored for hind limb paralysis by standard methods. Tissues from each animal are evaluated for demyelination, cytochrome C release, caspase activation, tyrosine hydroxylase and iNOS protein levels using Western blots and histochemical staining. Additionally, methods of detecting transcript levels such as Northern blots, quantitative PCR and microarrays, are used to evaluate message levels.

### Example 13: In vivo Stroke Rat Model

In this example, a rat model is used to determine the ability of the tetracycline compounds to treat stroke. Other models which can be used are described in Yrjanheikki J. et al. PNAS 1998 Dec 22;95(26):15769-74 and Yrjanheikki J et al. PNAS 1999 Nov 9;96(23):13496-500.

Male Sprague-Dawley rats weighing 210-250 grams are housed at a standard temperature and in a light-controlled environment with *ad libitum* access to food and water. The animals are divided randomly into tetracycline-treatment and control groups. Focal cerebral ischemia is produced by introduction of an intraluminal nylon thread.

The rats are anesthetized with 5% (vol/vol) isoflurane (70% N₂O/30% O₂); during the operation, isoflurane concentration was reduced to 0.5%. The rectal temperature is maintained between 37.0 °C and 37.5 °C with a heating pad. The right common carotid artery is exposed, and the external carotid artery ligated. A 0.25-mm monofilament nylon thread (Kuusamo Uis-tin, Kuusamo, Finland) with the tip blunted with sandpaper is inserted 22-23 mm into the internal carotid artery up to the MCA. After 90 minutes of ischemia, the MCA blood flow is restored by removing the thread.

For recording physiological variables, a polyethylene catheter is inserted into the femoral artery. Arterial blood pressure, PO₂, PCO₂, pH, and plasma glucose are measured during and 15 minutes after ischemia. Section of brain tissue are evaluated to determine the size of the infarct. Tissues from each animal are evaluated for demyelination, cytochrome C release, caspase activation, tyrosine hydroxylase and iNOS protein levels using Western blots and histochemical staining. Additionally, methods of detecting transcript levels such as Northern blots, quantitative PCR and microarrays are used to evaluate message levels.

### Example 14: In vivo Rabbit Cornea Angiogenesis Model

In this example, a rabbit cornea is used to determine the ability of the tetracycline compounds to treat cancer. The cornea provides an avascular matrix into which blood vessels can grow and be quantitated. Other models which can be used are described in Tamargo RJ et al. Cancer Res. 1991 Jan 15;51(2):672-5 and Masumori N. et al. Adv Dent Res. 1998 Nov;12(2):111-3.

The corneas of anesthetized New Zealand white rabbits are implanted with a serially transplantable tumor syngeneic to the animals. The test tetracycline compound are administered orally, intravenously, intraperitoneally or by using a controlled release polymer. The angiogenesis response of the transplanted tumor material is quantitated by measuring both vessel length, number of vessels and the span of blood vessels.

### Example 15: In vitro Cancer Assay: Matrigels as Model of Angiogenesis

In this example, matrigels are used to determine the ability of the tetracycline compounds to treat cancer.

In this example, a solid gel of basement proteins is prepared from a Engelbreth Holm-Swarm (EHS) mouse tumor. When placed in a 96-well tissue culture plate, the gel forms an *in vitro* analog of the basement membrane. Endothelial cells, in solution, are placed on top of the gel, allowing the cells to align and form tube-like structures which are observed under an inverted light microscope. Tube formation is a multi-step process involving cell adhesion, migration, differentiation and growth. The test tetracycline compounds are added to the matrigel. The ability of the test tetracycline compounds to alter cell adhesion, migration, differentiation and growth is determined by observing the effects of the compounds on the cells of the matrigel.

### Example 16: In vivo Cancer Mouse Model: Metastasis in Immunosuppressed Mice

In this example, immunosuppressed mice are used to determine the ability of the tetracycline compounds to treat cancer. Other models which can be used are described in Parangi S. et al. PNAS 1996 Mar 5;93(5):2002-7 and Seftor R.E. et al. Clin Exp Metastasis. 1998 Apr;16(3):217-25.

Immunosuppressed mice (scid/scid females) are injected via the tail vein with C8161 cells, which results in a large number of lung metastases. Test tetracycline compounds are administered intravenously, intraperitoneally or orally. After 24 days, the animals are sacrificed and number of metastases to the lungs quantitated.

### Example 17: In vivo Aortic Aneurysms Model

In this example, mice are used to determine whether substituted tetracycline compounds are effective agents to treat aortic aneurysms, as described in Prall, et al. J. Vasc. Surg. 2002:35: 923-929. Other models which can be used are described in Curci, et al. J. Vasc. Surg. 2000; 31: 326-342.

C57BL/6 strain mice are given the substituted tetracycline compound beginning at 7 weeks of age prior to the induction of an aortic aneurysm. A separate group of mice is given the substituted tetracycline compound immediately following the induction of the aortic aneurysm. The tetracycline compound is dissolved in the drinking water of the mice and prepared in concentrations estimated based on the average weight of a C57BL/6 mouse and the average daily intake of water for a mouse.

Aortic aneurysms are induced in mice at 8 weeks of age according to the procedure described in Prall *et al., supra.* Briefly, mice undergo anesthesia and the entire infrarenal abdominal aorta is isolated from the surrounding retroperitoneal structures. The diameter of the aorta is measured to the nearest micrometer using a video micrometer. Diameter measurements are taken at the maximal aortic diameter. The aneurysm is induced by bathing the aorta with 0.25 mol/L CaCl₂ for 15 minutes, and then rinsing with 0.9% NaCl. Control mice are just bathed in NaCl. At five and ten weeks following surgery, blood samples are taken from the mice to assess the level of the substituted tetracycline compound.

Ten weeks following the surgery, a laparotomy is repeated and the infrarenal aorta is isolated and measured. The initial and final aortic diameters are compared. The presence of an aortic aneurysm is defined as an increase in the aortic diameter of greater than 50% of the original diameter. Data from the aortic aneurysm and the determined blood serum level of the substituted tetracycline compound are then compared among the experimental and control groups to determine whether the substituted tetracycline compound is able to suppress an aortic aneurysm.

### Example 18: In vivo Diabetic Complications Rat Model

In this example, rats are used to determine if tetracycline compounds are effective agents that can be used to treat diabetic complications. Other models which can be used are described in Ryan et al. Curr. Med. Chem. 2001;8(3):305-316.

Viral-free adult male Sprague-Dawley rats are distributed into nine groups, with 5-7 rats in each group. Diabetes is induced in each rat by I.V. administration of streptozotocin (70 mg/kg body weight). A weekly diagnostic test, including a glucose test strip (Tes-Tape, Eli Lilly), is given to determine the diabetic status of each rat. One week after the diabetes induction, diabetic rats are given a daily oral dose, including but not limited to 15mg/kg, of the tetracycline compound for a period of 3 weeks.

After the 3 week period, rats are sacrificed, anaesthetized with pentobarbital and their blood is collected by cardiac puncture. Serum samples from the collected blood are then analyzed for glucose concentration using a glucose oxidase (Sigma Chemical Co., St. Louis, MO). At sacrifice, skins from the rats are also removed for further study. Skin samples are homogenized and protein extracts from the tissue are removed through standard protocols known in the art.

The protein extract from the skin of the diabetic rats is examined for collagenase and gelatinase activity to determine if the tetracycline compound is effective at reducing and/or inhibiting MMP activity in diabetic rats. Collagenase activity can be determined by a standard collagenolysis assay as described in Golub et al. J Peridontal Res. 1983:18:23. Gelatinase activity can be determined by standard methods, including those described in McCroskey et al. Biochem J. 1975;152:131.

### Example 19: In vivo Arteriosclerosis Rat Model

In this example, rats are used to determine if tetracycline compounds are effective agents that can be used to treat arteriosclerosis. Other models which can be used are described in Bendeck, et al. Amer. J. Path. 2001:160(3): 1089-1095.

Sprague-Dawley rats (3 to 4 months old) are anaesthetized by intraperitoneal injection of xylazine and ketamine. A balloon catheter injury of the left common carotid artery is performed as described in Bendeck et al. Circ. Res. 1994:75: 539-545. Tetracycline compounds are administered to the rats through their drinking water at a dose of 30 mg/kg beginning 24 hours prior to the surgery described above.

Rats are then sacrificed at various time points after the surgery based on previous studies that have determined the kinetics of the injury response. Medial smooth muscle cell (SMC) proliferation is measured in the media (2, 4, 7, and 14 days) and intima (7 and 14 days) as determined in Clowes et al. Lab. Invest. 1983:49:327-333. Migration of cells from media to the intima and MMP activity is measured at 4 days (Bendeck *et al.,* 1994, *supra*)*.* Cells are labeled using a 50 mg pellet of 5-bromo-2'deoxyuridine according to Bendeck *et al,* 2002, *supra*)*.*

SMC replication is determined following sacrifice by immunostaining carotid cross-sections for BrdU and determining the percentage of BrdU-labeled cells as described in Strauss et al. J. Clin. Invest. 1992:90: 2044-2049. SMC migration is determined as described in Bendeck *et al.* 2002, *supra.* MMP activity is measured by gelatin zymography as described in Bendeck *et al.* 1994, *supra.*

### Example 20: In vivo Acute Respiratory Distress Syndrome Pig Model

In this example, hybrid pigs are used to determine the ability of the tetracycline compounds to treat ARDS. Other models which can be used are described in Carney D.E. et al. Circulation, 1999 Jul 27;100(4):400-6.

Yorkshire hybrid pigs (15 to 20 kg) are anesthetized with sodium pentobarbital and a bolus infusion of pancuronium bromide. The technique for initiating CPB is described in the literature (e.g., Picone A.L. et al. Ann Thorac Surg. 1999;67:978 -985.) Pigs receiving lipopolysaccharide (LPS) were infused with 1 mg/kg of *Escherichia coli* lipopolysaccharide mixed in 500 mL of saline and delivered over 1 hour via a volumetric infusion pump. Pigs randomized to an arm not exposed to LPS received sham LPS (500 mL saline vehicle only). The test tetracycline compounds are dissolved in a suitable vehicle and administered intravenously. Blood oxygenation levels are monitored in the animals. Tissue and fluid from the animals are additionally assayed for markers of ARDS, such as MMP levels, elastase levels, NO levels and neutrophil infiltration.

### Example 21: In vivo Septic Shock Mouse Model

In this example, mice are used to determine the ability of the tetracycline compounds to treat endotoxic shock. Other models which can be used are described in Milano S. et al. Antimicrob Agents Chemother. 1997 Jan;41(1):117-21 and Shapira L. et al. Infect Immun. 1996 Mar;64(3):825-8.

Sabra mice are injected with *Salmonella typhosa* lipopolysaccharide (LPS) intravenously as a model for endotoxic shock. LPS is dissolved in a sterile pyrogen-free saline solution and dispersed by brief sonication. The experimental animals are given a solution containing the tetracycline compounds by gavage 20 minutes prior to intravenous LPS injection. The tetracycline compounds may, however, be given by other routes. Drug administration is repeated 6 and 24 hours after the LPS injection but at half of the original dose. The control animals receive saline. Mouse mortality is monitored twice daily for 72 hours, and in some experiments, monitoring is continued once daily for up to 3 weeks.

For the determination of TNF-α levels in serum, mice are challenged with 500 mg of LPS intravenously. Simultaneously, 1 ml of a solution containing the test tetracycline compound is given by gavage to the experimental animals, while the control animals receive saline. The animals are bled from the infraorbital plexus 2 hours after the LPS challenge, and the levels of TNF-α in the serum are determined by two-site enzyme-linked immunosorbent assay (ELISA) with anti-mouse TNF-α antibodies. Additional markers of inflammation such as NO are also assayed using standard techniques.

### Example 22: In vivo Wound Healing Rat Model

In this example, rats are used to determine if substituted tetracycline compounds are effective agents that can be used to help wounds heal. Other models which can be used are described in Pirila, et al. Curr. Med. Chem. 2001;8:281-294.

Sprague-Dawley rats (6 months old) are either sham operated or ovariectomized. After 120 days, both control and ovariectomized are anaesthetized with xylazine and ketamine. The dorsal skin is shaved and wiped with a 75% alcohol solution, and washed with 0.9% saline. Eight full thickness skin wounds are made in the dorsal thorax using a 6 mm diameter circular biopsy punch. Wounds are allowed to heal. The wound biopsies are standardized by coring the skin until the biopsy punch reaches the cutaneous muscle. White petroleum is directly applied to the wound immediately after the injury, and daily thereafter for 7 days.

A test group of rats which were operated upon receives a daily dose of the tetracycline compounds orally by gavage at 15 mg/kg body weight). Rats are anaesthetized 7 days following surgery, blood samples are collected, and the skin containing four wounds is excised for histological analysis. Collagenase and gelatinase activity is measured from the excised wounds according to the methods described in Golub et al.Ann. N. Y. Acad. Sci. 1994: 732: 96. Wound tissue is also removed under sterile conditions for sectioning purposes. Immunohistochemistry and *in situ* hybridization is then performed on the sections, as described in Pirila, *et al., supra*

### Example 23: In vivo Traumatic Brain Injury Mouse Model

In this example, mice are used to determine if tetracycline compounds are effective agents that can be used to treat traumatic brain injury. Other models which can be used are described in Meijia, et al. Neurosurgery. 2001:48(6):1393-1399.

To study the pretreatment effects of tetracycline compounds on traumatic brain injury, 12 hours prior to surgery, adult C57BL/6 mice are injected intraperitoneally with tetracycline compounds at a dose of 45 mg/kg body weight. To perform the traumatic brain injury surgery, adult C57BL/6 mice are anaesthetized with isoflurane in 70% N₂O and 30% O₂. Mice then undergo an atraumatic craniotomy removing the right parietal bone to the coronal, lateral to the sagittal, and anterior to the lamboid suture. Laterally, the craniotomy is extended to the temporalis muscle insertion. A 20g weight is then dropped from a height of 150 mm inside a cylinder onto a piston, which is positioned over the craniotomy window. For the pretreatment and posttreatment test groups of mice, beginning 30 minutes after the trauma, the mice receive a dose of the tetracycline compound every 12 hours, at a dose of 90 mg/kg body weight for the first 24 hours after trauma, and then 45 mg/kg body weight thereafter until the mice are sacrificed.

After sacrifice, the mice are perfused with 4% paraformaldehyde and their brains are removed and frozen in chilled isopentane after cryoprotection in 30% sucrose. Sections are prepared and immunohistochemistry is performed in them using specific antibodies against caspase-1 and caspase-3.

### Example 24: In vivo Arthritic-Osteoporosis Rat Model

In this example, rats are used to determine if tetracycline compounds are effective agents that can be used to treat arthritic-osteoporosis. Models which can be used are described in Ramamurthy, et al. Curr. Med. Chem. 2001;8:295-303, as is detailed below.

Mature female rats are either sham operated or are ovariectomized, which leads to an overall increase in bone turnover, whereby bone resorption exceeds bone formation. Rats are divided into experimental and control groups. Designated groups receive tetracycline compounds through oral administration at 2 mg/day. Ninety days following the initial administration of the tetracycline compounds, rats are anesthetized with a mixture of ketamine and rompun, their blood is drawn, and they are sacrificed. One femur from each rat is collected, frozen under steril conditions in liquid nitrogen, and stored for RNA preparation. The tibiae from each rat is also removed, dissected to the periosteum, and measured in length, after which each is stored in 70% ethanol.

RNA is extracted from the femurs according to standard methods. Extracted mRNA is analyzed by performing a Northern blot analysis using probes to known bones transcripts, including type I collagen, osteopontin, and collagenase. Levels of RNA are quantified by dot blot analysis. Levels are compared among the experimental and control groups.

Bone mineral density (BMD) among the control and experimental gorups is determined on the metaphyseal region of the proximal tibia at a site that is equidistant between the proximal articular surface and the midpoint of the diaphysis. A single, 0.5mm slice perpendicular to the long axis of the tibia shaft is collected and analyzed. The BMC and BMD area properties are determined using software available in the field. Bones are also sectioned and their histology studied according to the methods of Ramamurthy *et al., supra.*

### Example 25: In vitro Motor Neuron Disease Assay

In this example, spinal cord cultures are used to test the ability of tetracycline compounds to reduce apoptotic neuronal death and microglial activation, as described in Tikka et al. Brain. 2002:125(4):722-731.

Spinal cell cultures are prepared from 14 day old mouse embryos according to methods known in the art. Primary spinal cell cultures are cultured both on the presence and absence of tetracycline compounds. Primary spinal cell cultures are then exposed to neurotoxic cerebral spinal fluid (CSF) from patients suffering from motor neuron disease (MND). On day 7, spinal cord cultures are exposed to CSF samples (medium containing 25% CSF in DMEM and 1% HS-HIU) for 24 hours. Following exposure, spinal cord cultures are fixes with 4% paraformaldehyde, rinsed in 0.1M phosphate-buffered saline and incubated with the nuclear binding dye bis-benzimide for 5 minutes. This nuclear dye reveals cells undergoing apoptotic death. The stained cultures are also processed for immunohistochemical analysis of neurofilament phosphorylation using antibodies against neurofilaments. Results are compared between the control group of cells and those cells incubated with tetracycline compounds.

### EQUIVALENTS

Those skilled in the art will recognize, or be able to ascertain using no more than routine experimentation, many equivalents to the specific embodiments and methods described herein. Such equivalents are intended to be encompassed by the scope of the following claims.

All patents, patent applications, and literature references cited herein are hereby expressly incorporated by reference.

For the avoidance of doubt, the present application is directed to the subject-matter defined in the following numbered paragraphs (referred to as "para" or "paras"):
1. A method for treating a disease with a tetracycline compound having a target therapeutic activity, comprising administering to a subject an effective amount of a tetracycline compound having said target therapeutic activity, such that the disease is treated.
2. The method of para 1, wherein said disease is an inflammatory process associated state.
3. The method of para 2, wherein said inflammatory process associated state is acute lung injury, adult respiratory distress syndrome, acute respiratory distress syndrome, aortic or vascular aneurysms, arteriosclerosis, atherosclerosis, bone or cartilage degradation, bronchiectasis, cancer, chronic obstructive pulmonary disease, corneal ulceration, cystic fibrosis, diabetes, diabetic complications, diabetic ulcers, dry eye, emphysema, ischemia, restenosis, malaria, metastasis, multiple sclerosis, osteoarthritis, osteoporosis, osteosarcoma, osteomyelitis, periodontitis, rheumatoid arthritis, neurological disorders, senescence, skin and eye diseases, stroke, tissue wounds, tumor growth, tumor invasion, ulcerative colitis, or vascular stroke.
4. The method of para 2 or 3, wherein said inflammatory process associated state is associated with a matrix metalloproteinase.
5. The method of para 4, wherein said matrix metalloproteinase is MMP-1, MMP-2, MMP-3, MMP-4, MMP-5, MMP-6, MMP-7, MMP-8, MMP-9, MMP-10, MMP-11, MMP-12, MMP-13, MMP-14, MMP-15, MMP-16, MMP-17, MMP-18, MMP-19 or MMP-20.
6. The method of para 2, wherein said inflammatory process associated state is a NO associated state.
7. The method of para 2, wherein said inflammatory process associated state is a chronic or recurrent inflammatory disorder.
8. The method of para 2, wherein said inflammatory process associated state is an acute inflammatory disorder.
9. The method of para 3, wherein said inflammatory process associated state is diabetes.
10. The method of para 9, wherein said diabetes is juvenile diabetes.
11. The method of para 9, wherein said diabetes is diabetes mellitus.
12. The method of any one of paras 9-11, wherein said tetracycline compound inhibits protein glycosylation in said subject.
13. The method of para 3, wherein said inflammatory process associated state is rheumatoid arthritis or osteoarthritis.
14. The method of para 2, wherein disease is a bone mass disorder.
15. The method of para 14, wherein said bone mass disorder is osteoporosis.
16. The method of para 3, wherein inflammatory process associated state is a vascular aneurysm of vascular tissue.
17. The method of para 16, wherein said tetracycline compound prevents the formation of said vascular aneurysm.
18. The method of para 16, wherein said tetracycline compound induces the regression of said vascular aneurysm.
19. The method of any one of paras 16-18, wherein said vascular tissue is an artery of said subject.
20. The method of para 3, wherein said disease is acute respiratory distress syndrome or adult respiratory distress syndrome.
21. The method of para 3, wherein said disease is a tissue wound.
22. The method of para 3, wherein said disease is ischemia or stroke.
23. The method of para 3, wherein said disease is dry eye.
24. The method of para 2, wherein said disease is an acute, chronic or recurrent lung disorder.
25. The method of para 24, wherein said chronic lung disorder is asthma, emphysema, bronchitis, or cystic fibrosis.
26. The method of para 2, wherein said disease is hepatitis or sinusitis.
27. The method of para 3, wherein said disease is diabetic complications or diabetic ulcers.
28. The method of para 1, wherein said disease is a neurological disorder.
29. The method of para 28, wherein said neurological disorder is Alzheimer's disease, a dementia related to Alzheimer's disease, Parkinson's disease, Lewy diffuse body disease, senile dementia, Huntington's disease, Gilles de la Tourette's syndrome, multiple sclerosis, amyotrophic lateral sclerosis (ALS), progressive supranuclear palsy, epilepsy, Creutzfeldt-Jakob disease, an autonomic function disorder, hypertension, a sleep disorder, a neuropsychiatric disorder, depression, schizophrenia, schizoaffective disorder, Korsakoff's psychosis, mania, anxiety disorders, a phobic disorder, a learning disorder, a memory disorder, amnesia, age-related memory loss, attention deficit disorder, dysthymic disorder, major depressive disorder, mania, obsessive-compulsive disorder, psychoactive substance use disorders, anxiety, panic disorder, bipolar affective disorder, BP-1, migraine, traumatic brain injury, spinal cord trauma, motor neuron disease, or nerve damage.
30. The method of para 1, wherein said disease is cancer.
31. The method of para 30, wherein said cancer is a tumor.
32. The method of para 30 or 31, wherein said tetracycline compound inhibits tumor metastasis.
33. The method of para 31 or 32, wherein said tumor is a carcinoma or a sarcoma.
34. The method of any one of paras 30-33, wherein said tetracycline compound decreases angiogenesis.
35. The method of any one of paras 1-34, wherein said tetracycline compound is administered in combination with a second agent.
36. The method of para 35, wherein said second agent is a chemotherapeutic agent or radiation therapy.
37. The method of para 35, wherein said second agent is a neuroprotective agent.
38. The method of para 37, wherein said neuroprotective agent comprises a compound that remove protein build up, anti-inflammatory agents, omega-3 fatty acids, minocycline, dexanabionol, compounds that increase energy available to cells, antioxidants, gingko biloba, co-enzyme Q-10, vitamin E, vitamin C, vitamin A, selenium, lipoic acid, selegine, anti-glutamate therapies, remacemide, riluzole, lamotrigine, gabapentin, GABA-ergic therapies baclofen, muscimol, gene transcription regulator, glucocorticoids, retinoic acid, erythropoietin, TNF-α antagonists, cholinesterase inhibitors, N-methyl-D-aspartate (NMDA) antagonists, opiod antagonists, neuronal membrane stabilizers, CDP-choline, calcium channel blockers, sodium channel blockers, or prednisone.
39. The method of para 35, wherein said second agent is an antiinfective agent.
40. The method of any one of paras 1-39, wherein said tetracycline compound is administered with a suitable pharmaceutical carrier.
41. The method of any one of paras 1-40, wherein said subject is a human.
42. The method of any one of paras 1-41, wherein said tetracycline compound is of formula I: 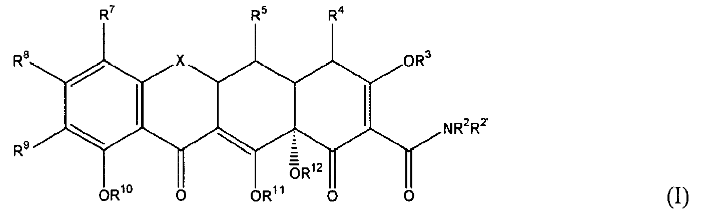 wherein
   R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R^{2'}, R³, R¹⁰, R¹¹ and R¹² are each hydrogen or a pro-drug moiety;
   R⁴ is NR^{4'}R^{4"}, alkyl, alkenyl, alkynyl, hydroxyl, halogen, or hydrogen;
   R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl, alkylsulfonyl, alkylamino, arylalkyl, alkyl carbonyloxy, or aryl carbonyloxy;
   R⁶ and R^{6'} are each independently hydrogen, methylene, absent, hydroxyl, halogen, thiol, alkyl, alkenyl, alkynyl, aryl, alkoxy, alkylthio, alkylsulfinyl, alkylsulfonyl, alkylamino, or an arylalkyl;
   R⁷ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃NR^{7c}C(=W')WR^{7a};
   R⁸ is hydrogen, hydroxyl, halogen, thiol, nitro, alkyl, alkenyl, alkynyl, aryl, alkoxy, alkylthio, alkylsulfinyl, alkylsulfonyl, alkylamino, amino, arylalkenyl, arylalkynyl, acyl, aminoalkyl, heterocyclic, thionitroso, or -(CH₂)₀₋₃NR^{8c}C(=E')ER^{8a};
   R⁹ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃NR^{9c}C(=Z')ZR^{9a};
   R^{7a}, R^{7b}, R^{7c}, R^{7d}, R^{7e}, R^{7f}, R^{8a}, R^{8b}, R^{8c}, R^{8d}, R^{8e}, R^{8f}, R^{9a}, R^{9b}, R^{9c}, R^{9d}, R^{9e}, and R^{8f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R¹³ is hydrogen, hydroxy, alkyl, alkenyl, alkynyl, alkoxy, alkylthio, aryl, alkylsulfinyl, alkylsulfonyl, alkylamino, or an arylalkyl;
   E is CR^{8d}R^{8e}, S, NR^{8b} or O;
   E' is O, NR^{8f}, or S;
   W is CR^{7d}R^{7e}, S, NR^{7b} or O;
   W' is O, NR^{7f}, or S;
   X is CHC(R¹³Y'Y), C=CR¹³Y, CR^{6'}R⁶, S, NR⁶, or O;
   Y' and Y are each independently hydrogen, halogen, hydroxyl, cyano, sulfhydryl, amino, alkyl, alkenyl, alkynyl, alkoxy, alkylthio, alkylsulfinyl, alkylsulfonyl, alkylamino, or an arylalkyl;
   Z is CR^{9d}R^{9e}, S, NR^{9b} or O;
   Z' is O, S, or NR^{9f}, and pharmaceutically acceptable salts, esters and enantiomers thereof.
43. The method of para 42, wherein R², R^{2'}, R⁸, R¹⁰, R¹¹, and R¹² are each hydrogen, X is CR⁶R^{6'}, and R⁴ is NR^{4'}R^{4"}, wherein R^{4'} and R^{4"} are each methyl.
44. The method of para 42 or 43, wherein R⁹ is hydrogen.
45. The method of para 44, wherein R⁷ is substituted or unsubstituted aryl.
46. The method of para 45, wherein said aryl is substituted with an amino group.
47. The method of para 44, wherein R⁷ is a substituted or unsubstituted heterocycle.
48. The method of para 47, wherein said heterocycle is substituted with an amino group.
49. The method of para 44, wherein R⁷ is substituted or unsubstituted alkenyl.
50. The method of para 44, wherein R⁷ is substituted or unsubstituted alkynyl.
51. The method of para 44, wherein R⁷ is substituted or unsubstituted alkyl.
52. The method of para 51, wherein R⁷ is substituted with an aryl group.
53. The method of para 51, wherein R⁷ is substituted with a carbonyl group.
54. The method of para 51, wherein R⁷ is substituted with an amino group.
55. The method of para 54, wherein said amino group is alkylamino.
56. The method of para 44, wherein R⁷ is -CH₂NR^{7c}C(=W')WR^{7a}.
57. The method of para 56, wherein R^{7c} is hydrogen, and W and W' are each oxygen.
58. The method of para 44, wherein R⁷ is -NR^{7c}C(=W')WR^{7a}.
59. The method of para 58, wherein R^{7c} is hydrogen, and W and W' are each oxygen.
60. The method of para 44, wherein R⁷ is substituted or unsubstituted acyl.
61. The method of para 44, wherein R⁷ is substituted or unsubstituted amino.
62. The method of para 44, wherein R⁷ is substituted or unsubstituted oximyl.
63. The method of para 43, wherein R⁷ is hydrogen or dimethylamino.
64. The method of para 63, wherein R⁹ is substituted or unsubstituted amino.
65. The method of para 64, wherein said amino is alkylamino.
66. The method of para 63, wherein R⁹ is substituted or unsubstituted alkyl.
67. The method of para 66, wherein said substituted alkyl is substituted with an substituted or unsubstituted amino or amido group.
68. The method of para 67, wherein said amino group is substituted or unsubstituted alkylamino.
69. The method of para 63, wherein R⁹ is substituted or unsubstituted aryl.
70. The method of para 69, wherein said aryl group is substituted or unsubstituted phenyl.
71. The method of para 70, wherein said phenyl group is substituted with amino.
72. The method of para 63, wherein R⁹ is a substituted or unsubstituted heterocycle.
73. The method of para 63, wherein R⁹ is substituted or unsubstituted alkynyl.
74. The method of para 63, wherein R⁹ is -CH₂NR^{9c}C(=Z')ZR^{9a}.
75. The method of para 74, wherein R^{9c} is hydrogen, Z' is oxygen and Z is nitrogen.
76. The method of para 74, wherein R^{9c} is hydrogen, Z' and Z are oxygen.
77. The method of para 73, wherein R⁹ is -NR^{9c}C(=Z')ZR^{9a}.
78. The method of para 77, wherein R^{9c} is hydrogen, Z' is oxygen and Z is nitrogen.
79. The method of para 43, wherein R⁹ is substituted or unsubstituted alkyl.
80. The method of para 79, wherein R⁹ is substituted with amino.
81. The method of para 80, wherein R⁹ is substituted or unsubstituted alkylaminoalkyl.
82. The method of para 79 or 80, wherein R⁷ is substituted or unsubstituted alkyl.
83. The method of para 82, wherein R⁷ is substituted with amino.
84. The method of para 79, wherein R⁷ is substituted or unsubstituted alkynyl.
85. The method of para 81, wherein R⁷ is a substituted or unsubstituted heterocycle.
86. The method of para 43, wherein R⁷ is substituted or unsubstituted alkyl.
87. The method of para 86, wherein R⁷ is substituted with substituted or unsubstituted amino.
88. The method of para 87, wherein R⁹ is -NR^{9c}C(=Z')ZR^{9a}, R^{9c} is hydrogen, Z' is oxygen and Z is oxygen.
89. The method of para 43, wherein X is C=CR¹³Y, R¹³ is aryl and Y is hydrogen.
90. The method of para 42, wherein R⁷ is a dimeric moiety.
91. The method of any one of paras 1-41, wherein said tetracycline compound is selected from the group consisting of: 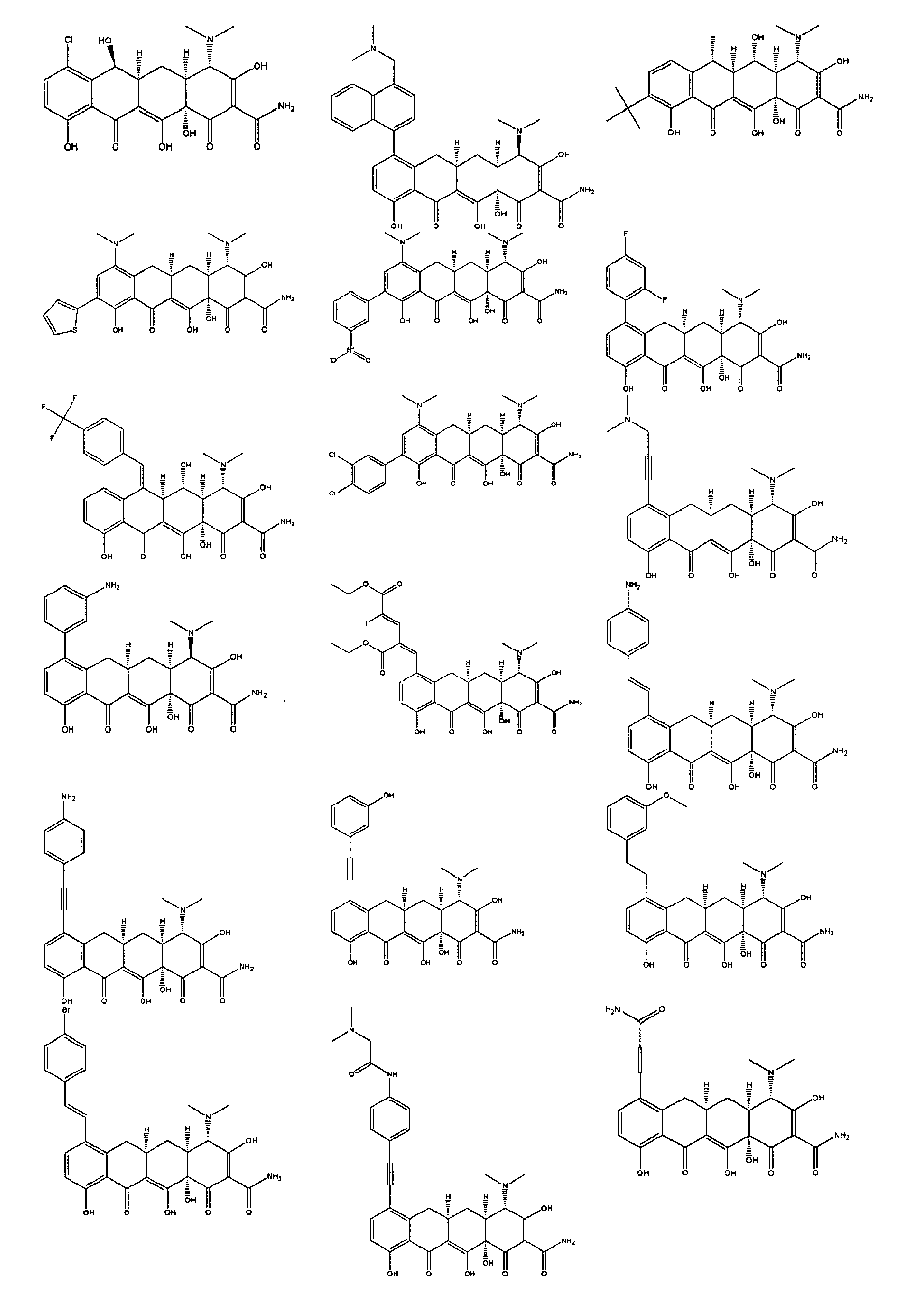 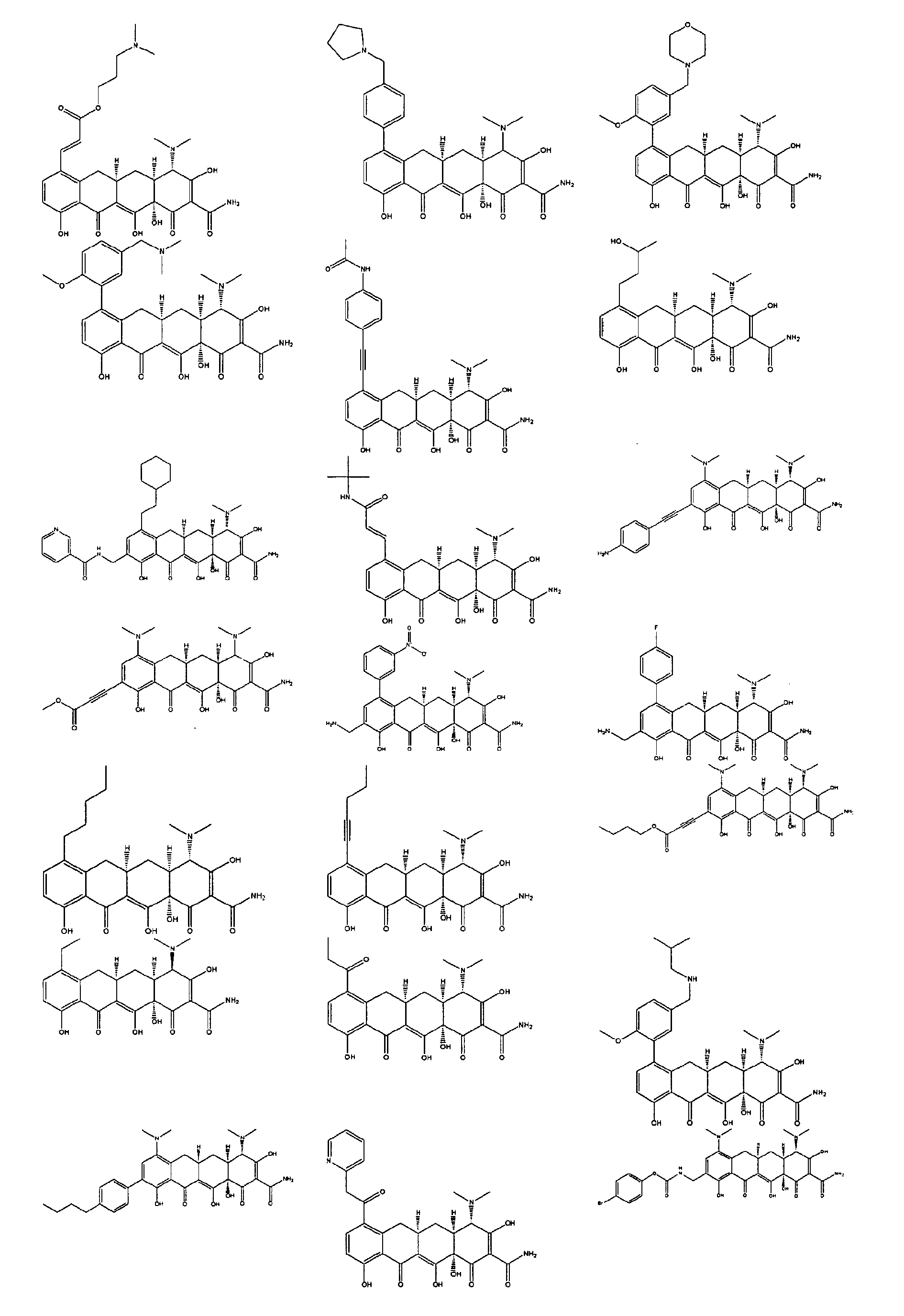 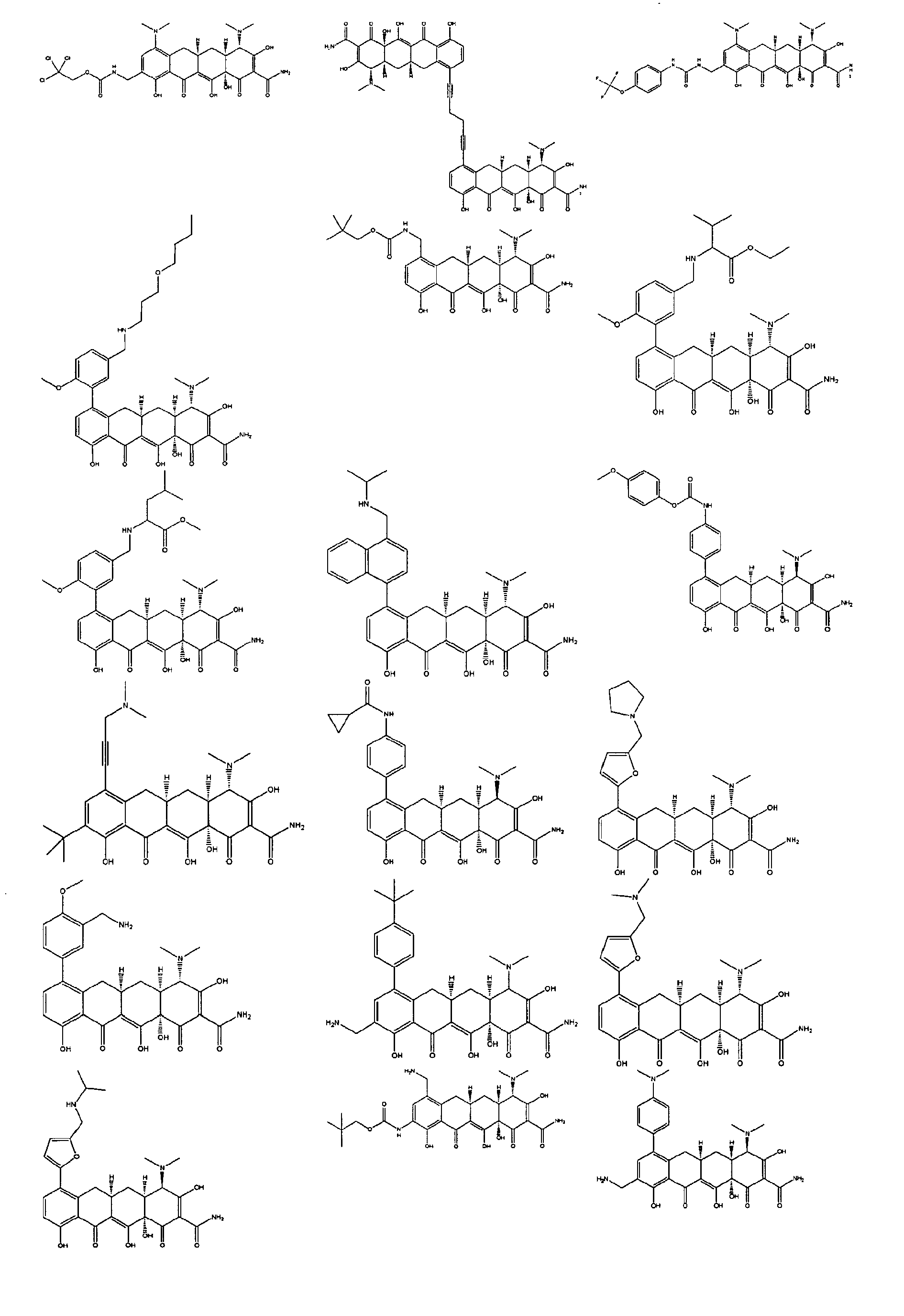 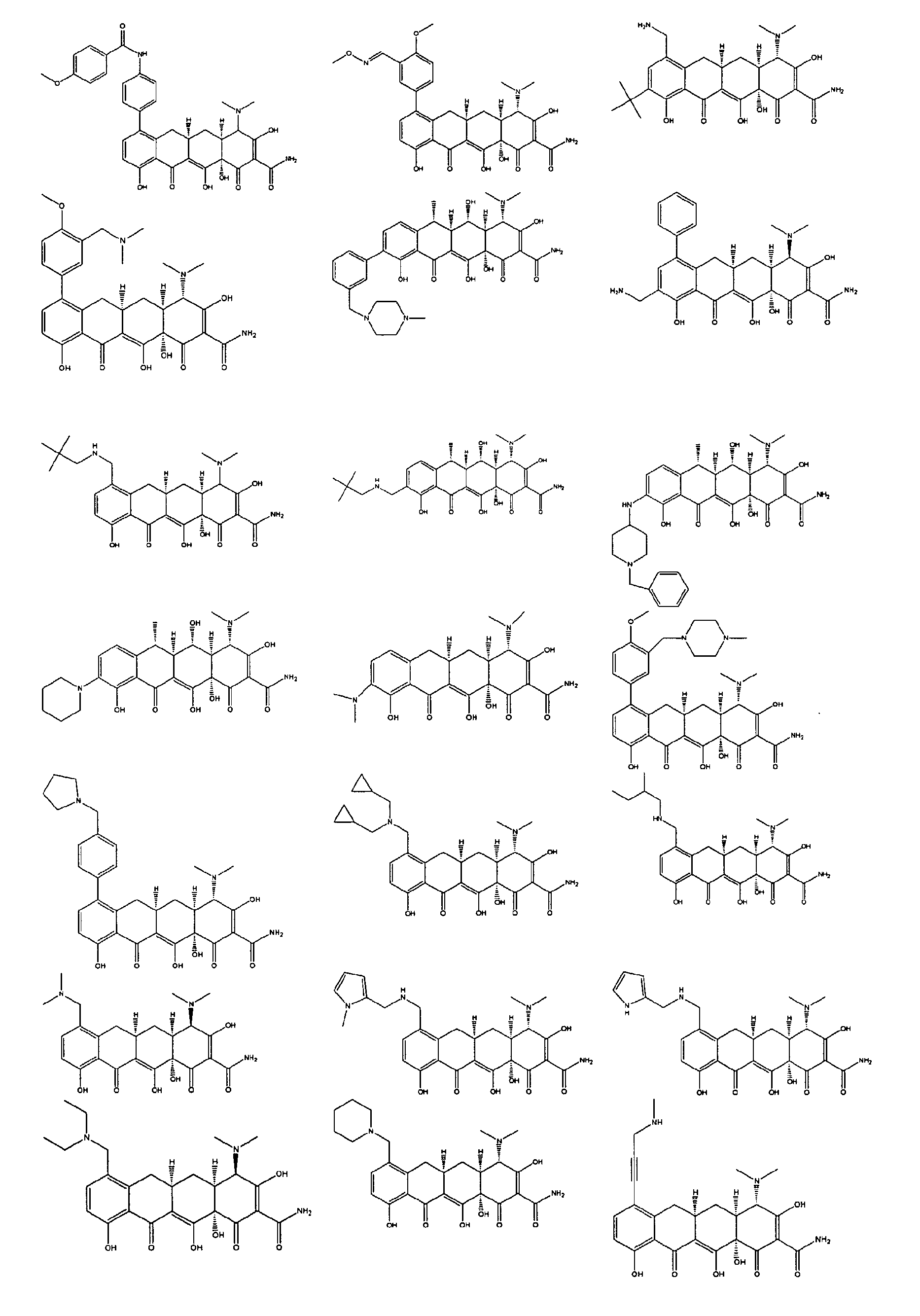 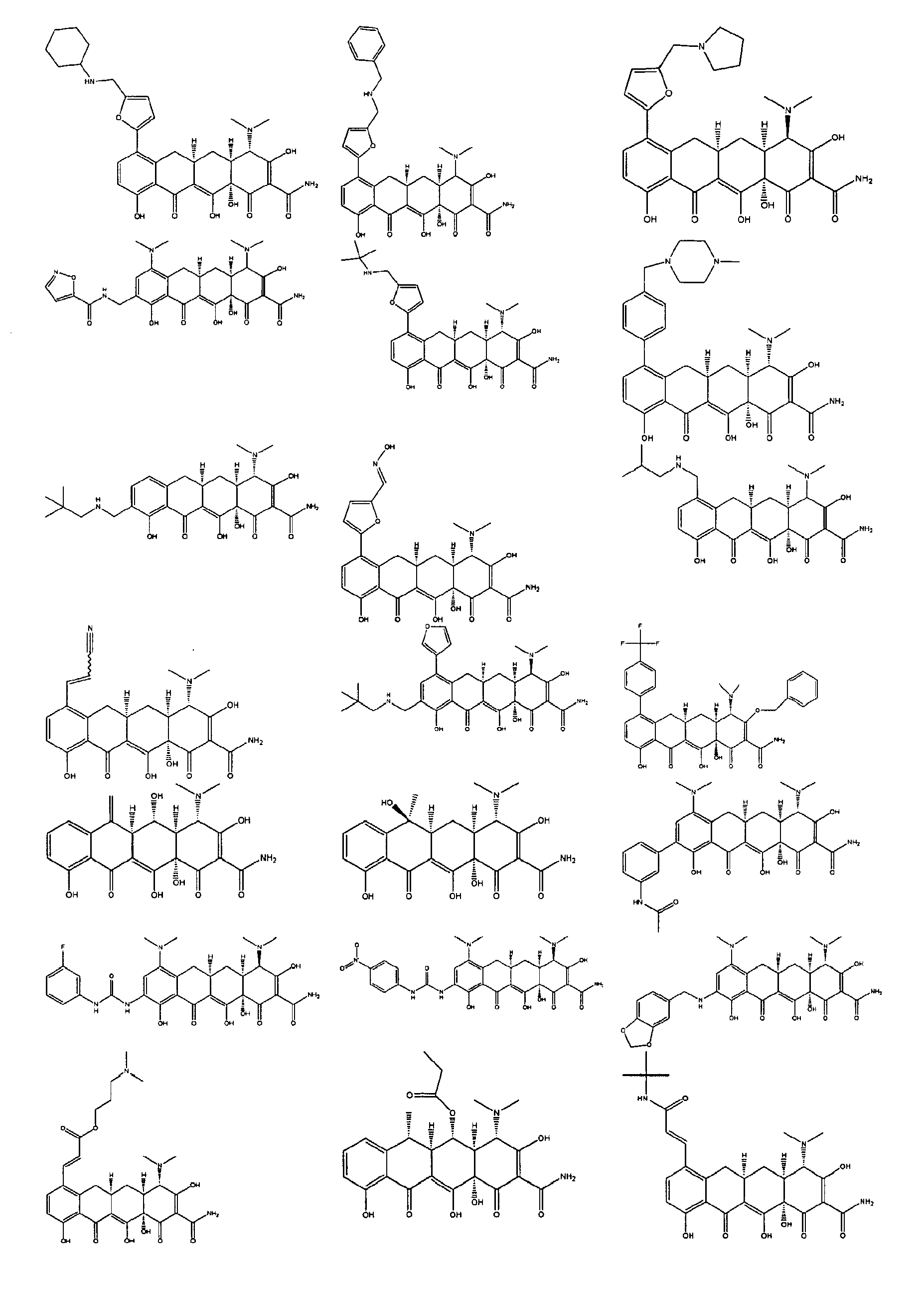 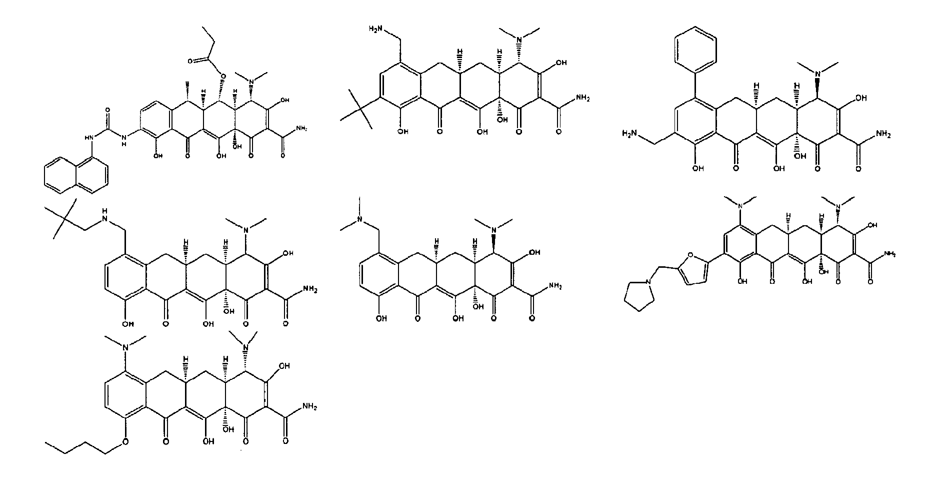
92. The method of any one of paras 1-41, wherein said tetracycline compound is a compound of Table 2 or Table 3.
93. The method of any one of paras 1-92, wherein said tetracycline has antibacterial activity.
94. The method of any one of paras 1-93, wherein said tetracycline compound is a 3, 5, 7, 9, and/or 10, substituted tetracycline compound.
95. The method of any one of paras 1-94, wherein said tetracycline compound is anti-infective.
96. The method of any one of paras 1-94, wherein said tetracycline compound is not anti-infective.
97. The method of any one of paras 1-96, wherein said tetracycline compound is administered with a suitable pharmaceutical carrier.
98. The method of any one of paras 1-97, wherein said subject is a human.
99. A pharmaceutical composition comprising an effective amount of a tetracycline compound in combination with a second agent, wherein said tetracycline compound has a target therapeutic activity.
100. The pharmaceutical composition of para 99, wherein said tetracycline compound is a substituted tetracycline compound.
101. The pharmaceutical composition of para 99 or 100, wherein said second agent is a neuroprotective agent.
102. The pharmaceutical composition of para 99 or 100, wherein said second agent is a chemotherapeutic agent.
103. The pharmaceutical composition of para 99 or 100, wherein said second agent is an antiinfective agent.
104. The pharmaceutical composition of para 103, wherein said antiinfective agent is an antibacterial, antifungal, antiparasitic or antiviral agent.
105. The pharmaceutical compositions of para 100, wherein said substituted tetracycline compound is of the formula: 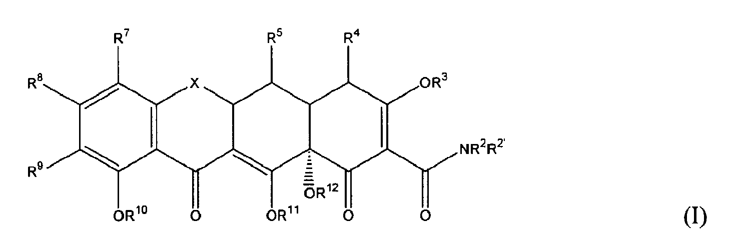 wherein
   R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R^{2'}, R³, R¹⁰, R¹¹ and R¹² are each hydrogen or a pro-drug moiety;
   R⁴ is NR^{4'}R^{4"}, alkyl, alkenyl, alkynyl, hydroxyl, halogen, or hydrogen;
   R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl, alkylsulfonyl, alkylamino, arylalkyl, alkyl carbonyloxy, or aryl carbonyloxy;
   R⁶ and R^{6'} are each independently hydrogen, methylene, absent, hydroxyl, halogen, thiol, alkyl, alkenyl, alkynyl, aryl, alkoxy, alkylthio, alkylsulfinyl, alkylsulfonyl, alkylamino, or an arylalkyl;
   R⁷ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃NR^{7c}C(=W')WR^{7a};
   R⁸ is hydrogen, hydroxyl, halogen, thiol, nitro, alkyl, alkenyl, alkynyl, aryl, alkoxy, alkylthio, alkylsulfinyl, alkylsulfonyl, alkylamino, amino, arylalkenyl, arylalkynyl, acyl, aminoalkyl, heterocyclic, thionitroso, or -(CH₂)₀₋₃NR^{8c}C(=E')ER^{8a};
   R⁹ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃NR^{9c}C(=Z')ZR^{9a};
   R^{7a}, R^{7b}, R^{7c}, R^{7d}, R^{7e}, R^{7f}, R^{8a}, R^{8b}, R^{8c}, R^{8d}, R^{8e}, R^{8f}, R^{9a}, R^{9b}, R^{9c}, R^{9d}, R^{9e}, and R^{8f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R¹³ is hydrogen, hydroxy, alkyl, alkenyl, alkynyl, alkoxy, alkylthio, aryl, alkylsulfinyl, alkylsulfonyl, alkylamino, or an arylalkyl;
   E is CR^{8d}R^{8e}, S, NR^{8b} or O;
   E' is O, NR^{8f}, or S;
   W is CR^{7d}R^{7c}, S, NR^{7b} or O;
   W' is O, NR^{7f}, or S;
   X is CHC(R¹³Y'Y), C=CR¹³Y, CR^{6'}R⁶, S, NR⁶, or O;
   Y' and Y are each independently hydrogen, halogen, hydroxyl, cyano, sulfhydryl, amino, alkyl, alkenyl, alkynyl, alkoxy, alkylthio, alkylsulfinyl, alkylsulfonyl, alkylamino, or an arylalkyl;
   Z is CR^{9d}R^{9e} S, NR^{9b} or O;
   Z' is O, S, or NR^{9f}, and pharmaceutically acceptable salts, esters and enantiomers thereof.
106. The pharmaceutical composition of any one of paras 99-104, wherein said tetracycline compound is a compound of Table 2 or Table 3.
107. The pharmaceutical composition of any one of paras 99-106, wherein said pharmaceutical composition further comprises a pharmaceutically acceptable carrier.
108. The pharmaceutical composition of para 102, wherein said effective amount is effective to treat cancer.
109. The pharmaceutical composition of para 101, wherein said effective amount is effective to treat a neurological disorder.
110. A packaged composition for treatment of a disease with a tetracycline compound with a target therapeutic activity, comprising a tetracycline compound having said target therapeutic activity and directions for using said tetracycline compound for treating said disease.
111. The packaged composition of para 110, further comprising a pharmaceutically acceptable carrier.
112. The packaged composition of para 110 or 111, wherein said disease is an IPAS.
113. The packaged composition of para 110 or 111, wherein said disease is a neurological disorder.
114. The packaged composition of para 110 or 111, wherein said disease is cancer.
115. The packaged composition of any one of paras 110-114, wherein said tetracycline compound is a substituted tetracycline compound.
116. The packaged composition of any one of paras 110-115, further comprising a second agent.
117. The packaged composition of para 116, wherein said second agent is a chemotherapeutic agent.
118. The packaged composition of para 116, wherein said second agent is an antiinfective agent.
119. The packaged composition of para 116, wherein said second agent is an neuroprotective agent.

## Claims

1. A substituted tetracycline compound for use in treating osteomyelitis, bronchiectasis, cystic fibrosis, malaria or periodontitis, wherein the tetracycline compound has a target therapeutic activity, and further wherein the tetracycline compound is of formula I: 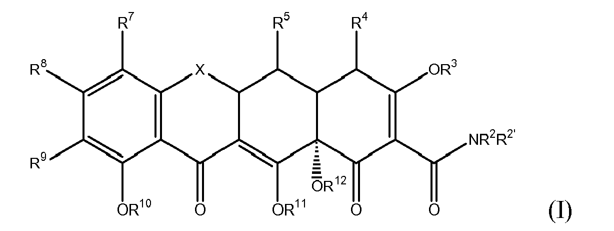 wherein:
X is CR^{6'}R⁶;
R² and R^{2'} are each hydrogen;
R^{4'} and R^{4"} are each methyl;
R⁴ is NR^{4'}R^{4"};
R³, R¹¹ and R¹² are each hydrogen;
R¹⁰ is hydrogen;
R⁵ is hydrogen;
R⁶ and R^{6'} are each hydrogen;
R⁷ is dimethylamino;
R⁹ is substituted or unsubstituted alkylaminoalkyl;
R⁸ is hydrogen;
or a pharmaceutically acceptable salt thereof.

2. The compound for use as claimed in claim 1, wherein said tetracycline compound is administered in combination with a second agent.

3. The compound for use as claimed in claim 2, wherein said second agent is a chemotherapeutic agent or radiation therapy.

4. The compound for use as claimed in claim 2, wherein said second agent is a neuroprotective agent.

5. The compound for use as claimed in claim 1, wherein said tetracycline compound is selected from the group consisting of: 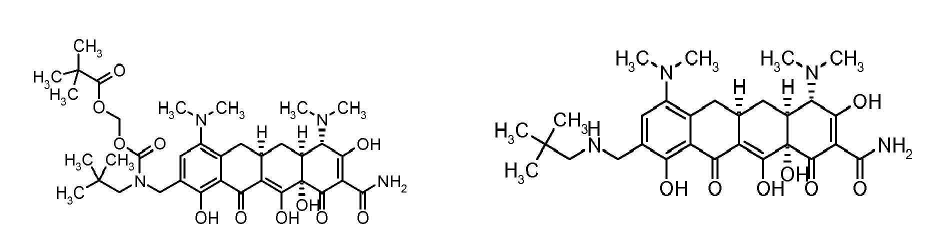 and pharmaceutically acceptable salts thereof.

6. The compound for use as claimed in claim 1, wherein said tetracycline compound is selected from Table 2 or Table 3.

7. A pharmaceutical composition comprising an effective amount of a tetracycline compound according to claim 1 for use in treating osteomyelitis, bronchiectasis, cystic fibrosis, malaria or periodontitis in combination with a second agent.

8. A packaged composition for treatment of a disease with a tetracycline compound according to claim 1 for use in treating osteomyelitis, bronchiectasis, cystic fibrosis, malaria or periodontitis, comprising a tetracycline compound and directions for using said tetracycline compound for treating said osteomyelitis, bronchiectasis, cystic fibrosis, malaria or periodontitis.

9. A tetracycline compound, wherein said compound is: 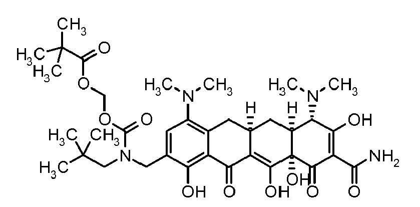 or a pharmaceutically acceptable salt thereof.
